# EUROPEAN PATENT SPECIFICATION

(11) **EP 2 097 420 B1**
(45) Date of publication and mention of the grant of the patent: **04.04.2012**
(21) Application number: 07825738.3
(22) Date of filing: 16.11.2007
(51) Int. Cl.: C07D 491/107, A61K 31/39, A61P 5/00

(54) **SPIROKETONE INHIBITORS OF ACETYL-COA CARBOXYLASE**
SPIROKETONE ALS INHIBITOREN VON ACETYL-COA-CARBOXYLASE
SPIROCÉTONE INHIBITEURS DE LA ACÉTYL-COA CARBOXYLASE

(30) Priority: 29.11.2006 US 861779 P
(43) Date of publication of application: 09.09.2009
(73) Proprietor: Pfizer Products Inc., Groton, CT 06340 (US)
(72) Inventor: CORBETT, Jeffrey Wayne, Groton, CT 06340 (US); ELLIOTT, Richard Louis, Groton, CT 06340 (US); BELL, Andrew Simon, Sandwich Kent CT13 9NJ (GB)
(74) Representative: Dekker, Henrike Cornelie Christine
(86) International application number: PCT/IB2007/003639
(87) International publication number: WO 2008/065508

(56) References cited:
- WO-A-2007/011809
- DATABASE WPI Week 200537 Derwent Publications Ltd., London, GB; AN 2005-359210 XP002471702 & JP 2005 119987 A (AJINOMOTO KK) 12 May 2005 (2005-05-12)

## Description

### FIELD OF THE INVENTION

This invention relates, to substituted 1'-(benzoyl)spiro[chromene-2,4'-piperidin]-4(3H)-one compounds that act as inhibitors of acetyl-CoA carboxylases and their use in treating obesity.

### BACKGROUND OF THE INVENTION

Extreme obesity is a major illness in the United States and other countries. Its complications include hypertension, diabetes, coronary artery disease, stroke, congestive heart failure, venous disease, multiple orthopedic problems and pulmonary insufficiency with markedly decreased life expectancy. Medical management including dietary, psychotherapy, medications and behavioral modification techniques have yielded extremely poor results in multiple trials. Several surgical techniques have been tried which have bypassed the absorptive surface of the small intestine or have been aimed at reducing the stomach size by either partition or bypass. These procedures have been proven both hazardous to perform in morbidly obese patients and have been fraught with numerous life-threatening postoperative complications. Moreover such operative procedures are often difficult to reverse.

Acetyl-CoA carboxylases (ACC) are a family of enzymes found in most species and are associated with fatty acid synthesis and metabolism through catalyzing the production of malonyl-CoA from acetyl-CoA. In mammals, two isoforms of the ACC enzyme have been identified. ACC1, which is expressed at high levels in lipogenic tissues, such as fat and the liver, controls the first committed step in the biosynthesis of long-chain fatty acids. If acetyl-CoA is not carboxylated to form malonyl-CoA, it is metabolized through the Krebs cycle. ACC2, which is a minor component of hepatic ACC but the predominant isoform in heart and skeletal muscle, and catalyzes the production of malonyl-CoA at the cystolic surface of mitochondria, regulates how much fatty acid is utilized in β-oxidation by inhibiting carnitine palmitoyl transferase. Thus, by increasing fatty acid utilization and by preventing increases in de novo fatty acid synthesis, chronic administration of an ACC-I may also deplete liver and adipose tissue TG stores in obese subjects consuming a high or low-fat diet, leading to selective loss of body fat.

JP 2005 119987 discloses acyl sulfonamide derivatives of 1'-(phenylaminocarbonyl)spiro[chromene-2,4'-piperidin]-4(3H)-one useful as Acetyl-CoA carboxylase inhibitors for treating obesity.

Therefore, there is a continuing need for medicaments containing ACC1 and ACC2 inhibitors to respectively treat obesity by inhibiting fatty acid synthesis and by increasing fatty acid oxidation.

### SUMMARY OF THE INVENTION

The present invention relates to compounds having the structure of Formula (1) 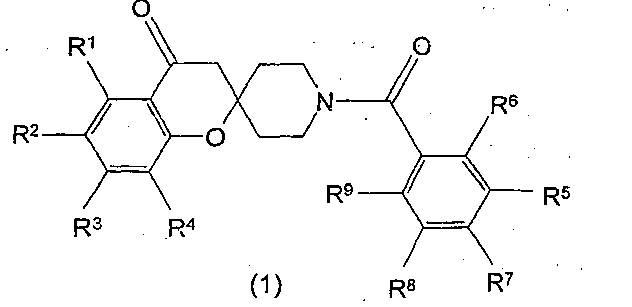 or a pharmaceutically acceptable salt thereof, wherein:
(a) R¹ is H, OH, halo, cyano, C₁₋₃ alkyl, C₁₋₃ alkoxy, C₁₋₃ haloalkyl, C₁₋₃ haloalkoxy, C₁₋₃ alkylsulfonyl-, - CO(O)H, -C(O)OC₁₋₃ alkyl or phenyl, wherein said phenyl is optionally substituted with one to five R¹⁰;
(b) each R¹⁰ is independently OH, halo, cyano, C₁₋₃ alkyl, C₁₋₃ alkoxy, C₁₋₃ haloalkyl or C₁₋₃ haloalkoxy;
(c) R² and R³ are each independently H, OH, halo, cyano, C₁₋₃ alkyl, C₁₋₃ alkoxy, C₁₋₃ haloalkyl, C₁₋₃ haloalkoxy, C₁₋₃ alkylsulfonyl-, -CO(O)H, -C(O)OC₁₋₃ alkyl, -C(O)NR¹¹R¹², or phenyl wherein said phenyl is optionally substituted with one to five R¹⁰;
(d) R¹¹ and R¹² are taken separately and are each independently H or C₁₋₃ alkyl, or R¹¹ and R¹² are taken together, with the nitrogen to which they are attached, to form a 4-7-membered heterocycloalkyl;
(e) R⁴ is H, halo, cyano, C₁₋₃ alkyl or C₁₋₃ haloalkyl;
(f) R⁶ is taken separately and is H, OH, halo, C₁₋₃ alkyl, C₁₋₃ alkoxy, C₁₋₃ haloalkyl or C₁₋₃ haloalkoxy;
(g) R⁷ is taken separately and is H, OH, halo, C₁₋₃ alkyl, C₁₋₃ alkoxy, C₁₋₃ haloalkyl or C₁₋₃ haloalkoxy;
(h) R⁵ is taken separately and is a 4-7-membered heteroaryl optionally substituted with halo, C₁₋₃ alkyl, C₁₋₃ alkoxy, C₁₋₃ alkyl-OH, C₁₋₃ haloalkyl or C₁₋₃; or
(i) R⁵ is taken together with R⁶ or R⁷, and with the phenyl to which R⁵ and R⁶ or R⁷ are attached, to form a polycyclic heterocyclic radical, with a nitrogen-bearing ring wherein at least one nitrogen atom is bound to a carbon atom of said phenyl, wherein the nitrogen-bearing ring is optionally fused to cyclohexene, 5,6-dihydro-pyridine or 5,6-dihydro-1H-pyridin-2-one, and wherein the nitrogen-bearing ring is optionally substituted independently with one to two oxo, halo, C₁₋₃ alkyl, C₁₋₃ alkoxy, C₁₋₃ alkyl-OH, C₁₋₃ haloalkyl, C₁₋₃ haloalkoxy, 4-7-membered heteroaryl, 4-7-membered heterocycloalkyl or phenyl, wherein said phenyl is optionally substituted with one to five R¹⁰, provided that R⁵ is not taken together with R⁶ to form a benzotriazolyl or a benzooxadiazolyl and provided that R⁵ is not taken together with R⁷ to form a benzooxadiazolyl; and
(j) R⁸ and R⁹ are independently H, OH, halo; C₁₋₃ alkyl, C₁₋₃ alkoxy, C₁₋₃ haloalkyl or C₁₋₃ haloalkoxy, provided that said compound is not 1'-(1H-1,2,3-benzotriazol-5-ylcarbonyl)-5-methoxyspiro[chromene-2,4'-piperidin]-4(3H)-one, 6-chloro-7-methyl-1'-[3-(1H-pyrazol-4-yl)benzoyl]spiro[chromene-2,4'-piperidin]-4(3H)-one, 6,7-dimethyl-1'-[3-(1H-pyrazol-4-yl)benzoyl]spiro[chromene-2,4'-piperidin]-4(3H)-one or 6,7-dimethyl-1'-[3-(1H-pyrazol-4-yl)benzoyl]spiro[chromene-2,4'-piperidin]-4(3H)-one, or a pharmaceutically acceptable salt thereof.

The present invention also relates to a pharmaceutical composition comprising a compound of Formula (1) or one of the compounds 1'-(1H-1,2,3-benzotriazol-5-ylcarbonyl)-5-methoxyspiro[chromene-2,4'-piperidin]-4(3H)-one; 6-chloro-7-methyl-1'-[3-(1H-pyrazol-4-yl)benzoyl]spiro[chromene-2,4'-piperidin]-4(3H)-one; 6,7-dimethyl-1'-[3-(1H-pyrazol-4-yl)benzoyl]spiro[chromene-2,4'-piperidin]-4(3H)-one; and 6,7-dimethyl-1'-[3-(1H-pyrazol-4-yl)benzoyl]spiro[chromene-2,4'-piperidin]-4(3H)-one, or a pharmaceutically acceptable salt of the compound, and a pharmaceutically acceptable carrier, vehicle, diluent or excipient.

The present invention further relates to compounds of formula (1) for use in a method of treating a condition of being overweight in a mammal in need of such treatment, which comprises administering to the mammal a therapeutically effective amount of a compound of Formula (1) or 1'-(1H-1,2,3-benzotriazol-5-ylcarbonyl)-5-methoxyspiro[chromene-2,4'-piperidin]-4(3H)-one; 6-chloro-7-methyl-1'-[3-(1H-pyrazol-4-yl)benzoyl]spiro[chromene-2,4'-piperidin]-4(3H)-one; 6,7-dimethyl-1'-[3-(1H-pyrazol-4-yl)benzoyl]spiro[chromene-2,4'-piperidin]-4(3H)-one; and 6,7-dimethyl-1'-[3-(1H-pyrazol-4-yl)benzoyl]spiro[chromene-2,4'-piperidin]-4(3H)-one, or a pharmaceutically acceptable salt thereof.

The compounds, salts and pharmaceutical compositions of the present invention are used to treat Type 2 diabetes, insulin resistance, metabolic syndrome, atherosclerosis, hyperlipidemia, dislipidemia, congestive heart failure, coronary heart disease, stroke and cancer. Preferably, the compounds, salts and pharmaceutical compositions of the present invention are used to treat an overweight or obese condition in a mammal.

### DETAILED DESCRIPTION

The terms used to describe the present invention have the following meanings herein.

The compounds and intermediates of the present invention were generally named herein according to the IUPAC (International Union for Pure and Applied Chemistry) recommendations on Nomenclature of Organic Chemistry and the CAS Index rules.

The carbon atom content of the various hydrocarbon-coritaining moieties herein may be indicated by a prefix designating the minimum and maximum number of carbon atoms in the moiety, for example, the prefixes (Cₐ-C_{b})alkyl, and C_{a-b}alkyl, indicate an alkyl moiety of the integer "a" to "b" carbon atoms, inclusive. Thus, for example, (C₁-C₆)alkyl and C₁₋₆alkyl refer to an alkyl group of one to six carbon atoms inclusive.

The term "substituted" means that a hydrogen atom on a carbon, nitrogen or sulfur atom within the radical has been replaced with a different atom or radical. The atom or molecule replacing the hydrogen atom is denoted as a "substituent."

The term "radical" denotes a group of atoms that behaves as a single reactant in a chemical reaction, e. g., an organic radical is a group of atoms that imparts characteristic properties to a compound containing it, or which remains unchanged during a series of reactions, or transformations.

The term "alkyl" denotes a straight or branched, saturated chain of carbon atoms. Examples of alkyl groups include, but are not limited to, ethyl, ethyl, propyl, isopropyl, butyl and *tert*-butyl. The term "alkoxy" denotes a straight or branched, monovalent, saturated chains of carbon atoms bonded to an oxygen atom. Examples of alkoxy groups include methoxy, ethoxy, propoxy, iso-butoxy, *tert*-butoxy, and the like.

The term "halo" refers to chloro, fluoro or bromo.

The term "haloalkyl" refers to an alkyl group wherein one or more carbons are substituted with halo groups. Examples of haloalkyl groups include, but are not limited to, difluoromethyl, trifluoromethyl, and 1,2-difluoroethyl.

The term, "4-7-membered heterocycloalkyl" means a radical having a non-aromatic ring containing four to seven ring atoms, which include one nitrogen, and, optionally, one to two additional heteroatoms selected from the group consisting of O, N and S. Examples of said 4-7 membered heterocycloalkyl ring include, but are not limited to, azetidine, pyrrolidine, piperidine, azepane, pyrroline, imidazoline, imidazolidine, pyrazolidine morpholine, thiomorpholine and piperazine.

The term "4-7-membered heteroaryl" refers to a radical having a monocyclic aromatic ring containing four to seven ring atoms consisting of carbon and one to three heteroatoms each selected from the group consisting of O, N and S. Examples of such heteroaryls include, but are not limited to, pyrrole, pyrazole, pyridine, imidazole, oxadiazole, pyrimidine, oxazole, isoxazole, triazole, tetrazole, pyridazine, pyrazine, thiazole and thiadiazole. A heteroaryl group of the present invention can be optionally substituted one to two times with substituents independently selected from halo, C₁₋₃alkyl, C₁₋₃ alkoxy, C₁₋₃ alkyl-OH, C₁₋₃ haloalkyl and C₁₋₃ haloalkoxy.

The term "polycyclic heterocyclic radical" refers to a two or three ring heterocyclic radical comprising a benzene ring which is fused to an aromatic or non-aromatic 5-6-membered nitrogen-bearing ring wherein the nitrogen-bearing ring contains one nitrogen atom which is bound to a carbon atom on the benzene ring and optional contains an additional one to two heteroatoms selected from N, O and S. The nitrogen-bearing ring of the polycyclic heterocycle may optionally be fused to a third ring selected from the group consisting of cyclohexene and 5,6-dihydro-1H-pyridin-2-one. Examples of polycyclic heterocycle groups include, but are not limited to, 1H-indazole, 1H-benzoimidazole, quinoline, 1,2,3,4-tetrahydroquinoline, quinoxaline, 1H-indole, 2,3-dihydro-1H-benzoimidazole and 1H-benzo[d][1,2,3]triazole, 6,7,8,9-tetrahydro-5H-carbazole, 2,3,4,9-tetrahydro-1H-pyrido[3,4-b]indole and benzooxazole. In a polycyclic heterocycle of the present invention, the nitrogen-bearing ring is optionally substituted with one to two substituents independently selected from oxo, halo, C₁₋₃ alkyl, C₁₋₃ alkoxy, C₁₋₃ alkyl-OH, C₁₋₃ haloalkyl, C₁₋₃ haloalkoxy, phenyl, 4-7-membered heterocyclic ring.

The phrase "related salts" as used herein means pharmaceutically acceptable salts of compounds of the present invention.

The phrase "pharmaceutically acceptable" indicates that the designated carrier, vehicle, diluent, excipient(s), and/or salt is generally chemically and/or physically compatible with the other ingredients comprising the formulation, and physiologically compatible with the recipient thereof.

The term "mammal" relates to an individual animal that is a member of the taxonomic class Mammalia. Examples of mammals include, but are not limited to, humans, dogs, cats, horses and cattle. In the present invention, the preferred mammals are humans, dogs and cats. More preferably, the mammal is a human.

The phrase "therapeutically effective amount" means an amount of a compound of the present invention that (i) treats or prevents the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein.

The terms "treating", "treated", or "treatment" as employed herein includes preventing (e.g., prophylaxis), palliating, slowing progression and curing a disease, such as obesity.

In one embodiment of the compounds of Formula (1), and of the pharmaceutically acceptable salts thereof, wherein R⁵ is taken together with R⁷, the optionally substituted nitrogen-bearing ring optionally contains a second N, O, or S heteroatom. Said nitrogen-bearing ring is optionally fused to cyclohexene, 5,6-dihydro-pyridine or 5,6-dihydro-1H-pyridin-2-one.

More preferably, for the compounds and salts wherein R⁵ and R⁷ are taken together, said polycyclic heterocyclic radical is 1H-indazolyl, 1H-benzoimidazolyl, quinolyl, 1,2,3,4-tetrahydroquinolyl, quinoxalyl. 1H-indolyl, 2,3-dihydro-1H-benzoimidazolyl, 1H-benzo-[d][1,2,3]triazolyl, 6,7,8,9-tetrahydro-5H-carbazolyl, 2,3,4,9-tetrahydro-1H-pyrido-[3,4-b]indolyl or benzooxazolyl. The nitrogen-bearing ring of said polycyclic heterocyclic radical is optionally substituted.

In this embodiment, even more preferably, the polycyclic heterocyclic radical is optionally substituted 1H-indazolyl, 1H-benzoimidazolyl, 1H-indolyl or 2,3,4,9-tetrahydro-1H-pyrido[3,4-b]indolyl. Yet even more preferably, for the compounds and salts wherein R⁵ and R⁷ are taken together, R¹ is H, halo, CH₃ or OCH₃; R³ is H, halo, CH₃ or OCH₃; and R⁴ is H.

in another embodiment of the compounds of Formula (1), and of the pharmaceutically acceptable salts thereof, wherein R⁵ is taken together with R⁶, the optionally substituted nitrogen-bearing ring optionally contains a second N, O, or S heteroatom. Said nitrogen-bearing ring is optionally fused to cyclohexene, 5,6-dihydro-pyridine or 5,6-dihydro-1H-pyridin-2-one.

More preferably, for the compounds and salts wherein R⁵ and R⁶ are taken together, said polycyclic heterocyclic radical is 1H-indazolyl, 1H-benzoimidazolyl, 1H-indolyl or 2,3-dihydro-1H-benzoimidazolyl. The nitrogen-bearing ring of said polycyclic heterocyclic radical is optionally substituted.

In this embodiment, even more preferably, the polycyclic heterocyclic radical is optionally substituted 1H-indazolyl. Yet even more preferably, for the compounds and salts wherein R⁵ and R⁷ are taken together; R¹ is H, halo, CH₃ or OCH₃; R³ is H, halo, CH₃ or OCH₃; and R⁴ is H.

Wherein R⁵ is taken separately, it is preferred that R⁵ is an optionally substituted heteroaryl selected from the group consisting of pyrazolyl, imidazolyl, oxadiazolyl and pyrimidinyl. More preferably, R⁵ is an optionally substituted heteroaryl selected from the group consisting of pyrazolyl and imidazolyl. Even more preferably, R¹ is H, halo, CH₃ or OCH₃; R³ is H, halo, CH₃ or OCH₃; and R⁴ is H.

The compounds of the present invention may contain stereogenic centers These compounds may exist as mixtures of enantiomers or as pure enantiomers. Wherein a compound includes a stereogenic center, the compounds may be resolved into the pure enantiomers by methods known to those skilled in the art, for example by formation of diastereoisomeric salts which may be separated, for example, by crystallization; formation of stereoisomeric derivatives or complexes which may be separated, for example, by crystallization, gas-liquid or liquid chromatography; selective reaction of one enantiomer with an enantiomer-specific reagent, for example enzymatic esterification; or gas-liquid or liquid chromatography in a chiral environment, for example on a chiral support for example silica with a bound chiral ligand or in the presence of a chiral solvent. It will be appreciated that where the desired stereoisomer is converted into another chemical entity by one of the separation procedures described above, a further step is required to liberate the desired enantiomeric form. Alternatively, the specific stereoisomers may be synthesized by using an optically active starting material, by asymmetric synthesis using optically active reagents, substrates, catalysts or solvents, or by converting one stereoisomer into the other by asymmetric transformation.

Certain compounds of Formula (1) may exist in different stable conformational forms which may be separable. Torsional asymmetry due to restricted rotation about an asymmetric single bond, for example because of steric hindrance or ring strain, may permit separation of different conformers. The compounds of the present invention further include each conformational isomer of compounds of Formula (1) and mixtures thereof.

Pharmaceutically acceptable salts, as used herein in relation to compounds of the present invention, include pharmaceutically acceptable inorganic and organic salts of said compound. These salts can be prepared in situ during the final isolation and purification of a compound, or by separately reacting the compound or prodrug thereof, with a suitable organic or inorganic acid and isolating the salt thus formed. Representative salts include, but are not limited to, the hydrobromide, hydrochloride, hydroiodide, sulfate, bisulfate, nitrate, acetate, trifluoroacetate, oxalate, besylate, palmitate, pamoate, malonate, stearate, laurate, malate, borate, benzoate, lactate, phosphate, hexafluorophosphate, benzene sulfonate, tosylate, formate, citrate, maleate, fumarate, succinate, tartrate, naphthylate, mesylate, glucoheptonate, lactobionate and laurylsulphonate salts, and the like. These may also include cations based on the alkali and alkaline earth metals, such as sodium, lithium, potassium, calcium, magnesium and the like, as well as non-toxic ammonium, quaternary ammonium, and amine cations including, but not limited to, ammonium, tetramethylammonium, tetraethylammonium, methylammonium, dimethylammonium, trimethylammonium, triethylammonium, ethylammonium, and the like. For additional examples see, for example, Berge, et al., J. Pharm. Sci., 66, 1-19 (1977).

The compounds and salts of the invention may exist in both unsolvated and solvated forms. The term 'solvate' is used herein to describe a molecular complex comprising the compound of the invention and one or more pharmaceutically acceptable solvent molecules, for example, ethanol. The term 'hydrate' is employed when said solvent is water. Pharmaceutically acceptable solvates include hydrates and other solvates wherein the solvent of crystallization may be isotopically substituted, *e.g.* D₂O, d₆-acetone, d₆-DMSO (dimethyl sulfoxide).

Certain compounds of Formula (1) and their salts may exist in more than one crystal form. Polymorphs of compounds represented by Formula (1) form part of this invention and may be prepared by crystallization of a compound of Formula (1) under different conditions. For example, using different solvents or different solvent mixtures for recrystallization; crystallization at different temperatures; various modes of cooling, ranging from very fast to very slow cooling during crystallization. Polymorphs may also be obtained by heating or melting a compound of Formula (1) followed by gradual or fast cooling. The presence of polymorphs may be determined by solid probe nuclear magnetic resonance (NMR) spectroscopy, infrared (IR) spectroscopy, differential scanning calorimetry, powder X-ray diffraction or such other techniques.

This invention also includes isotopically-labeled compounds, which are identical to those described by Formula (1), but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, sulfur and fluorine, such as ²H, ³H, ¹³C, ¹⁴C, ¹⁵N, ¹⁸O, ¹⁷O, ³⁵S, ³⁶Cl, ¹²⁵I, ¹²⁹I, and ¹⁸F respectively. Compounds of the present invention, and pharmaceutically acceptable salts of the compounds which contain the aforementioned isotopes and/or other isotopes of other atoms are within the scope of this invention. Certain isotopically-labeled compounds of the present invention, for example those into which radioactive isotopes such as ³H and ¹⁴C are incorporated, are useful in drug and/or substrate tissue distribution assays. Tritiated (i.e., ³H), and carbon-14 (i.e., ¹⁴C), isotopes are particularly preferred for their ease of preparation and detectability. Further, substitution with heavier isotopes such as deuterium (i.e., ²H), can afford certain therapeutic advantages resulting from greater metabolic stability, for example increased *in vivo* half-life or reduced dosage requirements and, hence, may be preferred in some circumstances. Isotopically labeled compounds of Formula (1) of this invention and salts thereof can generally be prepared by carrying out the procedures disclosed in the schemes and/or in the Examples below, by substituting a readily available isotopically labeled reagent for a non-isotopically labeled reagent.

The compounds of the present invention may be isolated and used *per se* or in the form of their pharmaceutically acceptable salts. In accordance with the present invention, compounds with multiple basic nitrogen atoms can form salts with varying number of equivalents ("eq.") of acid. It will be understood by practitioners that all such salts are within the scope of the present invention.

The present invention further describes prodrugs of compounds of Formula (1). A prodrug of a compound of Formula (1) may be one formed in a conventional manner with a functional group of the compound, such as with an amino, hydroxy or carboxy group. The term "prodrug" means a compound that is transformed *in vivo* to yield a compound of Formula (1) or a pharmaceutically acceptable salt of the compound. The transformation may occur by various mechanisms, such as through hydrolysis in blood. A discussion of the use of prodrugs is provided by T. Higuchi and W. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

For example, as many of the compounds of the present invention incorporate an amine functional group, a prodrug can be formed by the replacement of a hydrogen atom in the amine group with a group such as R-carbonyl, RO-carbonyl, NRR'-carbonyl where R and R' are each independently (C₁-C₁₀)alkyl, (C₃-C₇)cycloalkyl, benzyl, or R-carbonyl is a natural α-aminoacyl or natural α-aminoacyl-natural α-aminoacyl, -C(OH)C(O)OY' wherein Y' is H, (C₁-C₆)alkyl or benzyl, -C(OY₀)Y₁ wherein Y₀ is (C₁-C₄) alkyl and Y₁ is (C₁-C₆)alkyl, carboxy(C₁-C₆)alkyl, amino(C₁-C₄)alkyl or mono-N- or di-N,N-(C₁-C₆)alkylaminoalkyl, -C(Y₂)Y₃ wherein Y₂ is H or methyl and Y₃ is mono-N- or di-N,N-(C₁-C₆)alkylamino, morpholino, piperidin-1-yl or pyrrolidin-1-yl.

Similarly, if a compound of the present invention contains an alcohol functional group, a prodrug can be formed by the replacement of the hydrogen atom of the alcohol group with a group such as (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, P(O)(OH)₂, - P(O)(O(C₁-C₆)alkyl)₂ or glycosyl (the radical resulting from the removal of a hydroxyl group of the hemiacetal form of a carbohydrate).

If a compound of the present invention contains a carboxylic acid functional group, a prodrug can comprise an ester formed by the replacement of the hydrogen atom of the acid group with a group such as (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamin_{O}(C₂-C₃)alkyl (such as β-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl.

### Synthesis

In general, the compounds of Formula (1) of this invention may be prepared by methods that include processes known in the chemical arts, particularly in light of the description contained herein. Certain processes for the manufacture of the compounds of Formula (1) of this invention are illustrated by the following reaction schemes. Other processes are described in the experimental section. Some of the starting compounds for the reactions described in the schemes and Examples are prepared as illustrated herein.

The compounds of Formula (1), wherein R¹, R², R³, R⁴ R⁵, R⁶, R⁷, R⁸ and R⁹ are as defined above, may be prepared, as shown below in Scheme A.

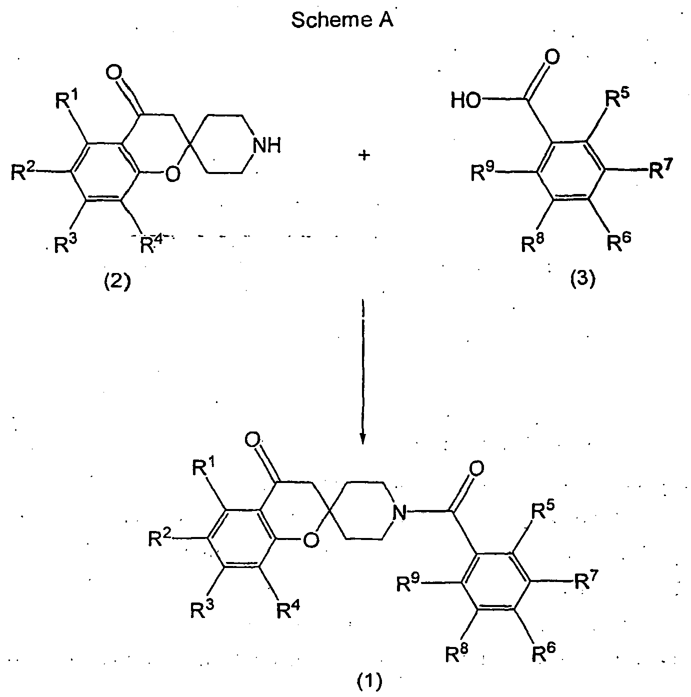

In Scheme A, a compound of Formula (1) is formed by coupling a spirocyclic ketone (2) with a carboxylic acid (3) while in solution. The spirocyclic ketone (2) and carboxylic acid (3) may be coupled by forming an activated carboxylic acid ester, such as by contacting the carboxylic acid (3) with a peptide coupling reagent, such as 2-(7-aza-1H-benzotriazole-1-yl)-1,1,3,3-tetramethyluronium hexafluorophosphate (HATU), in the presence of an activating agent, such as N,N-diisopropylethylamine (DIEA) and then contacting the activated carboxylic acid ester with the spirocyclic ketone (2) to form a compound of Formula (1). Alternately, compounds of Formula (1) can be formed by first converting the carboxylic acid (3) to an acid chloride, such as by reacting with thionyl chloride, and then reacting the acid chloride with the spirocyclic ketone (2) to form a compound of Formula (1).

The synthesis of compounds of Formula (2), and their starting materials, may be prepared as described in Schemes B through E.

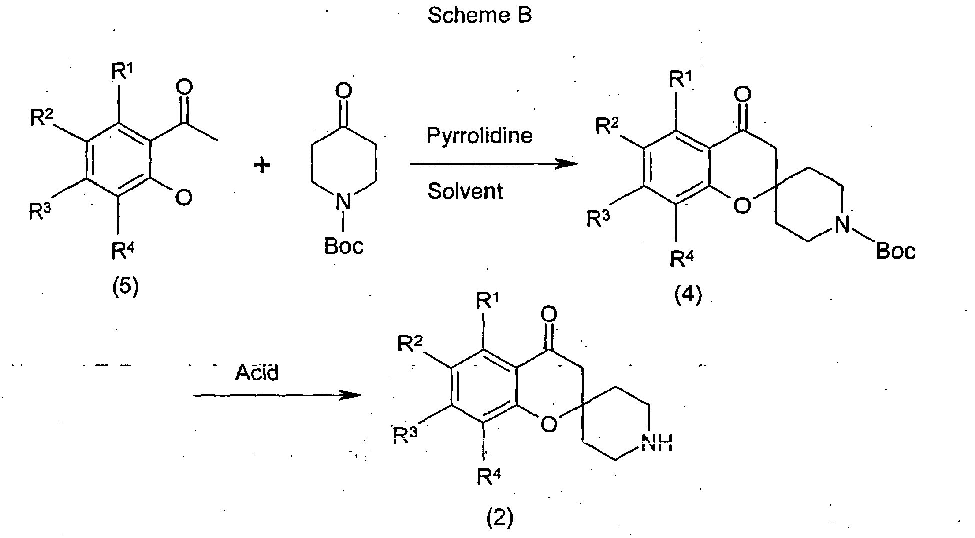

To prepare spiroketone (2), a solution of a substituted or unsubstituted 1-(2-hydroxyphenyl)ethanone (5), tert-butyl 4-oxopiperidine-1-carboxylate and pyrrolidine (1:1:1 molar ratio) in a solvent, such as methanol, is stirred for 24 hours at a temperature ranging from room temperature to reflux and evaporated to afford an N-Boc-Spiro[chromene-2,4'-piperidin]-4(3H)-one (4). An acid, such as HCl or trifluoroacetic acid is then added to a solution of the N-Boc-Spiro[chromene-2,4'-piperidin]-4(3H)-one (4) in solvent, such as dichloromethane, isopropanol or dioxane, with subsequent stirring, to deprotect the amine by removing the Boc (*t*-butyloxycarbonyl) group to form a spirocyclic ketone (2).

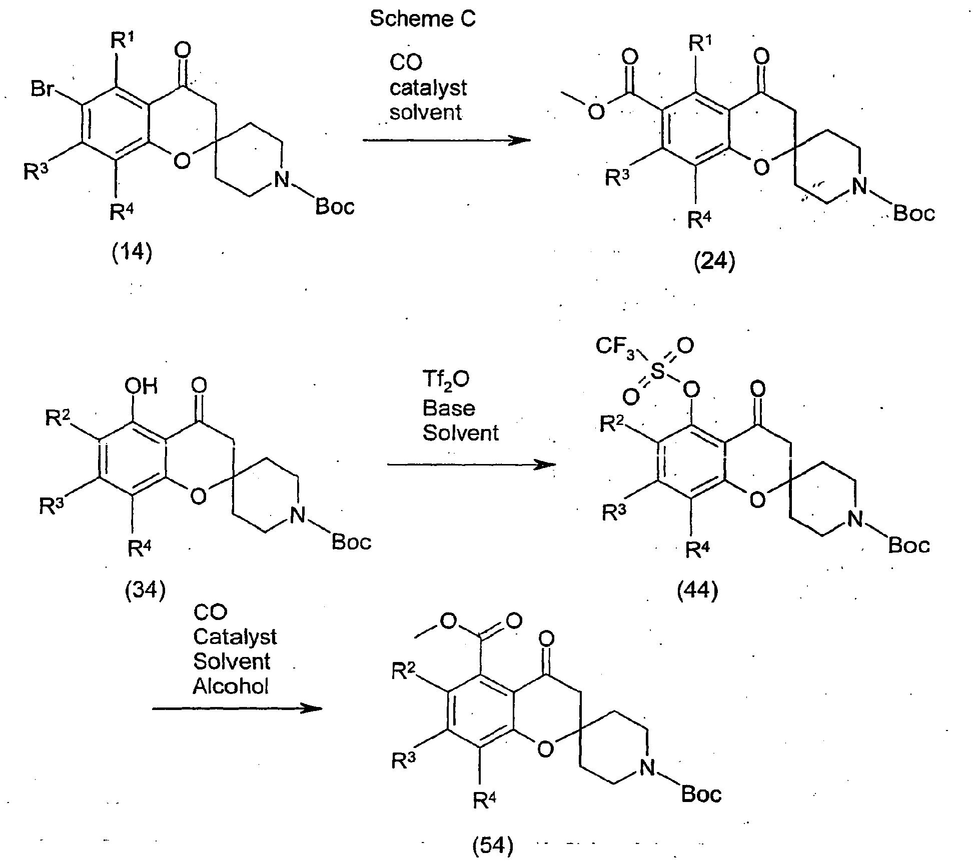

Alternatively, an N-Boc-spiro[chromene-2,4'-piperidin]-4(3H)-one-may be derivatized as shown in Scheme C. For instance, treating a N-Boc-spiro[chromene-2,4'-piperidin]-4(3H)-one wherein one of R¹, R², R³ or R⁴ is a bromo-group (14), with carbon monoxide in the presence of a suitable catalyst, such as dichlorobis(triphenylphosine)palladium II, a base such as triethylamine and in the presence of an alcohol solvent, such as methanol, provides the methyl ester N-Boc-spiro[chromene-2,4'-piperidin]-4(3H)-one derivatives (24). An alternate preparation entails subjecting a solution of a hydroxyl-N-Boc-spiro[chromene-2,4'-piperidin]-4(3H)-one (34) derivative compound in an inert solvent, such as methylene chloride, to triflic anhydride in the presence of a suitable base, such as pyridine provides the desired triflate N-Boc-spiro[chromene-2,4'-piperidin]-4(3H)-one derivative (44). Treating a solution of the triflate in a solvent such as dimethylformamide with a suitable catalyst mixture, such as palladium acetate and 1,1 - bis(diphenylphosphino)ferrocene, in the presence of a suitable base, such as triethylamine in the presence of an alcohol, such as methanol, with carbon monoxide affords the desired methyl ester (54).

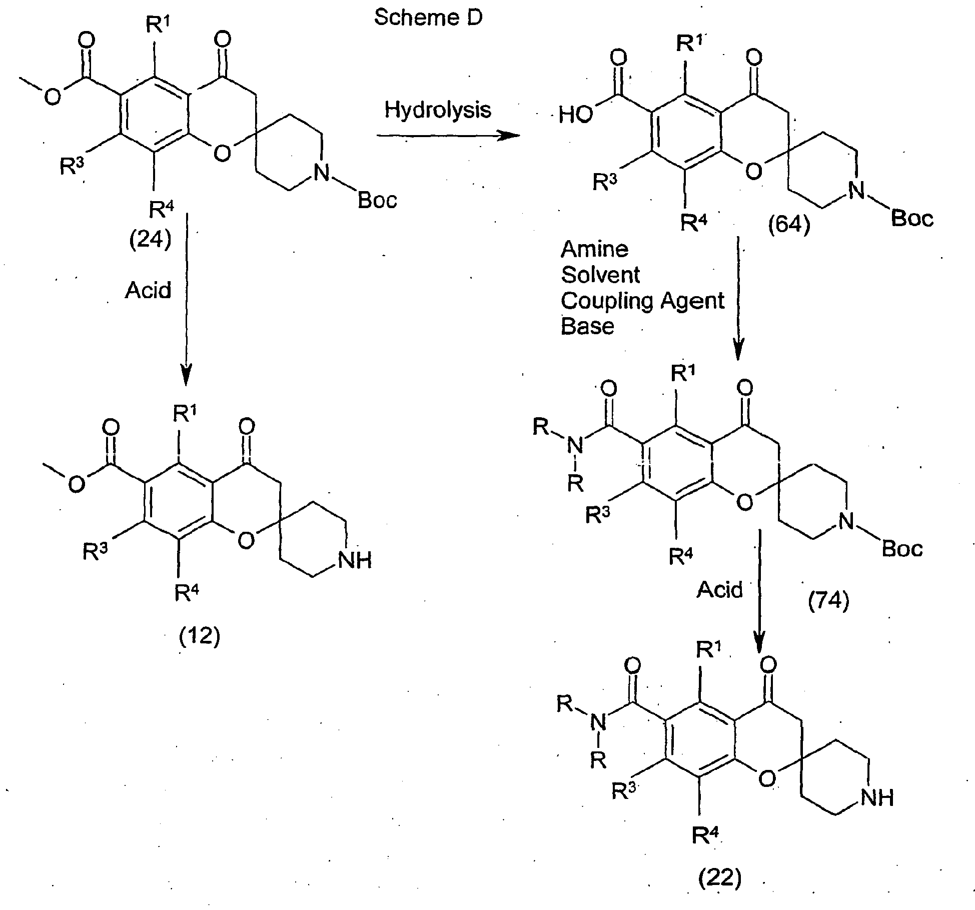

Spirocyclic esters prepared via methods described in Schemes B and C can be further derivatized to afford amides, esters and acids as shown in Scheme D. Acid mediated removal of the N-Boc protecting group from the methyl ester affords ester derivatized spirocycle (12). Alternatively, saponification of the N-Boc protected spirocycle provide the carboxylic acid intermediate (64). Amines can be coupled to the carboxylic acid utilizing methods known to those skilled in the art to provide amide derivatives (74) which, when treated with acid, afford the desired spirocyclic amines (22).

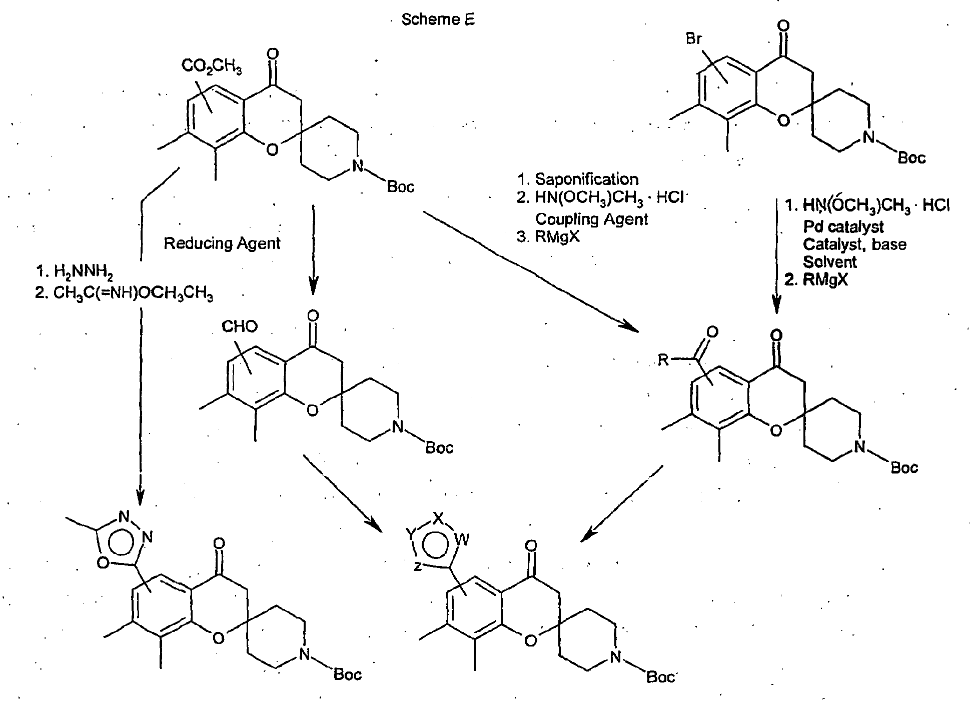

Spirocyclic esters prepared via methods described in Schemes B and C can be further derivatized into the corresponding aldehyde utilizing methods known to those skilled in the art as shown in Scheme E. The aldehyde can be converted into various five-membered heterocycles via methods described in Tanaka, A.; et al., J. Med. Chem. 1998, 41, 2390 - 2410. Alternatively, an aryl bromide can be converted directly into a Weinreb amide utilizing a known protocol (Buchwald, S. L., et al., Org. Lett. 2006, online preprint). The resulting Weinreb amide can be converted into the desired ketone using methods known to those skilled in the art. The resultant ketones can be transformed into various five-membered heterocycles via methods described in Tanaka, A.; et al., J. Med. Chem. 1998, 41, 2390-2410.

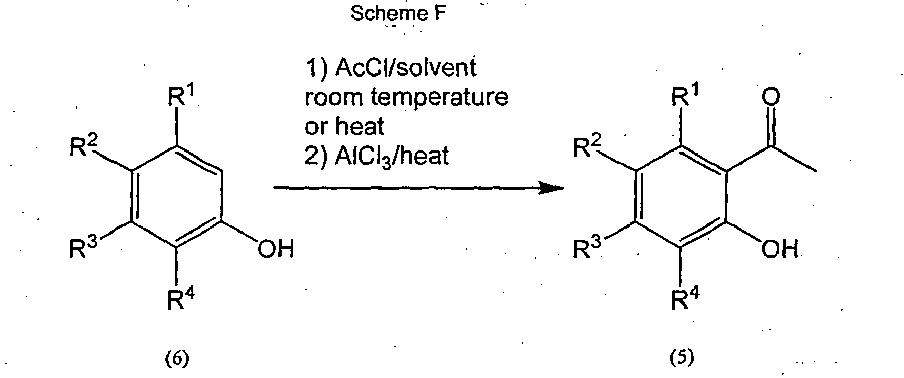

To prepare ortho-hydroxyacetophenone (5), an acetylation reagent, such as acetyl chloride or acetic anhydride, is added to phenol (6) and the reaction mixture stirred at a temperature between room temperature and reflux. Excess acetylation reagent is removed *in vacuo*, and then the O-acetylated phenol is treated with AlCl₃ and heated to a high temperature, such as 180° C. The reaction mixture is then cooled, quenched with aqueous acid, such as dilute hydrochloric acid, and filtered to afford the desired ortho-hydroxyacetophenone.

A pharmaceutical composition of the present invention comprises a therapeutically effective amount of a compound of Formula (1), or a pharmaceutically acceptable salt of the compound, and a pharmaceutically acceptable carrier, vehicle, diluent or excipient.

In one preferred embodiment of a pharmaceutical composition of the present invention wherein R⁵ and R⁷ are taken together, said polycyclic heterocyclic radical is 1H-indazolyl, 1H-benzoimidazolyl, quinolyl, 1 ,2,3 ,4-tetrahydroquinolyl, quinoxlayl, 1H-indolyl, 2,3-dihydro-1H-benzoimidazolyl, 1H-benzo-[d][1,2,3]triazolyl, 6,7,8,9-tetrahydro-5H-carbazolyl, 2,3,4,9-tetrahydro-1H-pyrido-[3,4-b]indolyl or benzooxazolyl. The nitrogen-bearing ring of said polycyclic heterocyclic radical is optionally substituted.

In another preferred embodiment of a pharmaceutical composition of the present invention wherein R⁵ and R⁶ are taken together, said polycyclic heterocyclic radical is 1H-indazolyl, 1H-benzoimidazolyl, 1H-indolyl or 2,3-dihydro-1H-benzoimidazolyl fused to cyclohexene, 5,6-dihydro-pyridine or 5,6-dihydro-1H-pyridin-2-one. The nitrogen-bearing ring of said polycyclic heterocyclic radical is optionally substituted.

In yet another preferred embodiment of a pharmaceutical composition of the present invention wherein R⁵ is taken separately and is optionally substituted pyrazolyl, imidazolyl, oxadiazolyl or pyrimidinyl.

The pharmaceutical compositions formed by combining the compounds of this invention and the pharmaceutically acceptable carriers, vehicles or diluents are then readily administered in a variety of dosage forms such as tablets, powders, lozenges, syrups, injectable solutions and the like. These pharmaceutical compositions can, if desired, contain additional ingredients such as flavorings, binders, excipients and the like.

Thus, for purposes of oral administration, tablets containing various excipients such as sodium citrate, calcium carbonate and/or calcium phosphate, may be employed along with various disintegrants such as starch, alginic acid and/or certain complex silicates, together with binding agents such as polyvinylpyrrolidone, sucrose, gelatin and/or acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the active pharmaceutical agent therein may be combined with various sweetening or flavoring agents, coloring matter or dyes and, if desired, emulsifying or suspending agents, together with diluents such as water, ethanol, propylene glycol, glycerin and/or combinations thereof.

For parenteral administration, solutions of the compounds or compositions of this invention in sesame or peanut oil, aqueous propylene glycol, or in sterile aqueous solutions may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, the sterile aqueous media employed are all readily available by standard techniques known to those skilled in the art.

For intranasal administration or administration by inhalation, the compounds or compositions of the invention are conveniently delivered in the form of a solution or suspension from a pump spray container that is squeezed or pumped by the patient or as an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain, a solution or suspension of a compound of this invention. Capsules and cartridges (made, for example, from gelatin) for use in an inhaler or insufflator may be formulated containing a powder mix of a compound or compounds of the invention and a suitable powder base such as lactose or starch.

Methods of preparing various pharmaceutical compositions with a certain amount of active ingredient are known, or will be apparent in light of this disclosure, to those skilled in this art. For examples of methods of preparing pharmaceutical compositions, see Remington's Pharmaceutical Sciences, Mack Publishing Company, Easton, Pa., 19th Edition (1995).

The present invention also relates to therapeutic methods for treating or preventing overweight or obese conditions in a mammal, including a human, wherein a compound of Formula (1) of this invention, or a salt thereof, is administered as part of an appropriate dosage regimen designed to obtain the benefits of the therapy. The appropriate dosage regimen, the amount of each dose administered and the intervals between doses of the compound will depend upon the compound of Formula (1) of this invention being used, the type of pharmaceutical compositions being used, the characteristics of the subject being treated and the severity of the conditions.

In general, an effective dosage for the compounds, and salts, of the present invention is in the range of 0.001 milligram(mg)/kg/day to 100 mg/kg/day, preferably 0.01 mg/kg/day to 10 mg/kg/day of active compound in single or divided doses. Some variation in dosage will necessarily occur, however, depending on the condition of the subject being treated. The individual responsible for dosing will, in any event, determine the appropriate dose for the individual subject. Practitioners will appreciate that "kg" refers to the weight of the patient measured in kilograms. Doses currently envisaged for human use range from 10-300 mg/kg. Compounds with increased potency and or improved pharmacodynamics would possess lower dose requirements, typically 0.1 10 mg/kg.

The compounds or compositions of this invention may be administered in single (e.g., once daily) or multiple doses or via constant infusion. The compounds of this invention may also be administered alone or in combination with pharmaceutically acceptable carriers, vehicles or diluents, in either single or multiple doses. Suitable pharmaceutical carriers, vehicles and diluents include inert solid diluents or fillers, sterile aqueous solutions and various organic solvents.

The compounds or compositions of the present invention may be administered to a subject in need of treatment by a variety of conventional routes of administration, including orally and parenterally, (e.g., intravenously, subcutaneously or intramedullary). Further, the pharmaceutical compositions of this invention may be administered intranasally, as a suppository, or using a "flash" formulation, i.e., allowing the medication to dissolve in the mouth without the need to use water.

### EXEMPLIFICATION

The Examples set forth herein below are for illustrative purposes only. The compositions, methods, and various parameters reflected herein are intended only to exemplify various aspects and embodiments of the invention, and are not intended to limit the scope of the claimed invention in any way.

Unless noted otherwise, all reactants were obtained commercially.

Flash chromatography was performed according to the method described by Still et al., J. Org. Chem., 1978, 43, 2923.

All Biotage® purifications, discussed herein, were performed using either a 40M or 40S Biotage® column containing KP-SIL silica (40-63 µM, 60 Angstroms) (Bioatge AB; Uppsala, Sweden).

All Combiflash® purifications, discussed herein, were performed using a CombiFlash® Companion system (Teledyne Isco; Lincoln, Nebraska) utilizing packed RediSep® silica columns

Mass Spectra were recorded on a Waters (Waters Corp.; Milford, MA) Micromass Platform II spectrometer. Unless otherwise specified, mass spectra were recorded on a Waters (Milford, MA) Micromass Platform II spectrometer.

Proton NMR chemical shifts are given in parts per million downfield from tetramethylsilane and were recorded on a Varian Unity 400 MHz (megaHertz) spectrometer (Varian Inc.; Palo Alto, CA). NMR chemical shifts are given in parts per million downfield from tetramethylsilane (for proton) or fluorotrichloromethane (for fluorine).

The following preparations were used in the synthesis of compounds of the present invention which are further exemplified in the following examples.

### Spirocyclic Ketones

Spirocyclic ketones, which were used to prepare exemplified compounds of the present invention, were prepared using the method of one of the following Spirocyclic Ketone Preparations 1-25.

### Spirocyclic Ketone Preparation 1

### 7-Methoyspiro[chromene-2,4'-piperidin]-4(3H)-one

To a solution of 1-(2-hydroxy-4-methoxyphenyl)ethanone (Acros Organics USA, Morris Plains, NJ) (83 milligrams ("mg"), 0.5 millimoles ("mmol") in methanol (1 milliliter ("mL")) was added tert-butyl 4-oxopiperidine-1-carboxylate (111 mg, 0.56 mmol) and pyrrolidine (42.5 microliters ("µL"), 0.51 mmol). The mixture was heated at reflux overnight. The mixture was cooled to room temperature, concentrated and purified by Biotage chromatography (8% acetone/heptane) to afford N-Boc-7 methoxyspiro[chromene-2,4'-piperidin]-4(3H)-one as a yellow solid (89 mg, 51%), 248(M-Boc, ES+). To a solution of N-Boc-7-methoxyspiro[chromene-2,4'-piperidin]-4(3H)-one (58 mg, 0.17 mmol) in methanol (1 mL) was added 4 N HCl in dioxane (0.40 mL). The mixture was stirred at room temperature for 3 hours. The mixture was concentrated to afford 7-methoxyspiro[chromene-2,4'-piperidin]-4(3H)-one hydrochloride which was used without further purification (45 mg, 95%), 248 (ES+).

### Spirocyclic Ketone Preparation 2

### 6-Methoxyspiro[chromene-2,4'-pipereridin]-4(3H)-one

To a solution of 1-(2-hydroxy-5-methoxyphenyl)ethanone (831 mg, 5.0 mmol) in toluene (5 mL) was added tert-butyl 4-oxopiperidine-1-carboxylate (1.20 grams ("g"), 6.0 mmol) and pyrrolidine (356 mg, 5.0 mmol). The mixture was heated at reflux overnight. The mixture was cooled to room temperature, concentrated and purified by Biotage chromatography (8% acetone/heptane) to afford N-Boc-6-methoxyspiro[chromene-2,4'-piperidin]-4(3H)-one as a yellow solid (1.27 g, 70%).
¹H NMR (CDCl₃) δ 7.27 (d, J=3, 1H), 7.10 (dd, J=9, 3, 1H), 6.91 (d, J=9, 1H), 3.84 (m, 2H), 3.78 (s, 3H), 3.18 (t, J=12, 2 H), 2.68 (br s, 2H), 2.01 (d, J=13, 2H), 1.57 (m, 2H), 1.44 (s, 9H).

To a solution of N-Boc-6-methoxyspiro[chromene-2,4'-piperidin]-4(3H)-one (42 mg, 0.12 mmol) in methanol (1 mL) was added 4 N HCl in dioxane (0.30 mL). The mixture was stirred at room temperature for 90 minutes. The mixture was concentrated to afford 6-chloro-7-methylspiro[chromene-2,4'-piperidin]-4(3H)-one which was used without further purification (34 mg, 100%).

### Spirocyclic Ketone Preparation 3

### 6-Chloro-7-methylspiro[chromene-2,4'-piperidin]-4(3H)-one

To a solution of 1-(5-chloro-2-hydroxy-4-methylphenyl)ethanone (371 mg, 2.0 mmol) in benzene (2 mL) was added tert-butyl 4-oxopiperidine-1-carboxylate. The mixture was heated at reflux overnight. The mixture was cooled to room temperature, concentrated and purified by Biotage chromatography (10% acetone/heptane) to afford N-Boc-6-chloro-7-methylspiro[chromene-2,4'-piperidin]-4(3H)-one as a yellow solid (670 mg, 91%). N-Boc-6-Chloro-7-methylspiro[chromene-2,4'-piperidin]-4(3H)-one, 91%, ¹H NMR (CDCl₃) δ 7.79 (s, 1H), 6.87 (s, 1H), 3.88 (m, 2H), 3.17 (m, 2H), 2.66 (s, 2H), 2.42 (m, 1H), 2.36 (s, 3H), 1.98 (m, 2H), 1.58 (m, 2H), 1.44(s, 9H).

To a solution of N-Boc-6-chloro-7-methylspiro[chromene-2,4'-piperidin]-4(3H)-one (60 mg, 0.16 mmol) in MeOH (1 mL) was added 4 N HCl in dioxane (0.40 mL). The mixture was stirred at room temperature for 4 hours. The mixture was concentrated to afford 6-chloro-7-methylspiro[chromene-2,4'-piperidin]-4(3H)-one hydrochloride which was used without further purification (50 mg, 100%), 266 (ES+)

### Spirocyclic Ketone Preparation 4

### 5,6,7-Trimethoxyspiro[chromene-2,4'-piperidin]-4(3H)-one

To a solution of 1-(6-hydroxy-2,3,4-trimethoxyphenyl)ethanone (452 mg, 2.0 mmol) in benzene (2 mL) was added tert-butyl 4-oxopiperidine-1-carboxylate (438 mg, 2.2 mmol) and pyrrolidine (0.2 mL, 2 mmol). The mixture was heated at reflux overnight. The mixture was cooled to room temperature, concentrated and purified by Biotage chromatography (15% acetone/heptane) to afford N-Boc-5,6,7-trimethoxyspiro-[chromene-2,4'-piperidin]-4(3H)-one as a yellow-brown solid (203 mg, 33%), 308 (ES+).

To a solution of N-Boc-5,6,7-trimethoxyspiro[chromene-2,4'-piperidin]-4(3H)-one (60 mg, 0.15 mmol) in methanol (1 mL) was added 4 N HCl in dioxane (0.40 mL). The mixture was stirred at room temperature for 4 hours. The mixture was concentrated to afford 5,6,7-trimethoxyspiro[chromene-2,4'-piperidin]-4(3H)-one hydrochloride which was used without further purification (53 mg, 100%), 308 (ES+).

### Spirocyclic Ketone Preparation 5

### 6-Chloro-5-methoxyspiro[chromene-2,4'-piperidin]-4(3H)-one

To a solution of 2-hydroxy-6-methoxyacetophenone (500 mg, 3.0 mmol) in diethyl ether (4.5 mL) at 0 °C was added sulfuryl chloride (0.27 mL, 3.4 mmol) drop wise: The resulting mixture was heated at reflux for 4 hours before cooling to room temperature. The diethyl ether solution was washed twice with water, the organic phase was separated and concentrated. The material was purified by Biotage chromatography (40 S column, 6% acetone/heptane) to provide 1-(3-chloro-6-hydroxy-2-methoxyphenyl)ethanone as a yellow liquid (563 mg, 93%). ES- m/z 199 (M-, 100%), ¹H NMR (CDCl₃) δ 12.68 (s, 1H), 7.41 (d, J=9.2, 1H), 6.72 (d, J=9.2, 1H), 3.92 (s, 3H), 2.74 (s, 3H).

To a solution of 1-(3-chloro-6-hydroxy-2-methoxyphenyl)ethanone (563 mg, 2.8 mmol) in methanol (3 mL) was added tert-butyl 4-oxopiperidine-1-carboxylate (623 mg, 3.13 mmol) and pyrrolidine (0.24 mL, 2.9 mmol). The mixture was heated at reflux overnight. The mixture was cooled to room temperature, concentrated and purified by Biotage chromatography (7 - 10% acetone/heptane) to afford N-Boc-6-chloro-5-methoxyspiro[chromene-2,4'-piperidin]-4(3H)-one as a red solid (670 mg, 63%), ¹H NMR (CDCl₃) δ 7.45 (d, J=9, 1H), 6.74 (d, J=9, 1H), 3.88 (s, 3H), 3.18 (m, 3H), 2.68 (s, 3H), 2.43 (t, J=5, 1H), 1.97 (br d, J=13, 2H), 1.60 (m, 4H), 1.44 (s, 9H).

To a solution of N-Boc-6-chloro-5-methoxyspiro[chromene-2,4'-piperidin]-4(3H)-one (64 mg, 0.17 mmol) in MeOH (1 mL) was added 4 N HCl in dioxane (0.40 mL). The mixture was stirred at room temperature for 3 hours. The mixture was concentrated to afford 6-chloro-5-methoxyspiro[chromene-2,4'-piperidin]-4(3H)-one which was used without further purification (56 mg, 100%), 282 (ES+).

### Spirocyclic Ketone Preparation 6

### Methyl 4-oxo-3,4-dihydrospiro[chromene-2,4'-piperidin]-6-carboxylate hydrochloride

(Step 1) To a solution 1-(5-bromo-2-hydroxyphenyl)ethanone (2.0 g, 9.3 mmol) in methanol (20 mL) was added pyrrolidine (0.8 mL, 9.6 mmol) and tert-butyl 4-oxopiperidine-1-carboxylate (1.91 g, 9.6 mmol). The mixture was stirred at room temperature overnight. The reaction mixture was concentrated and purified by Biotage chromatography (40M column, 8% - 20% ethyl acetate/heptane gradient) to provide tert-butyl 6-bromo-4-oxo-3,4-dihydro-1H-spiro[chromene2,4-piperidine]-1-carboxylate as a yellow solid (3.09 g, 84%). ¹H NMR (CDCl₃) δ 7.96 (d, J=2.5, 1H), 7.56 (dd, J=8.7, 2.5, 1H), 6.89 (d, J=8.7, 1H), 2.70 (s, 2H), 1.44 (s, 9H).
(Step 2) To a solution of tert-butyl 6-bromo-4-oxo-3,4-dihydro-1H-spiro[chromene-2,4-piperidine]-1-carboxylate (0.8 g, 2 mmol) in methanol (60 mL) was added triethylamine (0.32 mL) and dichlorobis(triphenylphosphine)palladium II (144 mg, 0.21 mmol). The mixture was heated at 80 °C under 50 psi carbon monoxide for 2 days. The mixture was cooled to room temperature, filtered through diatomaceous earth and purified by Biotage chromatography (40 S column, 15% EtoAc/heptane) to yield N-Boc Methyl 4-oxo-3,4-dihydrospiro[chromene-2,4'-piperidine]-6-carboxylate as a yellow solid (677 mg, 90%). ¹H NMR (CDCl₃) δ 8.55 (d, J=2.1, 1H), 8.16 (dd, J=8.8, 2.1, 1H), 7.04 (d, J=8.7, 1H), 3.90 (s, 3H), 2.76 (s, 2H), 1.46 (s, 9H).
(Step 3) To a solution of N-Boc Methyl 4-oxo-3,4-dihydrospiro[chromene-2,4'-piperidin]-6-carboxylate (225 mg, 0.60 mmol) in methanol (4 mL) was added 4 N HCl in dioxane (1.5 ml). The mixture was stirred at room temperature for 3 hours, concentrated to yield the title compound as a yellow solid (195 mg, 100%). MS (ACPI) *m*/*z* 276 (M+H)⁺, HPLC Retention Time ("RT") 1.0 minutes.

### Spirocyclic Ketone Preparation 7

### 1-(Tert-butoxycarbonyl)-4-oxo-3,4-dihydrospiro[chromene-2,4'-piperidin]-6-carboxylic acid

To a solution of N-Boc methyl 4-oxo-3,4-dihydrospiro[chromene-2,4'-piperidin]-6-carboxylate, from Spiroketone Preparaton 6, (375 mg, 1.0 mmol) in methanol/water (1:1 ratio, 5 mL) was added lithium hydroxide (49 mg). The mixture was heated at 50 °C for 2 hours before cooling to room temperature. The mixture was concentrated, diluted with water and acidified with KHSO₄ to pH 3. The precipitate that formed was extracted with EtOAc, dried over Na₂SO₄, filtered and concentrated to provide the title compound as a yellow solid (260 mg, 72%). MS (ACPI) *m*/*z* 360 (M-H)⁻, HPLC RT 2.4 minutes. ¹H NMR (CDCl₃) δ 8.63 (2.0, 1H), 8.20 (dd, J=8.7, 2.5, 1H), 6.69 (d, J=8.8, 1H), 2.76 (s, 2H), 1.45 (s, 9H).

### Spirocyclic Ketone Preparation 8

### 6-(Pyrrolidin-1-ylcarbonyl)spiro[chromene-2,4 -piperidin]-4(3H)-one

(Step 1) To a solution of 1-(tert-butoxycarbonyl)-4-oxo-3,4-dihydrospiro[chromene-2,4'-piperidin]-6-carboxylate (54 mg, 0.15 mmol) in CH₂Cl₂ (1 mL) was added pyrrolidine (17 mg, 20 µL, 0.24 mmol), O-(7-azabenzotriazol-1-yl)-N,N,N',N'-tetramethyluronium hexafluorophosphate (HATU) (60 mg, 0.16 mmol) and triethylamine (50 µL, 0.36 mmol). The mixture was stirred at room temperature overnight. The mixture was then concentrated and the crude material was dissolved in EtOAc, washed with water and the organic extract was concentrated to yield the title compound as a sticky gum that was used as is without further purification (79 mg). MS (ACPI) *m*/*z* 415 (M+H)⁺, HPLC RT 2.3 minutes.
(Step 2) To a solution of tert-butyl 4-oxo-6-(pyrrolidin-1-ylcarbonyl)-3,4-dihydro-1H-spiro[chromene-2,4-piperidine]-1-carboxylate (62 mg, 0.15 mmol) in methanol (0.5 mL) was added 4 N HCl in dioxane (0.15 mL). The mixture was stirred at room temperature for 2 hr and triethylamine (80 µL) was added to neutralize the acid and the mixture was concentrated to provide the crude product which was used without further purification. MS (ACPI) *m*/*z* 315 (M+H)⁺, HPLC RT 0.3 minutes.

### Spirocyclic Ketone Preparation 9

### N-Isopropyl-4-oxo-3,4-dihydrospiro[chromene-2,4-piperidine]-6-carboxylate

Following the procedure described in Spirocyclic Ketone Preparation 8, substituting isopropylamine, afforded tert-butyl 6-[(isopropylamino)carbonyl]-4-oxo-3,4-dihydro-1H-spiro[chromene-2,4-piperidine]-1-carboxylate as a gum that was used without further purification (89 mg). MS (ACPI) *m*/*z* 403 (M+H)⁺, HPLC RT 2.5 minutes.

The title compound was prepared from tert-butyl 6-[(isopropylamino)carbonyl]-4-oxo-3,4-dihydro-1H-spiro[chromene-2,4-piperidine]-1-carboxylate as described in Spirocyclic Ketone Preparation 8(step 2). MS (ACPI) *m*/*z* 303 (M+H)⁺, HPLC RT 0.6 minutes.

### Spirocyclic Ketone Preparation 10

### N,N-Dimethyl-4-oxo-3,4-dihydrospiro[chromene-2,4-piperidine]-6-carboxylate hydrochloride

Following the procedure described in Spirocyclic Ketone Preparation 8, substituting dimethylamine, afforded tert-butyl 6-[(dimethylamino)carbonyl]-4-oxo-3,4-dihydro-1H-spiro[chromene-2,4-piperidine]-1-carboxylate as a yellow solid that was used without further purification (58 mg). MS (ACPI) *m*/*z* 389 (M+H)⁺, HPLC RT 2.2 minutes.

The title compound was prepared from tert-butyl 6-[(dimethylamino)carbonyl]-4-oxo-3,4-dihydro-1H-spiro[chromene-2,4-piperidine]-1-carboxylate as described in Spirocyclic Ketone Preparation 8 (step 2), except no triethylamine was added. MS (ACPI) *m*/*z* 289 (M+H)⁺, HPLC RT 0.2 minutes.

### Spirocyclic Ketone Preparation 11

### 6-(Morpholin-4-ylcarbonyl)spiro[chromene-2,4-piperidin]-4(3H)-one

Following the procedure described in Spirocyclic Ketone Preparation 8, substituting morpholine, afforded tert-butyl 6-(morpholin-4-ylcarbonyl)-4-oxo-3,4-dihydro-1H-spiro[chromene-2,4-piperidine]-1-carboxylate as a yellow solid that was used without further purification (56 mg). MS (ACPI) *m*/*z* 431 (M+H)⁺, HPLC RT 2.3 minutes.

The title compound was prepared from tert-butyl 6-(morpholin-4-ylcarbonyl)-4-oxo-3,4-dihydro-1H-spiro[chromene-2,4-piperidine]-1-carboxylate as described in Spirocyclic Ketone Preparation 8 (step 2), except no triethylamine was added. MS (ACPI) *m*/*z* 331 (M+H)⁺, HPLC RT 0.3 minutes.

### Spirocyclic Ketone Preparation 12

### N-Methyl-4-oxo-3,4-dihydrospiro[chromene-2,4-piperidine]-6-carboxylamide

Following the procedure described in Spirocyclic Ketone Preparation 8, substituting methylamine, afforded tert-butyl 6-[(methylamino)carbonyl]-4-oxo-3,4-dihydro-1H-spiro[chromene-2,4-piperidine]-1-carboxylate as a yellow solid that was used without further purification (52 mg). MS (ACPI) *m*/*z* 375 (M+H)⁺, HPLC RT 1.8 minutes.

The title compound was prepared from tert-butyl 6-[(methylamino)carbonyl]-4-oxo-3,4-dihydro-1H-spiro[chromene-2,4-piperidine]-1 -carboxylate as described in Spirocyclic Ketone Preparation 8 (step 2). MS (ACPI) *m*/*z* 275 (M+H)⁺, HPLC RT 0.3 minutes.

### Spirocyclic Ketone Preparation 13

### 4-Oxo-3,4-dihydrospiro[chromene-2,4-piperidine]-6-carboxylamide

A vial was charged with N-Boc methyl 4-oxo-3,4-dihydrospiro[chromene-2,4'-piperidin]-6-carboxylate (56.mg, 0.15 mmol), prepared as described in Spirocyclic Ketone Preparation 6 (step 2), and ammonium hydroxide (1 mL). The mixture was heated at 65 °C overnight. The reaction mixture was cooled to room temperature and concentrated to yield tert-butyl 6-(aminocarbonyl)-4-oxo-3,4-dihydro-1H-spiro[chromene-2,4-piperidine]-1-carboxylate (56 mg, 100%). MS (ACPI) *m*/*z* 361 (M+H)⁺.

The title compound was prepared from tert-butyl 6-(aminocarbonyl)-4-oxo-3,4-dihydro-1H-spiro[chromene-2,4-piperidine]-1-carboxylate as described in_Spirocyclic Ketone Preparation 8.
MS (ACPI) *m*/*z* 261 (M+H)⁺, HPLC RT 1.1 minutes.

### Spirocyclic Ketone Preparation 14

### 6-Isopropoxyspiro[chromene-2,4'-piperidin]-4(3H)-one hydrochloride

A solution of 1-(2,5-dihydroxyphenyl)ethanone (3.82 g, 25.1 mmol),), tert-butyl 4-oxopiperidine-1-carboxylate (5.0 g, 25.1 mmol) and pyrrolidine (2.1 mL, 25.1 mmol) in methanol (100 mL). The mixture was heated at 60 °C for 2 days before concentrating and purifying by Biotage chromatography to provide tert-butyl 6-hydroxy-4-oxo-3,4-dihydro-1'H-spiro[chromene-2,4'-piperidine]-1'-carboxylate as a yellow solid (7.80 g, 93%).

A mixture of tert-butyl 6-hydroxy-4-oxo-3,4-dihydro-1'H-spiro[chromene-2,4'-piperidine]-1'-carboxylate (1.00 g, 3.00 mmol), acetone (5.0 mL), isopropyl iodide (3.06 g, 1.80 mL, 18 mmol), and K₂CO₃ (1.24 g, 9.0 mmol) was heated in a sealed tube at 70 °C overnight. The solids were removed by vacuum filtration and the filtrate was concentrated. The residue was purified by Biotage chromatography to provide tert-butyl 6-isopropoxy-4-oxo-3,4-dihydro-1'H-spiro[chromene-2,4'-piperidine]-1'-carboxylate (890 mg, 79%).

A mixture of tert-butyl 6-isopropoxy-4-oxo-3,4-dihydro-1'H-spiro[chromene-2,4'-piperidine]-1'-carboxylate (890 mg, 2.37 mmol), methanol (5.0 mL), and conc. HCl was stirred at room temperature overnight to provide 6-isopropoxyspiro[chromene-2,4'-piperidin]-4(3H)-one hydrochloride (750 mg, 100%).

### Spirocyclic Ketone Preparation 15

### 6-Ethoxyspiro[chromene-2,4'-piperidin]-4(3H)-one hydrochloride

A mixture of tert-butyl 6-hydroxy-4-oxo-3,4-dihydro-1'H-spiro[chromene-2,4'-piperidine]-1'-carboxylate (1.00 g, 3.00 mmol), prepared as described in Spirocyclic Ketone Preparation 14, acetone (5.0 mL), iodoethane (2.81 g, 1.45 mL, 18 mmol), and K₂CO₃ (1.24 g, 9.0 mmol) was heated in a sealed tube at 70 °C overnight. The solids were removed by vacuum filtration and the filtrate was concentrated. The residue was purified by Biotage chromatography to provide tert-butyl 6-ethoxy-4-oxo-3,4-dihydro-1'H-spiro[chromene-2,4'-piperidine]-1'-carboxylate (1.00 g, 92%).

A mixture of tert-butyl 6-ethoxy-4-oxo-3,4-dihydro-1'H-spiro[chromene-2,4'-piperidine]-1'-carboxylate (1.00 g, 2.77 mmol), methanol (5.0 mL), and conc. HCl was stirred at room temperature overnight to provide 6-ethoxyspiro[chromene-2,4'-piperidin]-4(3H)-one hydrochloride (830 mg, 100%).

### Spirocyclic Ketone Preparation 16

### 5-Chloro-7-methoxyspiro[chromene-2,4'-piperidin]-4(3H)-one Hydrochloride

To 3-chloro-5-methoxyphenol (4.00 g, 25.2 mmol) was added acetyl chloride (9.0 mL, 5.0 mmol) and the mixture was heated at 60 °C overnight. The acetyl chloride was removed under reduced pressure and AlCl₃ (1.96 g, 14.7 mmol) was added and the mixture was heated at 180 °C for 1 hour. The reaction mixture was cooled to room temperature. To this was slowly added 38% HCl/water (30 mL/100 mL) and stirred vigorously for 4 h. The mixture was filtered to obtain a mixture of 1-(2-chloro-6-hydroxy-4-methoxyphenyl)ethanone and 1-(4-chloro-2-hydroxy-6-methoxyphenyl)ethanone (5.38 g).

To a mixture of 1-(2-chloro-6-hydroxy-4-methoxyphenyl)ethanone and 1-(4-chloro-2-hydroxy-6-methoxyphenyl)ethanone (5.38 g, 26.8 mmol), tert-butyl 4-oxopiperidine-1-carboxylate (5.34 g, 26.8 mmol), methanol (100 mL), pyrrolidine (1.9 g, 2.2 mL, 27 mmol). The mixture was heated at 60 °C overnight. The solvents were removed unde reduced pressure and purified by CombiFlash® [Silica chromatography] to obtain tert-butyl 7-chloro-5-methoxy-4-oxo-3,4-dihydro-1'H-spiro[chromene-2,4'-piperidine]-1'-carboxylate (2.32 g) and tert-butyl 5-chloro-7-methoxy-4-oxo-3,4-dihydro-1'H-spiro[chromene-2,4'-piperidine]-1'-carboxylate (1.40 g).

A mixture of tert-butyl 5-chloro-7-methoxy-4-oxo-3,4-dihydro-1'H-spiro[chromene-2,4'-piperidine]-1'-carboxylate (500 mg, 1.31 mmol) in methanol (5.0 mL) and conc. HCl (6.6 mL) was stirred at room temperature overnight. The reaction mixture was concentrated to obtain the title product (425 mg, 100%).

### Spirocyclic Ketone Preparation 17

### 7-Chloro-5-methoxyspiro[chromene-2,4'-piperidin]-4(3H)-one

A solution of tert-butyl 7-chloro-5-methoxy-4-oxo-3,4-dihydro-1'H-spiro[chromene-2,4'-piperidine]-1'-carboxylate (prepared as described in Spirocyclic Ketone Preparation 16) (500 mg, 1.31 mmol) in methanol (5.0 mL) containing concentrated HCl (6.6 mL) was stirred at room temperature overnight. The reaction mixture was concentrated to provide the title compound (420 mg, 100%).

### Spirocyclic Ketone Preparation 18

### 7-Methoxy-5-methylspiro[chromene-2,4'-piperidin]-4(3H)-one hydrochloride

To a solution 1-(2-hydroxy-4-methoxy-6-methylphenyl)ethanone (0.81 g, 4.5 mmol) in methanol (15 mL) was added pyrrolidine (0.38 mL, 4.5 mmol) and tert-butyl 4-oxopiperidine-1-carboxylate (896 mg, 4.5 mmol). The mixture was stirred at 60 °C overnight. The solvents were removed and the residue was purified by CombiFlash® [Silica chromatography] to obtain tert-butyl 7-methoxy-5-methyl-4-oxo-3,4-dihydro-1'H-spiro[chromene-2,4'-piperidine]-1'-carboxylate (960 mg, 59%).

To a mixture of tert-butyl 7-methoxy-5-methyl-4-oxo-3,4-dihydro-1'H-spiro[chromene-2,4'-piperidine]-1'-carboxylate (960 mg, 2.66 mmol) in methanol (5.0 mL) was added 2 M HCl (13.3 mL). The mixture was stirred at room temperature overnight and then concentrated to obtain the title product (780 mg, 99%).

### Spirocyclic Ketone Preparation 19

### Spiro[chromene-2,4'-piperidin]-4(3H)-one

A solution of 1-(2-hydroxyphenyl)ethanone (100 g, 0.74 mol), tert-butyl 4-oxopiperidine-1-carboxylate (146 g, 0.74 mol) and.pyrrolidine (61 mL, 0.74 mol) in methanol (600 mL) was stirred for 24 hours and evaporated. The residue was subjected to chromatography (hexane/ethyl acetate 100:0→ 90:10) on silica gel to afford N-Boc-Spiro[chromene-2,4'-piperidin]-4(3H)-one in 97% (225 g) yield, 218 (M-Boc; ES+).

Neat trifluoroacetic acid (80 mL) was added to a solution of [N-Boc-Spiro[chromene-2,4'-piperidin]-4(3H)-one or tert-Butyl 4-Oxo-3,4-dihydro-1'H-spiro[chromene-2,4'-piperidine]-1'-carboxylate] (40 g, 0.126 mol) in dichloromethane (250 mL). The solution was then stirred overnight and then evaporated. Water (about 300 mL) was added to the residue, and the obtained solution was made alkaline with 10 N NaOH to pH about 14. The product was then extracted with chloroform. The extract was dried over Na₂SO₄ and evaporated to give spiro[chromene-2,4'-piperidin]-4(3H)-one in 93.7% (25.6 g) yield, 218 (ES+)

### Spirocyclic Ketone Preparation 20

### 7-Fluorospiro[chromene-2,4'-piperidin]-4(3H)-one Hydrochloride Hydrate

A solution of 1-(4-fluoro-2-hydroxyphenyl)ethanone (200 g, 1.3 mol), tert-butyl 4-oxopiperidine-1-carboxylate (258 g, 1.3 mol) and pyrrolidine (108 mL, 1.3 mol) in methanol (800 mL) was stirred for 24 hours and then evaporated. Next the residue was dissolved in ethyl acetate, washed with water, 0.5 N HCl, NaHCO₃ solution and saturated aqueous NaCl and passed through a thin layer of SiO₂ and Na₂SO₄. The filtrate was evaporated, and the residue was washed with hexane/ethyl acetate (9:1) mixture and subjected to chromatography (hexane/ethylacetate 90:10→ 80:20) to afford N-Boc-7-Fluorospiro[chromene-2,4'-piperidin]-4(3H)-one in 33% (144 g) yield, 236 (M-Boc; ES+).

HCl (90 mL) was added to a solution of N-Boc-7-Fluorospiro[chromene-2,4'-piperidin]-4(3H)-one] (44.2 g, 0.132 mol) in isopropanol (150 mL), and the obtained mixture was refluxed for 3 hours. After this, the mixture was evaporated, and the residue was co-evaporated twice with isopropanol, washed with ether and dried to give 7-fluorospiro[chromene-2,4'-piperidin]-4(3H)-one hydrochloride hydrate in 99% (35.8 g) yield, 236 (API+).

### Spirocyclic Ketone Preparation 21

### 6-Methylspiro[chromene-2,4'-piperidin]-4(3H)-one

A solution of 1-(2-hydroxy-5-methylphenyl)ethanone (100 g, 0.67 mol), tert-butyl 4-oxopiperidine-1-carboxylate (133 g, 0.67 mol) and pyrrolidine (55.8 mL, 0:67 mol) in methanol (600 mL) was stirred for 24 hours and filtered. The separated crystals were washed with hexane/ethyl acetate (9:1) mixture, then refluxed with hexane (150 mL) and separated by filtration again. The crystals were finally dried to give tert-butyl 6-methyl-4-oxo-3,4-dihydro-1'H-spiro[chromene-2,4'-piperidine]-1'-carboxylate in 89% (195.5 g) yield. ¹H NMR (DMSO-d₆) δ 7.48 (s, 1H), 7.36 (dd, J=3, 8, 1H), 6.94.(d, J=8, 1H), 3.67 (m, 2H), 3.08 (m, 2H), 2.76 (s, 2H), 2.23 (s, 3H), 1.82 (m, 2H), 1.56 (m, 2H), 1.36 (s, 9H).

Neat trifluoroacetic acid (80 mL) was added to a solution of tert-butyl 6-methyl-4-oxo-3,4-dihydro-1'H-spiro[chromene-2,4'-piperidine]-1'-carboxylate (40 g, 0.12 mol) in dichloromethane (300 mL). The obtained solution was stirred overnight and then evaporated. Water (200 mL) and chloroform (200 mL) were then added to the residue, and the obtained solution was made alkaline with 19 N NaOH to about pH 12. The product was then extracted with chloroform. The extract was passed through a layer of SiO₂ and Na₂SO₄ and evaporated to give 6-methylspiro[chromene-2,4-piperidin]-4(3H)-one in 96% (26.7 g) yield, 232 (ES+).

### Spirocyclic Ketone Preparation 22

### 6,7-Dimethylspiro[chromene-2,4'-piperidin]-4(3H)-one

1-(2-Hydroxy-4,5-dimethylphenyl)ethanone (150 g, 0.914 mol) and pyrrolidone (76.3 mL, 0.914 mol) in methanol (1 L) were stirred for 15 minutes. Then tert-butyl 4-oxopiperidine-1-carboxylate (182.2 g, 0.914 mol) was added and the mixture was stirred for 24 hours. The formed precipitate was separated by filtration, washed with hexane and dried to give 245 g of pure tert-butyl 6,7-dimethyl-4-oxo-3,4-dihydro-1'H-spiro[chromene-2,4'-piperidine]-1'-carboxylate. The combined filtrates were evaporated, and the residue was crystallized from some hexane to give an additional portion (21 g) of product. The total yield was 77.1 % (266 g). ¹H NMR (CDCl₃) δ 7.58 (s, 1H), 6.76 (s, 1H), 3.84 (br d, J=13, 2H), 3.18 (m, 2 H), 2.64 (s, 2H), 2.25 (s, 3H), 2.19 (s, 3H), 1.99 (br d, J=12, 2H), 1.59 (m, 2H), 1.44 (s, 9H), 344 (API).

Neat trifluoroacetic acid (80 mL) was added under cooling with cold water to tert-butyl 6,7-dimethyl-4-oxo-3,4-dihydro-1'H-spiro[chromene-2,4'-piperidine]-1'-carboxylate (50 g, 0.145 mol) in dichloromethane (300 mL). The mixture was stirred at room temperature overnight and then the volatiles were evaporated: The residue was dissolved in water and the aqueous layer washed twice with ether, then made alkaline with NaOH to about pH 14. The product was extracted with CHCl₃, dried (Na₂SO₄), and concentrated to afford the product (27.9 g; 78.6%). ¹H NMR (DMSO-d₆) δ 7.49 (s, 1H), 6.94 (s, 1H), 3.34 (br s, 6 H), 3.95 (br d, J=12, 1H), 3.82 (t, J=12, 1H), 2.82 (s, 1H), 2.24 (s, 2H), 2.18 (s, 2H), 2.59 (d, J=15, 1H), 2.34 (t, J=11, 1H). LC/MS API+ 246 (MH+).

### Spirocyclic Ketone Preparation 23

### 6-Chlorospiro[chromene-2,4'-piperidin]-4(3H)-one

A solution of 1-(5-chloro-2-hydroxyphenyl)ethanone (ASDI Inc., Newark, DE) (103.9 g, 0.609 mol), tert-butyl 4-oxopiperidine-1-carboxylate (2; 121.2 g, 0.609 mol) and pyrrolidine (50.8 mL, 0.609 mol) in methanol (500 mL) was stirred for 24 h, and then the precipitated crystals were separated by filtration and washed with hexane/ethyl acetate (9:1) mixture. Then hexane (150 mL) was added; and the obtained mixture was refluxed and then filtered. The separated crystals were dried to give N-Boc-6-chlorospiro[chromene-2,4'-piperidin]-4(3H)-one in 72% (153 g) yield.

Neat trifluoroacetic acid (80 mL) was added to a solution of N-Boc-6-chlorospiro[chromene-2,4'-piperidin]-4(3H)-one (50 g, 0.14 mol) in dichloromethane (300 mL). The mixture was stirred for 24 hours and then evaporated. Water (200 mL) and chloroform (200 mL) were added to the residue, and the obtained mixture was made alkaline with 19 N NaOH to about pH 12. The product was extracted with chloroform. The extract was passed through a thin layer of SiO₂ and Na₂SO₄ to give 6-chlorospiro[chromene-2,4'-piperidin]-4(3H)-one in 88% (30.8 g) yield, 252/254 (ES+).

### Spirocyclic Ketone Preparation 24

### 5-Methoxyspiro[chromene-2,4'-piperidin]-4(3H)-one

1-(2-Hydroxy-6-methoxyphenyl)ethanone (40.5 g, 0.244 mol) and pyrrolidine (20.3 mL; 0.244 mol) in methanol (250 mL) were stirred for 15 minutes, and then tert-butyl 4-oxopiperidine-1-carboxylate (48.6 g, 0.244 mol) was added. The mixture was stirred for a further 24 hours and filtered. The separated precipitate was washed with hexane and dried to give N-Boc-5-methoxyspiro[chromene-2,4'-piperidin]-4(3H) one in 83.5% (70.7 g) yield, 248 (M-Boc; ES+)

Neat trifluoroacetic acid (80 mL) was added to N-Boc-5-methoxyspiro[chromene-2,4'-piperidin]-4(3H)-one (50 g, 0.144 mol) in dichloromethane (200 mL), and the mixture was stirred at room temperature overnight and then evaporated. The residue was diluted with water (500 mL) and made alkaline with 10 N NaOH to pH 14. The product was extracted with chloroform, and the extract was dried over Na₂SO₄ and evaporated. The residue was subjected to chromatography (chloroform/methanol/triethylamine 100:0:0→ 91:9→ 0:86:14) on silica gel to give 5-methoxyspiro[chromene-2,4'-piperidin]-4(3H)-one in 84.7% (30 g) yield, 248 (ES+).

### Spirocyclic Ketone Preparation 25

To a mixture of an ortho-hydroxyacetophenone or 2-hydroxybenzamide and pyrrolidine (0.75 to1 equivalents) in a suitable solvent, such-as-methanol, benzene or toluene, was added tert-butyl 4-oxopiperidine-1-carboxylate (1 equivalent) and the mixture stirred 18-48 hours at a temperature between room temperature and reflux. Product was isolated by filtration, optionally purified via silica chromatography following an aqueous workup.

At room temperature, a solution of N-Boc-spiro[chromene-2,4'-piperidin]-4(3H)-one in a suitable solvent, such as dichloromethane, dioxane or methanol, was treated with a suitable acid, such as trifluoroacetic acid or 4 N HCl in dioxane, until the reaction was complete. The volatiles were evaporated to provide the salt of the desired product. The product was carried on as the salt form or, when the free base was prepared, it was done by dissolving the residue in water washing the aqueous layer with ether, rendering the aqueous phase basic with NaOH to pH about 14. The product was extracted with CHCl₃, dried (Na₂SO₄), and concentrated afford the free base of the desired product.

Using this method, spirocyclic ketones were prepared from the following commercially available ortho-hydroxyacetophenones: 2'-hydroxy-4'-methylacetophenone (Sigma-Aldrich, St. Louis, MO), 1-(4-chloro-2-hydroxy-phenyl)ethanone (Wako Pure Chemical Industries, Ltd., Osaka, Japan), 1-(3-hydroxy-biphenyl-4-yl)ethanone (Bradsher, C. K.; et al, J. Am. Chem. Soc. 1954, 76, 2357-2362), 1-(2-hydroxy-3,5-dimethylphenyl)ethanone (Oakwood Products, Inc., West Columbia, SC), 1-(2-hydroxy-5-trifluoromethoxyphenyl)ethanone (Apollo Scientific Ltd., Cheshire, UK), 3-acetyl-4-hydroxy-benzonitrile (Ramidus AB, Lund, Sweden), 4',5'-dimethoxy-2'-hydroxyacetophenone (Indofine Chemical Company, Inc., Hillsborough, NJ), 1-(3,5-dichloro-2-hydroxyphenyl)ethanone (ASDI Inc., Newark, DE), 3-acetyl-4-hydroxybenzoic acid (Princeton BioMolecular Research Inc., Monmouth Junction, NJ), 1-(2-hydroxy-5-(methylsulfonyl)phenyl)ethanone (CiventiChem, Research Triangle Park,NC), and 1-(2-hydroxy-5-isopropylphenyl)ethanone (AstaTech, Inc., Bristol, PA).

In addition, the following ortho-hydroxyacetophenones, which were prepared as described below, were used to prepare spirocyclic ketones, using the method of Spirocyclic Ketone Preparation 25: 4'-chloro-2'-hydroxy-5'-methylacetophenone, 4'-chloro-2'-hydroxyacetophenone, 4',5'-dichloro-2'-hydroxyacetophenone, 4-acetyl-3-hydroxy-benzonitrile, 1-(2-hydroxy-5-trifluoromethyl-phenyl)ethanone; 1-(5-chloro-4-fluoro-2-hydroxyphenyl)ethanone, 1-(4-chloro-5-fluoro-2-hydroxyphenyl)ethanone, 1-(5-bromo-2-hydroxy-4-methylphenyl)ethanone, 1-(2,4-dichloro-6-hydroxyphenyl)ethanone, 1-(3-chloro-6-hydroxy-2,4-dimethylphenyl)ethanone, and 1-(2-fluoro-6-hydroxy-3-methoxyphenyl)ethanone.

### Ortho-Hydroxyacetophenones

Ortho-hydroxyacetophenones, which were used to prepare exemplified compounds of the present invention, were prepared using the method of one of the following Hydroxyacetophenone Preparations 1-6.

### Ortho-Hydroxyacetophenone Preparation 1

### 1-(4-chloro-2-hydroxy-5-methylphenyl)ethanone

To 3-chloro-4-methylphenol (1.97 g, 13.8 mmol)was added acetyl chloride (1.15 g, 1.04 mL, 14.6 mmol) and the resulting mixture was heated at 60° C for 2 hours. To this was added AlCl₃ (1.84 g, 13.8 mmol) and the mixture was heated at 180° C for 30 minutes. The reaction mixture was then cooled to room temperature and slowly quenched with 38% HCl/water (8 mL/17 mL) and stirred for 30 minutes. The solids were removed by filtration, with water, concentrated and dried to afford the title compound as a yellow solid (1.97 g, 77%). ¹H NMR (CDCl₃) δ 11.21 (s, 1H), 7.54 (s, 1 H), 7.00 (s, 1 H), 2.59 (s, 3 H), 2.32 (s, 3 H).

### Ortho-Hydroxyacetophenone Preparation 2

### 1-(2-Hydroxy-5-trifluoromethyl-phenyl)ethanone

A mixture of 4-trifluoromethyl-2-bromophenol (1.00 g, 4.15 mmol), toluene (20 mL), (1-ethoxyvinyl)tributyl stannane (1.65 g, 4.56 mmol) and dichlorobis (triphenylphoshine)palladium (146 mg, 0.207 mmol) was heated at 100 °C overnight before cooling to room temperature. To this mixture was added 1 N HCl (6 mL) and the mixture was then vigorously stirre for about 90 minutes. The organic phase was washed with water (30 mL), saturated aqueous NaCl, dried over Na₂SO₄, filtered and concentrated to obtain a black oil. The product was purified by Biotage chromatography (EtOAc/heptane gradient) to obtain the title compound as a pale yellow oil (335 mg, 39.5%).

### Ortho-Hydroxyacetophenone Preparation 3

### 1-(2-hydroxy-6-methoxy-4-methylphenyl)ethanone

To a solution of 5-methyl-cyclohexane-1,3-dione (1.01 g, 8.0 mmol) in CH₂Cl₂ (15 mL) was added triethylamine (1.2 mL, 8.6 mmol) followed by acetyl chloride (0.6 mL, 8.4 mmol). The mixture was stirred at room temperature for 3 hours before washing with water (2x). The aqueous phase was back extracted with EtOAc. The combined organic extracts were concentrated and purified by Biotage (40S column, 15% acetone/heptane) to provide 5-methyl-3-oxocyclohex-1-enyl acetate (1.2 g, 89%).

A mixture of 5-methyl-3-oxocyclohex-1-enyl acetate (1.2 g, 7.1 mmol), CH₃CN (15 mL), triethylamine (2.1 mL) and sodium cyanide (7 mg, 0.1 mmol) was stirred at room temperature overnight. The mixture was concentrated, re-dissolved in EtOAc and acidified with 1 N HCl. The organic phase was isolated , concentrated and purified by Biotage chromatography (40S column, 8% acetone/heptane) to afford 2-acetyl-5-methylcyclohexane-1,3-dione (0.96 g, 96%).

A solution of 2-acetyl-5-methylcyclohexane-1,3-dione (0.96 g, 5.7 mmol) in methanol (28 mL) containing iodine (2.90 g, 11.4 mmol) was heated at reflux overnight. The mixture was cooled to room temperature and concentrated. The material was dissolved in CH₂Cl₂ and washed with aqueous Na₂S₂O₃ and the aqueous phase was back extracted with CH₂Cl₂ (2x). The combined organic extracts were concentrated and purified by Biotage chromatography (40 M column, 10% acetone/heptane) to provide the title compound as a pale yellow solid (550 mg, 53%).

### Ortho-Hydroxyacetophenone Preparation 4

### 1-(2-fluoro-6-hydroxy-3-methoxyphenyl)ethanone

To a -78 °C solution of 1,4-dimethoxy-2-fluorobenzene (1.56 g, 10 mmol) in THF (15 mL) was added n-BuLi (5.0 mL of 2.5 M hexanes solution, 12 mmol). The mixture was stirred for 30 minutes before slow addition of acetaldehyde (0.79 mL, 14 mmol). The reaction mixture was stirred for an additional 30 minutes before the reaction was quenched by addition of methanol and saturated aqueous NH₄Cl. The reaction mixture was warmed to room temperature and extracted with EtOAc. The organic extract was concentrated and purified by Biotage chromatography (40 S column, 15% acetone/heptane) to afford 1-(2-fluoro-3,6-dimethoxyphenyl)ethanol (1.57 g, 79%).

To an ice cooled solution of 1-(2-fluoro-3,6-dimethoxyphenyl)ethanol (1.57 g, 7.84 mmol) in acetone (23 mL) was slowly added Jones reagent (prepared by addition of 1.57 g CrO₃ in 1.6 mL of concentrated H₂SO₄ to 4.7 mL of ice cold water). The mixture was stirred for 30 minutes before the cooling bath was removed and addition of isopropanol (2 mL). The resultant green precipitate was removed by filtration through diatomaceous earth, and the diatomaceous earth was washed with ethyl acetate ("EtOAc"). The filtrate was concentrated and redissolved in EtOAc, washed with saturated aqueous NaHCO₃, saturated aqueous NaCl, concentrated, and purified by Biotage chromatography (40 S column, 8% acetone/heptane) to provide the title compound as a yellow oil (1.06 g, 68%).

### Ortho-Hydroxyacetophenone Preparation 5

To a phenol was added an acetylation reagent, such as acetyl chloride (1.0-2.0) or acetic anhydride, and the reaction mixture stirred for 2-18 hours at a temperature between room temperature and reflux. Excess acetylation reagent was removed in vacuo, then the O-acetylated phenol was treated with AlCl₃ (1.0-1.25 eq) and heated to a high temperature, such as 180° C for 30-60 minutes. The reaction mixture was then cooled, quenched with aqueous acid, and filtered to afford the desired orthohydroxyacetophenone.

Hydroxyacetophenones were prepared, using the method of Hydroxyacetophenone Preparation 5, from commercially available reagents as follows: 4-Acetyl-3-hydroxy-benzonitrile from 3-hydroxybenzonitrile, 1-(5-chloro-4-fluoro-2-hydroxyphenyl)ethanone from 4-chloro-3-fluorophenol, 1-(4-chloro-5-fluoro-2-hydroxyphenyl)ethanone from 3-chloro-4-fluorophenol, 1-(5-bromo-2-hydroxy-4-methylphenyl)ethanone from 4-bromo-3-methylphenol, 1-(2,4-dichloro-6-hydroxyphenyl)ethanone from 3,5-dichlorophenol, 1-(3-chloro-6-hydroxy-2,4-dimethylphenyl)ethanone from 4-chloro-3,5-dimethylphenol.

### Ortho-Hydroxyacetophenone Preparation 6

### 1-(4,5-Dichloro-2-hydroxyphenyl)ethanone

To 3,4-dichlorophenol (2.00 g; 12.3 mmol) was added acetyl chloride (1.02 g, 13 mmol) at room temperature, and after 30 minutes the reaction mixture was heated to 60° C for 45 minutes. AlCl₃ (1.64 g, 12.3 mmol) was then added and the reaction mixture heated at 180° C for 30 minutes. The reaction mixture was allowed to cool and slowly quenched with 38% HCl/H2O (30 mL/100 mL), then stirred for 2.5 hours. The resultant precipitate was filtered, washed with water, and dried under high vacuum to obtain the product as an off-white solid (2.07 g; 82% yield). ¹H NMR (CDCl₃) δ 12.16 (s, 1 H), 7.78 (s, 1 H) 7.11 (s, 1H), 2.61 (s, 3H): LC-MS m/z @ 203, 205, 207 (ES-).

### Carboxylic Acids

The following commercially available carboxylic acids were used to prepare exemplified compounds of Formula (1) of the present invention: 2-ethyl-1-(3-methoxy-phenyl)-1H-benzoimidazole-5-carboxylic acid (DiscoveryLab Ltd., Russia), 1H-Indazole-5-carboxylic acid (Tyger Scientific, Inc., Ewing, NJ), 1 H-Indazole-6-carboxylic acid (Sinova Inc., Bethesda, MD), 1-Methyl-1H-indole-5-carboxylic acid (ASDI Inc., Newark, DE), 1H-Benzoimidazole-5-carboxylic acid (Infarmatik, Inc., Newark, DE), 2-Pyridin-2-yl-1H-benzoimidazole-5-carboxylic acid (Aurora Fine Chemicals Ltd., Graz, Austria), 2-Trifluoromethyl-1 H-benzoimidazole-5-carboxylic acid (Oakwood Products, Inc., West Columbia, SC), 2-methyl-1H-benzoimidazole-5-carboxylic acid (Acros Organics USA, Morris Plains, NJ), 1H-indazole-4-carboxylic acid; 1H-indole-7-carboxylic acid (J & W PharmLab LLC, Morrisville, PA), 1H-indole-6-carboxylic acid (Sigma-Aldrich, St. Louis, MO), 1-methyl-1H-indole-4-carboxylic acid (Maybridge, Cornwall, UK), 2-Pyridin-4-yl-3H-benzoimidazole-5-carboxylic acid (Infarmatik, Inc., Newark, DE), 2-hydroxymethyl-1H-benzoimidazole-4-carboxylic acid (Matrix Scientific, Columbia, SC), 1 H-benzoimidazole-4-carboxylic acid (Infarmatik, Inc., Newark, DE), 2-methyl-1H-benzoimidazole-4-carboxylic acid (Infarmatik, Inc., Newark, DE), 1-methyl-2-oxo-2,3-dihydro-1H-benzoimidazole-5-carboxylic acid (Chemstep, Carbon Blanc, France), quinoline-6-carboxylic acid (Alfa Aesar, Ward Hill, MA), 1H-benzotriazole-5-carboxylic acid (Sigma-Aldrich, St. Louis, MO), 1-methyl-1H-benzotriazole-5-carboxylic acid (Ryan Scientific, Mt. Pleasant, SC), 3-(1H-pyrazol-3-yl)-benzoic acid (Maybridge. Cornwall, UK), 3-pyrazol-1-yl-benzoic acid (ASDI Inc., Newark, DE),-1H-indole-4-carboxylic acid (Matrix Scientific, Columbia, SC), 1-methyl-1H-benzoimidazole-5-carboxylic acid (Ambinter Sari, Paris, France), 3-(5-methyl-[1,2,4]oxadiazol-3-yl)-benzoic acid (ASDI Inc. Newark, DE), 1-oxo-2,3,4,4a,9,9a-hexahydro-1H-beta-carboline-6-carboxylic acid (J & W PharmLab LLC, Morrisville, PA), 2-phenyl-1H-benzimidazole-6-carboxylic acid (Fluorochem Ltd., Derbyshire, UK), 1-(2,3,4,9-tetrahydro-1H-carbazol-6-yl)ethanone (Matrix Scientific, Columbia, SC), 1 H-benzimidazole-6-carboxylic acid (ASDI Inc., Newark, DE), 2,3-dimethyl-1H-indole-5-carboxylic acid (Matrix Scientific, Columbia, SC), benzotriazole-5-carboxylic acid (Sigma-Aldrich, St. Louis, MO), 2,3-dimethyl-1H-indole-5-carboxylic acid (Matrix Scientific, Columbia, SC), 5-carboxyindole (Apollo Scientific Ltd., Cheshire, UK), 1,2-dimethyl-1H-benzoimidazole-5-carboxylic acid (Matrix Scientific, Columbia, SC), 5-Benzimidazolecarboxylic acid Sigma-Aldrich, St. Louis, MO), Benzotriazole-5-carboxylic acid (Sigma-Aldrich, St. Louis, MO), 1-iso-Propylbenzotriazole-5-carboxylic acid (Fluorochem Ltd., Derbyshire, UK), 2-oxo-1,2,3,4-tetrahydroquinoline-6-carboxylic acid (Aurora Fine Chemicals Ltd., Graz, Austria) and quinoxaline-6-carboxylic acid (Ryan Scientific, Inc., Mount Pleasant, SC).

The following carboxylic acids, which were used to prepare compounds of the present invention as described in the Examples, were prepared by previously published means: 2-methyl-1H-indole-6-carboxylic acid (Journal of Organic Chemistry (1980), 45(8), 1546-7, example 7, page 1547), 1-isopropyl-1H-benzoimidazole-4-carboxylic acid (US 2005/020626, page 17, Preparation 11), 3-(1H-imidazol-2-yl)-benzoic acid (US 2003/0232860, page 14, example 14) and 1,3-benzoxazole-5-carboxylic acid (Sawada, Y.; et al., Pest Management Science (2003), 59(1), 25-35).

In addition, carboxylic acids, which were used to prepare compounds of the present invention as described in the Examples, were prepared as described in the following Acid Preparations:

### Acid Preparation 1

### 7-Methyl-1H-indazole-5-carboxylic acid

To a solution of 5-bromo-7-methylindazole, (purchased from PharmaLab, Morrisville, PA) (2.00 g, 9.47 mmol) in anhydrous THF (50 ml) was added NaH (570 mg, 14.25 mmol; 60% suspension in mineral oil) at room temperature. After 20 minutes the mixture was cooled to -78 °C and sec-butyllithium (1.4 M in cyclohexane, 17 ml; 23.8 mmol) was added drop wise and the resulting mixture was stirred for 4 hours. Dry CO₂ was then bubbled through the reaction mixture for 1 hour while allowing warming to room temperature. It was then stirred at room temperature overnight. 1 N HCl was added and the solution extracted with EtOAc. The organic layer was washed with saturated aqueous NaCl, dried (MgSO₄), then filtered and concentrated. The residue was re-dissolved in MeOH, filtered, then concentrated to provide the product as a brown solid (1.445 g, 86.6%). ¹H NMR (DMSO-d₆) δ 8.23 (s, 1H), 8.17 (s, 1H), 7.65 (s, 1H), 2.46 (s, 3H). LC/MS ES+ 177 (MH+).

### Acid Preparation 2

### 1H-Indazole-7-carboxylic acid

A mixture of 2-amino-3-methylbenzoic acid (15.2 g, 0.10 mol), dimethylformamide (333 mL) and CsCO₃ (49 g, 0.15 mol) was stirred at room temperature for about 40 minutes before drop wise addition of iodomethane (14.2 g, 6.2 mL, 0.10 mol) in dimethylformamide ("DMF") (115 mL). The mixture was stirred at room temperature overnight. The mixture was diluted with water (1 L), and extracted with diethyl ether. The aqueous phase was back extracted with diethyl ether. The combined organic extracts were washed with saturated aqueous NaCl, dried over MgSO₄, filtered and concentrated. The resultant material was dried at room temperature/0.5 mmHg to afford methyl 2-amino-3-methylbenzoate (17 g, 100%).

To a solution methyl 2-amino-3-methylbenzoate (16.5 g, 0.10 mol) in CHCl₃ (286 mL) was added acetic anhydride (23.5 g, 21.7 mL, 0.23 mol) so as to maintain the internal temperature <40 °C. The mixture was stirred at room temperature for 1 hour before addition of potassium acetate (2.94 g, 30 mmol) and isoamyl nitrite (25.8 g, 30 mL, 0.22 mol). The resultant mixture was heated at reflux overnight. To this was then added methanol (94 mL) and 6 N HCl (94 mL) and the mixture was stirred overnight. The reaction mixture was concentrated to provide an orange solid which was subsequently triturated with ethyl acetate and the solids were isolated by vacuum filtration. The solids were dried at room temperature/0.5 mmHg to afford methyl 1H-indazole-7-carboxylate (15.4 g, 88%).

A solution of methyl 1H-indazole-7-carboxylate (14.96 g, 84.9 mmol) in methanol (180 mL) was cooled to 0 °C before addition of 29% aqueous potassium hydroxide (36 mL). The ice bath was removed and the reaction mixture was stirred at room temperature overnight. The pH was adjusted to 5.5 using concentrated HCl. The volatiles were removed by vacuum filtration and the resultant material was suspended in water (100 mL) and ethyl acetate (200 mL). The resultant precipitate was isolated by vacuum filtration and rinsed with ethyl acetate. The solids were dried at room temperature/0.5 mmHg to afford the title compound (7.54 g, 55%).

### Acid Preparation 3

### 2-Methyl-2H-indazole-6-carboxylic acid

Methyl 1H-indazole-6-carboxylate was prepared according to the procedure disclosed in J. Med. Chem. 2000, 43 (1), 41-58 (example 12b, page 49). Alkylation under standard conditions (sodium hexamethyldisilazide, THF, iodomethane, reflux) provided methyl 2-methyl-2H-indazole-6-carboxylate (44%). Saponification under standard conditions (1 N NaOH) afforded the title product (53%).

### Acid Preparation 4

### 3-(5-Trifluoromethyl-1H-pyrazol-3-yl)-benzoic acid

Sodium hydride (60% in oil, 2.20 g, 55 mmol) was placed in an oven dried reaction flask under nitrogen and washed twice with 10 mL portions of hexane, removing the hexane by decantation. The sodium hydride was then suspended in 30 mL of dry 1,2-dimethoxyethane ("DME") with stirring. A solution of 9.0 mL (75 mmol) of purified ethyl trifluoroacetate and 3.63 g (25 mmol) of 3-cyanoacetophenone (Aldrich) in 40 mL of DME was added drop wise over 40 minutes. The reaction mixture was stirred for an additional 60 minutes, after which excess hydride was destroyed by addition of methanol (about 3 mL). The volatile components were removed by evaporation under vacuum and the residue was suspended in 30 mL of water. The mixture was acidified with 70 mL of 1 M hydrochloric acid and extracted with ether. The ether was washed with water, saturated aqueous NaCl, dried (MgSO₄) and evaporated to 7.02 g a solid residue of 3-(4,4,4-trifluoro-3-oxo-butyryl)-benzoic acid containing some residual trifluoroacetic acid.

The product of Step 1 (3.71 g, 15 mmol) was dissolved in 30 mL of ethanol and heated to reflux, at which point 0.97 mL of anhydrous hydrazine (31 mmol) was added in one portion. Heating was continued for 90 minutes, after which the mixture was concentrated under vacuum. The residue was treated with water and ether; the ether was washed with 1 M hydrochloric acid, water, saturated aqueous NaCl, dried (MgSO₄) and evaporated. Acetonitrile was added and the evaporation was repeated to afford a white solid, which was dissolved in 15 mL of glacial acetic acid, heated at reflux for 20 minutes and concentrated under vacuum to afford 3.12 g of 3-(5-trifluoromethyl-2H-pyrazol-3-yl)-benzonitrile as a white solid.

The product of Step 2 (3.12 g, 13 mmol) was dissolved in 20 mL of 1-propanol. A solution of 4.33 g of potassium hydroxide in 8 mL of water was added and the mixture was heated at reflux for 2 hours. The mixture was cooled and evaporated under vacuum. The residue was dissolved in 75 mL of water, heated to boiling, and acidified with concentrated hydrochloric acid. The mixture was allowed to cool and the precipitate was filtered, washed with water and dried to afford 3.21 g of the title compound as a white solid.

### Acid Preparation 5

### 7-Chloro-1H-indazole-5-carboxylic acid,

To a solution of 4-amino-3-chloro-5-methylbenzonitrile (3.00 g, 18.0 mmol) in CHCl₃ (50 mL) was added acetic anhydride (3.9 mL, 41.4 mmol). The mixture was stirred at room temperature overnight and then heated at reflux for 5 hr. The reaction mixture was cooled to room temperature and potassium acetate (530 mg, 5.40 mmol) and isoamyl nitrite (5.28 mL, 39.6 mmol) were added. The mixture was heated at reflux for 3 days. The reaction mixture was washed with saturated aqueous NaHCO₃, dried over Na₂SO₄ and concentrated. To this was added methanol followed by water (25 mL) and 38% HCl (25 mL). The mixture was stirred at room temperature overnight. The reaction mixture was concentrated and the pH was adjusted to about 7. The solids were isolated by filtration and then washed with water (2 x 30 mL) and heptane (2 x 30 mL). Purify by Biotage chromatography (CH₂Cl₂-heptane (1:1)/MeOH gradient to afford 7-chloro-1H-indazole-5-carbonitrile was isolated as a white solid (585 mg, 18%).

To a solution of 7-chloro-1H-indazole-5-carbonitrile (250 mg, 1:41 mmol) in ethanol/water (3:1 ration, 15 mL) was added potassium hydroxide (395 mg, 7.04 mmol) and the mixture was heated at reflux. After 3 houra, the majority of the ethanol was allowed to distill off, additional potassium hydroxide (614 mg) was added and heating was continued for overnight. The reaction mixture was cooled to room temperature, washed with Et₂O (3 x 20 mL) and the organic extract was acidified with 1 N HCl. The resultant precipitate was isolated by vacuum filtration, washed with water (about 15 mL) and heptane (about 15 mL), dried at room temperature/0.5 mmHg to provide the title compound (221 mg, 79.7%).

### Acid Preparation 6

### 5-Bromo-1H-indazole-7-carboxylic acid

To a solution of 2-amino-3-methylbenzoic acid (5.00 g, 33.1 mmol) in acetic acid (110 mL) at 0 °C was added drop wise a mixture of bromine (1.7 mL, 33 mmol) in acetic acid (50 mL) over about 5 minutes. Following addition, the cooling bath was removed and the mixture was stirred at room temperature for 30 minutes before removal of acetic acid under reduced pressure. The mixture was diluted with CH₂Cl₂ and washed with saturated aqueous Na₂CO₃. The aqueous phase was back extracted with CH₂Cl₂. The aqueous phase was acidified using concentrated HCl to pH 7.2, with intense foaming observed. Copious amounts of precipitate formed and were isolated by vacuum filtration . The filtrate was further acidified with concentrated HCl to pH 6.3 and a second crop of precipitate was collected. The combined solids were dried at 65 °C /0.5 mmHg to provide 2-amino-5-bromo-3-methylbenzoic acid (6.43 g, 85%).

A solution of 2-amino-5-bromo-3-methylbenzoic acid (6.43 g, 27.9 mmol) in DMF (93 mL) containing cesium carbonate (13.7 g, 41.9 mmol) was stirred at room temperature for 40 minutes before drop wise addition of a solution of iodomethane (1.7 mL, 28 mmol) in DMF (21 mL). The mixture was stirred at room temperature for 2 days. The mixture was diluted with water (300 mL) and extracted with EtOAc (2 x 100 mL). The combined organic extracts were dried over MgSO₄, filtered and concentrated to afford a brown oil that solidified into a beige solid after drying at room temperature/0.5 mmHg to provide methyl 2-amino-5-bromo-3-methylbenzoate (5.45 g, 80%).

To a solution of methyl 2-amino-5-bromo-3-methylbenzoate (5.45 g, 22.3 mmol) in CHCl₃(64 mL) was added acetic anhydride (4.9 mL) at such rate as to maintain the internal temperature below 40 °C. The resulting mixture was stirred at room temperature for 1 hour and then potassium acetate (0.66 g, 6.7 mmol) and isoamyl nitrite (6.6 mL, 49 mmol) were added. The reaction mixture was heated at reflux overnight and then cooled to room temperature and concentrated. The residue was dissolved in methanol (22 mL) and 6 N HCl (22 mL) and stirred at room temperature for about 4 hours. A yellow solid was isolated by vacuum filtration and rinsed with water. The solids were dried at 65 C/0.5 mmHg to provide methyl 5-bromo-1H-indazole-7-carboxylate (4.90 g, 86%).

To a solution of 5-bromo-1H-indazole-7-carboxylate (250 mg, 0.98 mmol) in methanol (2 mL) at 0 °C was added 30% aqueous KOH (0.15 g KOH in 0.5 mL water). The mixture was stirred at room temperature for 2 days. The resultant solids were isolated by vacuum filtration and rinsed with MeOH. The solid material was dried at 65 °C /0.5 mmHg to provide the title compound as a light yellow solid (182 mg, 67%).

### Acid Preparation 7

### 3-Methyl-1H-indazole-6-carboxylic acid

A solution of 2-fluoro-4-methoxyacetophenone (2.0 g, 12 mmol) in hydrazine (30 mL) was heated at reflux for 2 days. The mixture was cooled to room temperature, poured into water and extracted with EtOAc (3x). The combined organic extracts were concentrated, dissolved in a minimum amount of CH₂Cl₂, filtered to provide 6-methoxy-3-methyl-1H-indazole as a yellow solid (620 mg, 32%).

To a solution of 6-methoxy-3-methyl-1H-indazole (620 mg, 3.82 mol) in CH₂Cl₂ (25 mL) at 0 °C was added a dichloromethane solution of boron tribromide (17 mL of 1 M solution). The mixture was stirred at room temperature overnight. The solution was carefully quenched by pouring slowly into iced saturated aqueous NaHCO₃. The phases were separated and the aqueous phase was extracted with EtOAc (3x). The combined organic extracts were concentrated and the crude material was purified by Biotage chromatography (40S column, acetone/heptane 45% 500 mL and 60% 150 mL) to provide 3-methyl-1H-indazol-6-ol as an orange solid (458 mg, 81%).

A solution of 3-methyl-1H-indazol-6-ol (458 mg, 3.1 mmol) in THF (30 mL) was treated with sodium hydride (0.50 g of 60% oil dispersion). After the initial effervescence had subsided, the solution was heated to 50 °C for 1 hour before cooling to room temperature and adding N-phenyltrifluoromethane sulphonimide (2.50 g, 7.0 mmol). The mixture was stirred at room temperature for 2 hours before pouring into water. The aqueous phase was extracted with EtOAc (3x). The combined organic extracts were concentrated and the crude material was purified by Biotage chromatography (40M column, 12% acetone/heptane). To provide 3-methyl-1-(trifluoromethylsulfonyl)-1H-indazol-6-yl trifluoromethanesulfonate (1.13 g, 89%).

A solution of 3-methyl-1-(trifluoromethylsulfonyl)-1 H-indazol-6-yl trifluoromethanesulfonate (0.61 g, 1.5 mmol) in DMF (6 mL) was flushed with carbon dioxide for 5 minutes. To this was added palladium acetate (68 mg, 0.30 mmol), 1,1 -bis(diphenylphosino)ferrocene (167 mg, 0.30 mmol), triethylamine (0.33 g, 0.45 mL, 3.2 mmol), and methanol (4 mL). The solution was stirred at room temperature overnight under one atmosphere of CO. The solution was poured into water and extracted with EtOAc (3x). The combined organic extracts were concentrated and purified by Biotage chromatography (40S column, 8% EtOAc/heptanes) to provide methyl 3-methyl-1-(trifluoromethylsulfonyl)-1H-indazole-6-carboxylate (330 mg, 69%).

To a solution of 3-methyl-1-(trifluoromethylsulfonyl)-1H-indazole-6-carboxylate (590 mg, 1.83 mmol) in MeOH/water (3:1, 72 mL) was added potassium carbonate (1.01 g, 7.31 mmol) and the mixture was heated at reflux for 2 hours. The mixture was cooled to room temperature and methanol was removed under reduced pressure. The aqueous solution was acidified with KHSO₄ to pH 3'- 3.5. The white precipitate that formed was isolated by vacuum filtration, dissolved in EtOAc and washed with water. The organic extract was dried over MgSO₄, filtered, concentrated and dried to yield the title compound as a white solid (259 mg, 80%).

### Acid Preparation 8

### 7-Ethyl-1H-indazole-5-carboxylic acid

To a solution of 2-ethyl-6-methylaniline (2.03 g, 15 mmol) in DMF (50 mL) at 0 C was added N-bromosuccinimide (2.66 g, 14.9 mmol). The mixture was stirred at room temperature for 10 minutes before addition to saturated aqueous NaCl. The mixture was extracted with EtOAc, the organic phase was washed with set aqueous NaCl (2x), concentrated and the crude material was purified by Biotage chromatography (40M, 15% EtOAc/heptane) to provide 4-bromo-2-ethyl-6-methylbenzenamine as a red brown liquid (3.21 g, 100%).

A solution of 4-bromo-2-ethyl-6-methylbenzenamine (3.21 g, 15 mmol) in acetic acid (50 mL) was stirred for 3 hours before addition of a 2 M solution of sodium nitrite (11 mL, 22.5 mmol). The resulting mixture was stirred overnight at room temperature. The solution was concentrated and the solid was dissolved in EtOAc and washed with saturated aqueous NaCl (3x). The organic extract was dried over Na2SO4, filtered and concentrated. the crude material was purified by Biotage chromatography (40M, 15-30% EtOAc/heptane) to provide 5-bromo-7-ethyl-1H-indazole (1.11 g, 33%) and 5-bromo-3,7-dimethyl-1H-indazole (0.84 g, 25%).

To a solution of 5-bromo-7-ethyl-1H-indazole (225 mg, 1.00 mmol) in dioxane (1.5 mL), hexacarbonylmolybdenum (132 mg, 0.50 mmol), Herrmann's catalyst (trans-Bis(acetato)bis[o-(di-o-tolylphosphino)benzyl]dipalladium) (46.9 mg, 0.05 mmol) and a solution of sodium carbonate (318 mg, 3.00 mmol) in water (2 mL). The suspension was sealed and irradiated in a microwave at 165 °C for 15 minutes (high absorption setting). The vial was vented , filtered through diatomaceous earth, washed with EtOAc and concentrated to provide the title compound (140 mg, 74%).

### Acid Preparation 9

### 3-Chloro-1H-indole-5-carboxylic acid

To a solution of indole-6-carboxylic acid in dichloromethane (40 mL) and DMF (4 mL) was added N-chlorosuccinimide and allowed to stir at room temperature 3 hours. The reaction mixture was then concentrated under reduced pressure and the crude material was stirred in dichloromethane (100 mL) for 2 hours, filtered dried overnight to obtain the title compound (1.15 g, 95%).

### Acid Preparation 10

### 3,7-Dimethyl-1H-indazole-5-carboxylic acid

To a reaction vessel containing a re-purified solution of 5-bromo-3,7-dimethyl-1H-indazole (prepared as described for Acid Preparation 8, 285 mg, 1.27 mmol) in dioxane (1.3 mL) was added hexacarbonylmolybdenum (264 mg, 1.0 mmol), Herrmann's catalyst (93 mg, 0.1 mmol)and a solution of Na₂CO₃ in water (636 mg in 2 mL water). The suspension was heated in a microwave at 165 °C for 15 minutes (high absorption). The vial was vented, acidified with 1 N HCl (to pH 2). The reaction mixture was filtered through diatomaceous earth, washed with EtOAc and the organic layer was washed with saturated aqueous NaCl (3x). The organic extract was concentrated to yield the title compound as a pink solid (65 mg, 17%).

### Acid Preparation 11

### 1-methyl-1H-indole-6-carboxylic acid

To N-methylindole-6-carboxylic acid methyl ester in 3 mL 1:1:1:1 acetonitrile/THF/water/MeOH was added LiOH. and allowed to stir at room temperature over the weekend. The reaction mixture was concentrated under reduced pressure before adding EtOAc and water. The organic phase was separated and the water acidified with 1 N HCl and extracted into EtOAc (3x50 mL), washed with water, saturated aqueous NaCl, dried over MgSO₄, filtered, and concentrated under reduced pressure. The material was dried under reduced pressure to afford the title product (0.16 g, 86%).

### Acid Preparation 12

### 1-Methyl-2-pyrrolidin-1-yl-1H-benzimidazole-5-carboxylic acid

Methyl 3-nitro-4-chlorobenzoate (72.01 g, 0.334 mol) was suspended in freshly distilled acetonitrile (360 mL) under stirring. Anhydrous sodium acetate (41.1 g, 0.5 mol) and 30% aqueous solution of methylamine (69 mL, 0.67 mol) were added to this suspension under vigorous stirring. The obtained mixture was refluxed for 7 hours and then kept overnight with TLC monitoring (chloroform/CCl₄ 1:2). The yellow precipitate was separated by filtration and mixed with a solution of K₂CO₃ (25 g) in water (500 mL). The mixture was stirred for 30 minutes and filtered. The yellow precipitate was washed with water to attain pH 7. The filtrate was concentrated under a reduced pressure to a volume of about 200 mL and mixed with a solution of K₂CO₃ (5 g) in water (100 mL). The mixture was stirred for 30 minutes and filtered. The yellow precipitate was washed with water to attain pH 7. Two above precipitates were combined and dried to give methyl 4-(methylamino)-3-nitrobenzoate as a yellow powder in (67.63 g, 96%).

4-(Methylamino)-3-nitrobenzoate (63.06 g, 0.3 mol) was suspended under vigorous stirring in methanol (700 mL). A suspension of Raney nickel (15 g, freshly prepared by treatment of nickel-aluminum 50/50 alloy with the 2N NaOH solution) in methanol (30 mL) was added to the suspension. The obtained mixture was heated to 40 45 °C under vigorous stirring, and hydrazine monohydrate (60 mL, 1.2 mol) was added drop wise to the suspension for 3 hours at a temperature below 55 °C. The mixture was stirred at 50 55 °C for 3 hours and kept overnight at room temperature. The reaction mixture was heated again to 40 45 °C under vigorous stirring, and an additional amount of hydrazine hydrate (5 mL) was added to the mixture. The suspension was refluxed for 2 hours under vigorous stirring, cooled, and diluted with chloroform (1 L). The mixture was passed through diatomaceous earth (upper layer 2 cm, diameter 17 cm) and silica gel (lower layer 5 cm) to remove Raney nickel. The layers were washed with chloroform/methanol mixture (1:1, 5 × 600 mL). The filtrate was concentrated under a reduced pressure. The residue was diluted with benzene (100 mL), and the mixture was concentrated under a reduced pressure to remove water. This operation was repeated to give methyl 3-amino-4-(methylamino)benzoate as a brown crystalline solid in (53.6 g, 99%) which was used in the next stage without additional purification.

Methyl 3-amino-4-(methylamino)benzoate (53.6 g, 0.3 mol) was dissolved in anhydrous dichloromethane (700 mL). 1,1 Carbonyldiimidazole (CDI, 62.59 g, 0.386 mol) was added to this solution in several small portions under stirring for 2 hours. The reaction mixture was stirred at room temperature overnight. The formed precipitate was separated by filtration, washed with cold ether (3 × 50 mL), and dried to give methyl 1-methyl-2-oxo-2,3-dihydro-1H-benzimidazole-5-carboxylate as light-pink crystals in (49.75 g, 81% ).

Phosphoryl bromide (POBr₃, 102.4 g, 0.357 mol) was dissolved in dichloroethane (400 mL). Methyl 1-methyl-2-oxo-2,3-dihydro-1H-benzimidazole-5-carboxylate (36.7 g, 0.178 mol) was added to this solution in several small portions under stirring, and the obtained suspension was refluxed with TLC monitoring (chloroform/1;2-dimethoxyethane 10:1). After the reaction was completed (about 19 hour), the reaction mixture was cooled in an ice-bath and carefully neutralized for 3 hours with water (50 mL), and then with a solution of Na₂CO₃ (100 g) in water (800 mL) intense foaming observed. The quenched mixture was extracted with chloroform (2 L). The layers were separated, and the aqueous layer was extracted again with chloroform (500 mL). The organic layers were combined, washed with water (3 x 250 mL), and dried over CaCl₂. The organic solution was concentrated under a reduced pressure. The resulting pale-gray solid was recrystallized from acetonitrile to give methyl 2-bromo-1-methyl-1H-benzimidazole-5-carboxylate as a white solid in (37.1 g, 77.5%).

A mixture of methyl 2-bromo-1-methyl-1H-benzimidazole-5-carboxylate (40.0 g, 0.149 mol), pyrrolidine (25.37 g, 30 mL, 0.357 mol), cesium fluoride CsF (31.61 g, 0.208 mol), and DMSO (240 mL) was placed into a microwave reactor (MILESTONE Microwave Labstation; Shelton, CT). The reaction mixture was treated with microwave radiation under stirring at an internal temperature of 115 °C for 8 h, cooled, and poured into ice-cold water (1 L). The formed precipitate was separated by filtration, washed with cold water (2 × 50 mL), hexane (2 × 100 mL), and dried. The product was mixed with ether (250 mL) and acetonitrile (20 mL), and the mixture was placed into an ultrasonic bath for 1.5 hours. The precipitate was separated by filtration, washed with ether (2 × 50 mL), and dried to give methyl 1-methyl-2-pyrrolidin-1-yl-1H-benzimidazole-5-carboxylate in (28.82 g, 75%).

A suspension of methyl 1-methyl-2-pyrrolidin-1-yl-1H-benzimidazole-5-carboxylate (28.8 g, 0.111 mol) in methanol (200 mL) was mixed with a sotution of KOH (12.44 g, 0.222 mol) in water (200 mL). The mixture was refluxed for 3 hours and kept overnight at room temperature. The reaction mixture was concentrated under a reduced pressure to remove methanol. The residue was mixed with a solution of KHSO₄ (30.21 g, 0.222 mol) in water (200 mL), and the mixture was stirred for 1 hours. The reaction mixture was concentrated under a reduced pressure to dryness, and the product was extracted from the solid residue with a warm mixture of chloroform and isopropanol (1:1, about 7 L). The obtained extract was concentrated under a reduced pressure, and the residue was dissolved in a boiling mixture of dichloromethane and isopropanol (1:1, 500 mL). The solution was refluxed for 30 minutes and cooled in a freezer. The formed precipitate was separated by filtration and dried to give 1-methyl-2-pyrrolidin-1-yl-1H-benzimidazole-5-carboxylic acid as a pale-yellow crystalline solid in (18.3 g, 67%).

### Acid Preparation 13

### 3-chloro-1H-indole-6-carboxylic acid

To a solution of 1H-indole-5-carboxylic acid in dichloromethane (20 mL) and DMF (2 mL) was added N-chlorosuccinimide and allowed to stir at room temperature for 3 hours. The reaction mixture was concentrated under reduced pressure. The crude material was stirred in dichloromethane (100 mL) overnight, filtered and dried to provide the title compound (0.439 g, 72%).

### Acid Preparation 14

### 1-Methyl-1H-indazole-6-carboxylic acid

methyl 1H-indazole-6-carboxylate was prepared according to the procedure disclosed in J. Med. Chem. 2000, 43 (1), 41-58 (example 12b, page 49). Alkylation was done under standard conditions (sodium hexamethyldisilazide, THF, iodomethane, reflux) provided methyl 1-methyl-1H-indazole-6-carboxylate (43%). Saponification was done under standard conditions (1 N NaOH) afforded the title product (96%).

### Acid Preparation 15

### 1,3-Dimethyl-1H-indazole-6-carboxylic acid

Methanesulfonic acid 2-acetyl-5-bromophenyl ester was prepared as described in International Application Publication Number WO 2005/090305 (Example 40a). Methanesulfonic acid 2-acetyl-5-bromophenyl ester was then treated with methylhydrazine and ammonium acetate at reflux for 6 days to provided 6-bromo-1,3-dimethyl-1H-indazole (60%). A solution of 6-bromo-1,3-dimethyl-1H-indazole in THF was treated with n-BuLi followed by carbon dioxide to afford the title compound (1.23 g, 60%).

### Acid Preparation 16

### 3-Ethyl-1-methyl-1H-indazole-6-carboxylic acid

5-bromo-2-propionylphenyl methanesulfonate was treated with methylhydrazine and ammonium acetate at reflux for 6 days provided 6-bromo-3-ethyl-1-methyl-1H-indazole (33%). A solution of 6-bromo-3-ethyl-1-methyl-1H-indazole in THF was treated with n-BuLi followed by carbon dioxide to afford the title compound (66%).

### Acid Preparation 17

### 2-Fluoro-5-(1H-pyrazol-3-yl)-benzoic acid

5-Bromo-2-fluorobenzoic acid (100 g, 0.457 mol) was added to a solution of HCl in methanol (400 mL; about 11 %). The suspension was refluxed for 8 hours, and then evaporated *in vacuo.* The residue was dissolved in benzene (500 mL), and the solution was washed with aqueous K₂CO₃ solution (2 × 50 mL) and water (3 × 100 mL), dried over Na₂SO₄ and evaporated in vacuo to give methyl 5-bromo-2-fluorobenzoate as a yellow oil in 88% (93.7 g) yield.

Methyl 5-bromo-2-fluorobenzoate (154.2 g, 0.66 mol), dry benzene (450 mL), ethynyl(trimethyl)silane (78.0 g, 0.79 mol), diisopropylamine (100 g, 0.99 mol) and tetra(triphenylphosphine)palladium (20.0 g, 0.017 mol) were placed under an atmosphere of argon in a three-necked round-bottomed 1 liter flask, equipped with a magnetic stirrer and a thermometer. The mixture was stirred for 30 minutes and then cooled to 10 °C. Copper iodide (12.5 g, 0.066 mol) was added, and the obtained suspension was stirred for 2.5 hours at 20 °C and then at 60 °C for 3 hours and finally left to stand at room temperature overnight. After this, the mixture was diluted with ether (200 mL), and the precipitate was separated by filtration and washed with ether (2 × 100 mL). The obtained organic solution (800 mL) was washed with saturated aqueous solutions of NH₄Cl and NaCl, dried over Na₂SO₄ and evaporated. The crude product was purified by chromatography (hexane/ethylacetate 10:1) on a silica gel column to give methyl 2-fluoro-5-(2-trimethylsilyl)ethynyl)benzoate containing about 13% of methyl 5-bromo-2-fluorobenz4ate, in about 80% (148.1 g) yield.

A suspension of 2-fluoro-5-(2-trimethylsilyl)ethynyl)benzoate (171.1 g, 0.684 mol), mercury(2+) diacetate (12.0 g, 0.051 mol) in THF (400 mL) and concentrated H₂SO₄ (74 mL, 1.37 mol) was stirred at 50-60 °C for 2 h. Then the mixture was cooled, and THF (350 mL) was evaporated in vacuo. The residue was diluted with ether (700 mL) and filtered to remove mercury salts, which were washed from acid. The ether solution was then dried over-Na₂SO₄ and concentrated. The crude product (125 g) was purified by chromatography on a silica gel column, eluting at first with hexane/ethyl acetate (10:1) mixture to remove admixture of methyl 5-bromo-2-fluorobenzoate, and then with hexane/ethyl acetate (1:1) to give methyl 5-acetyl-2-fluorobenzoate in 75% (90.0 g) yield.

(Dimethoxymethyl)dimethylamine (92 mL, 0.69 mol) was added to a suspension of methyl 5-acetyl-2-fluorobenzoate (90.0 g, 0.46 mol) in toluene (90 mL). The mixture was refluxed for 7 hours, during which time forming methanol was distilled off. The solution was then concentrated in vacuo, and the residue (115.2 g) was purified by crystallization to give methyl 5-(3-(dimethylamino)acryloyl)-2-fluorobenzoate as yellow prisms in 80% (93.9 g) yield.

A mixture of methyl 5-(3-(dimethylamino)acryloyl)-2-fluorobenzoate (50 g, 0.2 mol), hydrazine hydrate (11.0 g, 0.22 mol) in methanol (500 mL) was allowed to stand at 20 °C for 48 hours. Then solvent was evaporated, and the residue was purified by chromatography (ethylacetate/hexane 1:2) on a silica gel column to afford 22.0 g of methyl 2-fluoro-5-(1 H-pyrazol-3-yl)benzoate, containing an impurity. The product was purified by crystallization from ethanol to give methyl 2-fluoro-5-(1H-pyrazot-3-yl)benzoate as yellow prisms in 45% (19.9 g) yield.

A solution of methyl 2-fluoro-5-(1H-pyrazol-3-yl)benzoate (25.2 g, 0.115 mol) was refluxed for 2 hours in concentrated HCl-(150 ml). Then the reaction mixture was cooled and filtered. The separated precipitate was washed with ethanol, dried and then refluxed in water (200 mL) for 30 minutes to remove traces of methyl ester and HCl, cooled and filtered. The separated precipitate was washed with water and dried to give the title compound in 90.7% (21.4 g) yield.

### Acid Preparation 18

### 2-Chloro-5-(1H-pyrazol-3-yl)-benzoic acid

Ethyl 5-bromo-2-chlorobenzoate (100 g, 0.38 mol), dry benzene (450 mL), ethynyl(trimethyl)silane (44:7 g, 0.45 mol), piperidine (38.3 g, 0.45 mol) and tetra(triphenylphosphine)palladium (22.0 g, 0.019 mol) were placed under an atmosphere of Ar in a three-necked round-bottomed 1 liter flask, equipped with a magnetic stirrer and a thermometer. The mixture was stirred for 30 minutes and then cooled to 0 °C. Copper.iodide (7.23 g, 0.038 mol) was added, and the obtained suspension was stirred for a further 2.5 hours at 30-35 °C and filtered. The separated precipitate was washed with benzene, and the combined filtrate was washed with saturated aqueous solutions of NH₄Cl and NaCl, dried over Na₂SO₄ and evaporated. The crude product was purified by chromatography (hexane/ethylacetate 10:1) on a silica gel column to give ethyl 2-chloro-5-(2-(trimethylsilyl)ethynyl)benzoate in 95% (101 g) yield.

A suspension of ethyl 2-chloro-5-(2-(trimethylsilyl)ethynyl)benzoate (101 g; 0.36 mol), mercury(2+) diacetate (8.6 g, 0.027 mol) in THF (250 mL) and concentrated H₂SO₄ (40 mL) was stirred at 60 °C for 3 hours. Then the mixture was cooled, diluted with ether (500 mL) and washed to obtain neutral medium. Then the solution was dried over Na₂SO₄ and evaporated. The residue was purified by chromatography (hexane/ethylacetate 4: 1) on a silica gel column to give ethyl 5-acetyl-2-chlorobenzoate in 70% (57 g) yield.

(Dimethoxymethyl)dimethylamine (40 mL) was added to a suspension of ethyl 5-acetyl-2-chlorobenzoate (57 g, 0.25 mol) in toluene (60 mL). The mixture was refluxed for 9 hours, during which time forming methanol was distilled off. The solution was then concentrated in vacuo, and the residue was purified by chromatography (ethyl acetate) on a silica gel column to afford ethyl 2-chloro-5-(3-(dimethylamino)acryloyl)benzoate as yellow prismatic crystals in 80% (57.6 g) yield.

Hydrazine hydrate (5.5 g, 0.11 mol) was added to a suspension of ethyl 2-chloro-5-(3-(dimethylamino)acryloyl)benzoate (28 g, 0.1 mol) in ethanol (100 mL). The reaction mixture was left to stand at 20 °C overnight and then concentrated. The residue was purified by chromatography (ethylacetate/hexane 1:2) on a silica gel column to afford ethyl 2-chloro-5-(1H-pyrazol-3-yl)benzoate in 94% (22.9 g) yield.

A suspension of compound ethyl 2-chloro-5-(1H-pyrazol-3-yl)benzoate (22.9 g, 0.091 mol), sodium methylate (7.4 g, 0.14 mol) in ethanol (250 mL) was refluxed for 10 minutes. The formed precipitate was separated and dissolved in water (1 L). The obtained aqueous solution was acidified with concentrated HCl to pH about 2 that caused precipitation. The precipitate was separated by filtration, washed with water and dried to give 2-chloro-5-(1H-pyrazol-3-yl)benzoic acid as yellowish crystals in 92% (18.9 g) yield.

### Acid Preparation 19

### 2-Hydroxy-5-(1H-pyrazol-3-yl)-benzoic acid

A mixture of methyl 5-acetyl-2-hydroxybenzoate (1; 20 g, 0.1341 mol), (chloromethyl)benzene (17.82 g, 0.1410 mol) and Na₂CO₃ (17.1 g, 0.1613 mol) in DMF (50 mL) was heated (110-115 °C) under stirring for 2 hours. Then the reaction mixture was cooled, and the residue was removed by filtration and washed with DMF (15 mL). The combined filtrate was evaporated in vacuo, and water (100 mL) was added to the residue. The obtained mixture was evaporated in vacuo to remove (chloromethyl)benzene. Next water traces were removed by co-evaporation with toluene (2 × 100 mL) in vacuo. The residue was triturated with hexane (100 mL), separated by filtration and dried to give methyl 5-acetyl-2-(benzyloxy)benzoate in 84.7% (27.17 g) yield.

(Dimethoxymethyl)dimethylamine (28.70 g, 32.0 mL, 0.241 mol) was added to a suspension of methyl 5-acetyl-2-(benzyloxy)benzoate (27.17 g, 0.1135 mol) in DMF (30 mL). The mixture was heated (110 - 115 °C) under stirring for 8 hours, during which time the forming methanol was distilled off. Next the solution was concentrated in vacuo, and the residue was treated with dry ether (150 mL), separated by filtration and washed with dry ether (30 mL) again to give methyl 2-(benzyloxy)-5-(3-(dimethylamino)-acryloyl)benzoate as yellow prisms in 82.1% (31.63 g) yield.

A mixture of methyl 2-(benzyloxy)-5-(3-(dimethylamino)acryloyl)benzoate (68.1 g, 0.20 mol) and hydrazine hydrate (11.0 g, 0.22 mol) in methanol (600 mL) was left to stand at room temperature for 48 hours. Then the solvent was removed, and the residue was purified by chromatography (ethylacetate) on a silica gel column to afford methyl 2-(benzyloxy)-5-(1H-pyrazol-3-yk)benzoate in 70% (43.0 g) yield.

A suspension of methyl 2-(benzyloxy)-5-(1H-pyrazol-3-yk)benzoate (43.0 g, 0.139 mol) was refluxed in concentrated HCl (250 mL) for 2.5 hours, removing benzyl chloride. The reaction mixture was then cooled, and the formed precipitate was separated by filtration, washed with water and dried to give the title compound in 75.4% (27.0 g) yield.

### Acid Preparation 20

### 2-Chloro-5-(1-methyl-1H-pyrazol-3-yl)-benzoic acid

A mixture of ethyl 2-chloro-5-(3-(dimethylamino)acryloyl)benzoate, prepared as described in Preparation 15, (31.7 g, 0.13 mol), methylhydrazine (12.44 g, 0.32 mol) in methanol (120 mL) was allowed to stand at room temperature for 24 hours. Then solvent was evaporated, and the residue was purified by chromatography (ethylacetate/hexane 1:2) on a silica gel column to afford ethyl 2-chloro-5-(1-methyl-1H-pyrazol-3-yl)benzoate in 28% (9.6 g) yield.

A suspension of ethyl 2-chloro-5-(1-methyl-1H-pyrazol-3-yl)benzoate (9.6 g, 0.046 mol), sodium methylate (4.8 g, 0.07 mol) in ethanol (150 mL) was refluxed for 30 minutes. The precipitate was separated by filtration and dissolved in water. The aqueous solution was acidified with concentrated HCl to pH about 2 that caused precipitation. The precipitate was separated by filtration, washed with water and dried to give the title compound as crystals in 92% yield.

### Acid Preparation 21

### 3-Pyrimidin-4-yl-benzoic acid

Methyl 3-bromobenzoate (110 g, 0.51 mol), dry acetonitrile (500 mL), ethynyl(trimethyl)silane (60.0 g, 0.61 mol), diisopropylamine (62.0 g, 0.61 mol) and tetra(triphenylphosphine)palladium (23.6 g, 0.02 mol) were placed under an atmosphere of argon in a three-necked round-bottomed 1 liter flask, equipped with a magnetic stirrer and a thermometer. The mixture was stirred for 30 minutes and then cooled to 10 °C. Copper iodide (9.7 g, 0.06 mol) was added, and the obtained suspension was stirred for a further 2.5 hours at 20 °C and finally for 3 hours at 60 °C. Then the mixture was left to stand at room temperature overnight and filtered. The precipitate of hydrobromide was washed with ether, and the combined filtrate was washed with saturated aqueous solutions of NH₄Cl and NaCl, dried over Na₂SO₄ and evaporated. The crude product was purified by chromatography (hexane) on a silica gel column to give methyl 3-(2-(trimethylsilyl)ethynyl)benzoate in 95% (112.8 g) yield.

A suspension of methyl 3-(2-(trimethylsilyl)ethynyl)benzoate (112.8 g, 0.48 mol), mercury(2+) diacetate (16.2 g, 0.005 mol) in THF (350 mL) and concentrated H₂SO₄ (40 mL) was stirred at 60 °C for 3 hours. Then the mixture was cooled, diluted with ether (500 mL), filtered to remove precipitated mercury salts and washed to obtained neutral medium. Then the solution was dried over Na₂SO₄ and evaporated. The residue was purified by chromatography (hexane/ethyl acetate 4:1) on a silica gel column to give methyl 3-acetylbenzoate in 75% (65.2 g) yield.

(Dimethoxymethyl)dimethylamine (90 mL) was added to a suspension of methyl 3-acetylbenzoate (65.2 g, 0.27 mol) in toluene (90 mL). The mixture was refluxed for 9 hours, during which time forming methanol was distilled off. The solution was then concentrated in vacuo, and the residue was purified from ether by crystallization to give methyl 3-(3-(dimethylamino)acryloyl)benzoate as yellow prismatic crystals in 80% (68.1 g) yield.

Imidoformamide acetate (20.3 g, 0.19 mol) was added to a suspension of methyl 3-(3-(dimethylamino)acryloyl)benzoate (30.3 g, 0.13 mol) in toluene (300 mL). The reaction mixture was refluxed for 20 hours, during which time toluene and dimethylamine acetate were distilled off. Then imidoformamide acetate (6.7 g) and toluene (175 mL) were added again, and after 8 hours the mixture was evaporated in vacuo. The residue was purified by chromatography (ethylacetate/hexane 3:1) on a silica gel column to afford methyl 3-(pyrimidin-4-yl)benzoate in 70% (19.5 g) yield.

A suspension of methyl 3-(pyrimidin-4-yl)benzoate (19.5 g, 0.091 mol), sodium methylate (7.6 g, 0.14 mol) in ethanol (250 mL) was refluxed for 30 minutes. Then the reaction mixture was cooled, and the formed precipitate was separated by filtration was dissolved in water. The obtained solution was acidified with concentrated HCl to pH about 2 that caused precipitation. The precipitate was separated by filtration, washed with water and dried to give the title compound as crystals in 94% (17.2 g) yield.

### Acid Preparation 22

### 1-Methyl-2-pyrrolidin-1-yl-1H-benzimidazole-5-carboxylic acid

Methyl 3-nitro-4-chlorobenzoate (72.01 g, 0.334 mol) was suspended in freshly distilled acetonitrile (360 mL) under stirring. Anhydrous sodium acetate (41.1 g, 0.5 mol) and 30% aqueous solution of methylamine (69 mL, 0.67 mol) were added to this suspension under vigorous stirring. The obtained mixture was refluxed for 7 hours and then kept overnight with TLC monitoring (chloroform/CCl₄ 1:2). The yellow precipitate was separated by filtration and mixed with a solution of K₂CO₃ (25 g) in water (500 mL). The mixture was stirred for 30 minutes and filtered. The yellow precipitate was washed with water to attain pH 7. The filtrate was concentrated under a reduced pressure to a volume of about 200 mL and mixed with a solution of K₂CO₃ (5 g) in water (100 mL). The mixture was stirred for 30 minutes and filtered. The yellow precipitate was washed with water to attain pH 7. Two above precipitates were combined and dried to give methyl 4-(methylamino)-3-nitrobenzoate as a yellow powder in 96% (67.63 g) yield.

Methyl 4-(methylamino)-3-nitrobenzoate (63.06 g, 0.3 mol) was suspended under vigorous stirring in methanol (700 mL). A suspension of Raney nickel (15 g, freshly prepared by treatment of nickel-aluminum 50/50 alloy with the 2N NaOH solution) in methanol (30 mL) was added to the suspension. The obtained mixture was heated to 40 45 °C under vigorous stirring, and hydrazine monohydrate (60 mL, 1.2 mol) was added drop wise to the suspension for 3 hours at a temperature below 55 °C. The mixture was stirred at 50 55 °C for 3 hours and kept overnight at room temperature. The reaction mixture was heated again to 40 45 °C under vigorous stirring, and an additional amount of hydrazine hydrate (5 mL) was added to the mixture. The suspension was refluxed for 2 hours under vigorous stirring, cooled, and diluted with chloroform (1 L). The mixture was passed through diatomaceous earth (upper layer 2 cm, diameter 17 cm) and silica gel (lower layer 5 cm) to remove Raney nickel. The layers were washed with chloroform/methanol mixture (1:1, 5 x 600 mL). The filtrate was concentrated under a reduced pressure. The residue was diluted with benzene (100 mL), and the mixture was concentrated under a reduced pressure to remove water. This operation was repeated to give methyl 3-amino-4-(methylamino)benzoate as a brown crystalline solid in 99% (53.6 g) yield. Methyl 3-amino-4-(methylamino)benzoate was used at the next stage without additional purification.

Methyl 3-amino-4-(methylamino)benzoate (53.6 g, 0.3 mol) was dissolved in anhydrous dichloromethane (700 mL). 1,1-Carbonyldiimidazole (CDI, 62.59 g, 0.386 mol) was added to this solution in several small portions under stirring for 2 h. The reaction mixture was stirred at room temperature overnight. The formed precipitate was separated by filtration, washed with cold ether (3 x 50 mL), and dried to give methyl 1-methyl-2-oxo-2,3-dihydro-1*H*-benzimidazole-5-carboxylate as light-pink crystals in 81% (49.75 g) yield.

Phosphoryl bromide (POBr₃, 102.4 g, 0.357 mol) was dissolved in dichloroethane (400 mL). Methyl 1-methyl-2-oxo-2,3-dihydro-1*H*-benzimidazole-5-carboxylate (36.7 g, 0.178 mol) was added to this solution in several small portions under stirring, and the obtained suspension was refluxed with TLC monitoring (chloroform/1,2-dimethoxyethane 10:1). After the reaction was completed (about 19 hours), the reaction mixture was cooled in an ice bath and carefully neutralized for 3 hours with water (50 mL), and then with a solution of Na₂CO₃ (100 g) in water (800 mL) with intense foaming observed. The obtained mixture was extracted with chloroform (2 L). The layers were separated, and the aqueous layer was extracted again with chloroform (500 mL). The organic layers were combined, washed with water (3 × 250 mL), and dried over CaCl₂. The organic solution was concentrated under a reduced pressure. The resulting pale-gray solid was recrystallized from acetonitrile to give methyl 2-bromo-1-methyl-1*H*-benzimidazole-5-carboxylate as a white solid in 77.5% (37.1 g) yield.

A mixture of methyl 2-bromo-1-methyl-1*H*-benzimidazole-5-carboxylate (40.0 g, 0.149 mol), pyrrolidine (25.37 g, 30 mL, 0.357 mol), cesium fluoride CsF (31.61 g, 0.208 mol), and DMSO (240 mL) was placed into a microwave reactor. The reaction mixture was treated with microwave radiation under stirring at an internal temperature of 115 °C for 8 h, cooled, and poured into ice-cold water (1 L). The formed precipitate was separated by filtration, washed with cold water (2×50 mL), hexane (2 × 100 mL), and dried. The product was mixed with ether (250 mL) and acetonitrile (20 mL), and the mixture was placed into an ultrasonic bath for 1.5 hours. The precipitate was separated by filtration, washed with ether (2×50 mL), and dried to give methyl 1-methyl-2-pyrrolidin-1-yl-1*H*-benzimidazole-5-carboxylate in 75% (28.82 g) yield.

A suspension of methyl 1-methyl-2-pyrrolidin-1-yl-1*H*-benzimidazole-5-carboxylate (28.8 g, 0.111 mol) in methanol (200 mL) was mixed with a solution of KOH (12.44 g, 0.222 mol) in water (200 mL). The mixture was refluxed for 3 hours and kept overnight at room temperature. The reaction mixture was concentrated under a reduced pressure to remove methanol. The residue was mixed with a solution of KHSO₄ (30.21 g, 0.222 mol) in water (200 mL), and the mixture was stirred for 1 hour. The reaction mixture was concentrated under a reduced pressure to dryness, and the product was extracted from the solid residue with a warm mixture of chloroform and isopropanol (1:1, about 7 L). The obtained extract was concentrated under a reduced pressure, and the residue was dissolved in a boiling mixture of dichloromethane and isopropanol (1:1, 500 mL). The solution was refluxed for 30 minutes and cooled in a freezer. The formed precipitate was separated by filtration and dried to give 1-methyl-2-pyrrolidin-1-yl-1H-benzimidazole-5-carboxylic acid as a pale-yellow crystalline solid in 67% (18.3 g) yield.

### Acid Preparation 23

### 7-Chloro-4-methoxy-2-methyl-1H-benzimidazole-5-carboxylic acid

To concentrated sulfuric acid (10 mL) cooled in an ice/acetone bath was added commercially available methyl 4-(acetylamino)-5-chloro-2-methoxybenzoate (500 mg, 2.0 mmol) and stirred for 5 minutes. To this was added drop wise fuming nitric acid (2 mL) over 10 minutes while keeping the temperature below 10 °C. The reaction mixture was stirred for stirred for an additional 20 minutes at 0 °C. The reaction mixture was carefully poured onto ice (30 mL), the resulting solid was isolated by filtration and dried in vacuo to provide methyl 4-(acetylamino)-5-chloro-2-methoxy-3-nitrobenzoate (497 mg, 85%).

A mixture of methyl 4-(acetylamino)-5-chloro-2-methoxy-3-nitrobenzoate (5.53 g, 18.3 mmol) and Raney nickel (500 mg) in ethanol (95 mL) and water (5 mL) was stirred under 30 pounds per square inch (psi) of hydrogen for 5 hours at room temperature. The reaction mixture was filtered through arbocel and the filtrate was concentrated in vacuo to afford methyl 4-(acetylamino)-3-amino-5-chloro-2-methoxybenzoate (4.60 g, 92%).

Methyl 4-(acetylamino)-3-amino-5-chloro-2-methoxybenzoate (4.60 g, 16.9 mmol) and p-toluenesulfonic acid (290 mg, 1.69 mmol) was dissolved in toluene and heated at reflux for 1 hour. The solvents were evaporated and portioned between CH₂Cl₂ and saturated aqueous NaHCO₃. The organic extract was concentrated to give methyl 7-chloro-4-methoxy-2-methyl-1H-benzimidazole-5-carboxylate (4.21 g, 98%).

A mixture of methyl 7-chloro-4-methoxy-2-methyl-1H-benzimidazole-5-carboxylate (2.00 g, 7.85 mmol) in THF containing 1 M NaOH (12 mL) was heated at reflux for 3 hours. The reaction was not complete, so additional 1 M NaOH (6 mL) was added and the reaction mixture was heated at reflux for an additional 4 hours. The THF was evaporated and the resulting solution was carefully neutralized with concentrated HCl (1.4 mL). A white precipitate appeared after stirring for 10 minutes. Stirring continued at room temperature for 10 minutes before collecting by vacuum filtration and washing with water. The solid was dried under vacuum, with residual water azeotroped with methanol, to afford 7-chloro-4-methoxy-2-methyl-1H-benzimidazole-5-carboxylic acid (1.89 g, 100%).

### Examples

### Preparation of Compounds of Formula (1)

The compounds of Formula (1) were prepared by one of the following six methods using the appropriate carboxylic acids and spiro ketones:

Method A: To 10x75 mm culture tubes was added 500 µL (1 equivalent ("eq")) of a 0.2 M solution of the appropriate carboxylic acid in anhydrous DMF. To this was added 500 µL (0.10 mmol) of a 0.2 M solution of spirocyclic amine 6,7-dimethylspiro[chromene-2,4'-piperidin]-4(3H)-one in anhydrous dimethylformamide (DMF). To this was added 200 µL (1 eq) of a 0.5 M solution of triethylamine in anhydrous DMF. To this was added 200 µL (1 eq) of a 0.5 M O-(7-azabenzotriazol-1-yl)-N,N,N',N'-tetramethyluronium hexafluorophosphate (HATU) solution in anhydrous DMF. The tubes were capped and the reaction mixtures were stirred for 16 hours at room temperature. The volatiles from the tubes were removed using a rotary evaporator system at 55 °C for 4 hours. Dimethylsulfoxide (1540 µL containing 0.01% 2,6-di-t-butyl-4-methylphenol (BHT)) was added to each tube (final theoretical concentration 0.05 M). The tubes were covered with cellophane and agitated for 5 minutes or until the product in each tube was dissolved. Product was analyzed by LC/MS.

Alternately in Method A, the following analysis and purification method was used (hereinafter, "Method A1"). Throughout Method A1, the solvents used were: A: water, B: acetonitrile and C: 1% aqueous trifluoroacetic acid. [percent by volume]

Pre-purification analysis was conducted on a 4.6 × 30 mm Waters (Waters Corp.) X-Bridge C18, 5µm column at a flow rate of 2.5 mL/minute in an injection volume of 2 µL in DMSO at the following gradient: 5% acetonitrile/95% water to 95% acetonitrile/5% water over 3.0 minutes, 1% aq. trifluoroacetic acid/99% water were held at 1%. Detectors used included: diode array detector (DAD), evaporative light scattering detector (ELSD), and time of flight mass spectrometry: electrospray positive mode (TOF MS: ES (+)).

Preparative chromatography was conducted on a 19 × 50 Waters X-Bridge C-18, 5µm at a flow rate of 25 mL/minutes in an injection volume of 900 µL in DMSO (10 - 30 mg) using a gradient that was determined based upon the retention time in pre-purification analyses using DAD, MS: ES (+) detectors with fraction collection triggered by selection ion recording MS; one tube per injection.

| Pro-Purification Retention Time (min) | Purification Method |
|---|---|
| 0.4 - 0.7 | Focused Gradient 1 |
| 0.7 - 1.0 | Focused Gradient 2 |
| 1.0 - 1.4 | Focused Gradient 3 |
| 1.4 - 1.8 | Focused Gradient 4 |
| 1.8 - 2.4 | Focused Gradient 5 |
| 2.4 - 3.0 | Focused Gradient 6 |

| | Time (min) | % A / B / C |
|---|---|---|
| Focused Gradient 1 | 0.0 | 90 / 5 / 5 |
| | 2.0 | 85 / 10 / 5 |
| | 4.0 | 5 / 90 / 5 |
| Focused Gradient 2 | 0.0 | 90 / 5 / 5 |
| | 2.0 | 70 / 25 / 5 |
| | 4.0 | 5 / 90 / 5 |
| Focused Gradient 3 | 0.0 | 85 / 10 / 5 |
| | 2.0 | 55 / 40 / 5 |
| | 4.0 | 5 / 90 / 5 |
| Focused Gradient 4 | 0.0 | 70 / 25 / 5 |
| | 2.0 | 40 / 55 / 5 |
| | 4.0 | 5 / 90 / 5 |
| Focused Gradient 5 | 0.0 | 55 / 40 / 5 |
| | 2.0 | 25 / 70 / 5 |
| | 4.0 | 5 / 90 / 5 |
| Focused Gradient 6 | 0.0 | 40 / 55 / 5 |
| | 2.0 | 10 / 85 / 5 |
| | 4.0 | 5 / 90 / 5 |

Post-purification analysis was conducted on a 4.6 x 30 mm Waters X-Bridge C8, 5 µm column at a flow rate of 2.5 mL/minutes using an injection volume of 2 µL in DMSO using a gradient of 4% B to 95% B over 3.0 minutes, C held at 1%. Detectors used included: DAD, ELSD, TOF MS: ES (+) mode.

Method B: To a flask was added the appropriate amine or amine hydrochloride (1 equivalents), DMF or CH₂Cl₂ (about 0.1 M), carboxylic acid, N,N-diisopropylethylamine (DIEA) (4-6 equivalents) or triethylamine (TEA) (4-6 equivalents) and HATU (1-1.3 equivalents). The mixture was stirred at room temperature until the reaction was complete as determined by LC/MS. The mixture was diluted with ethyl acetate and washed with saturated aqueous NaHCO₃ (2x) and then saturated aqueous NaCl. The organic extract was dried over MgSO₄, filtered and concentrated. The crude material was purified by liquid chromatography to afford product. Alternately, (hereinafter, "Method B1"), the crude reaction mixture was concentrated and directly purified by chromatography as described in Method A1.

Method C: To a solution of a spiro ketone, made by Method B, in MeOH/water (about 0.1 M; V:V 2:1), was added LiOH (1-5 eq.). The solution was heated at 50 °C for 3hours. The reaction mixture was then cooled, concentrated, and purified by column chromatography.

Method D: Into 1 dram vials was added 260 µL of 0.25 M solution of amines dissolved in a 1 M triethylamine solution in CH₂Cl₂. Into this was added 260 µL of a 0.25 M solution of the carboxylic acid in CH₂Cl₂. The mixture was vortexed and into this mixture was added 260 µL of HATU in CH₂Cl₂. The vial was vortexed and then shaken at room temperature for 16 hours. The crude reaction mixture was purified by liquid chromatography to provide the desired product.

Method E: Into a 2.2 mL well in a 96 deep-well plate was added a solution of the carboxylic acid (0.5 mL of 0.5 M DMF solution), a solution of the amine (0.5 mL of 0.5 M DMF solution), and a solution of HATU (0.5 mL of 0.5 M DMF solution). To this was added triethylamine (3 equivalents). The plate was sealed and agitated for 16 hours. The solvents were removed by centrifugal evaporation at reduced pressure. The residues were dissolved in CH₂Cl₂ (1 mL), and washed sequentially with K₂CO₃ (2 × 0.7 mL of 0.5 M solution) and water (0.7 mL) before being transferred to a collection plate. The final aqueous waste was re-extracted with CH₂Cl₂ (0.5 mL), combined with the first CH₂Cl₂ extract and evaporated to dryness.

Method F: To a solution of a spiro ketone in MeOH/water (about 0.1 M; V:V 2:1), was added LiOH (1-5 eq.). The solution was heated at 50 °C for 3 hours. The reaction mixture was then cooled down, concentrated, and purified by column chromatography.

Trifluoroacetic acid salts were obtained for the final products upon HPLC chromatography using an aqueous phase that contained TFA.

### Examples A1-A35

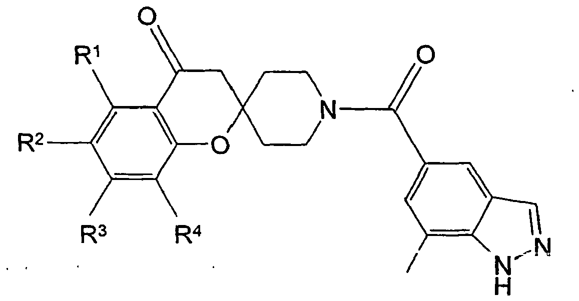

| Ex. | Method | R¹ | R² | R³ | R⁴ | ACC1 IC₅₀ (nM) | ACC1 n* | ACC2 IC₅₀ (nM) | ACC2 n* |
|---|---|---|---|---|---|---|---|---|---|
| A1 | B1 | H | CH₃ | CH₃ | H | 23.5 | 31 | 36.4 | 2 |
| A2 | A | H | CH₃ | CH₃ | H | 30.1 | 1 | | |
| A3 | B | H | OCH(CH₃)₂ | H | H | 14.5 | 1 | 17.2 | 1 |
| A4 | B | H | OCH₂CH₃ | H | H | 19.5 | 1 | 29.9 | 1 |
| A5 | B | H | C(O)NHCH₃ | H | H | 29.5 | 1 | | |
| A6 | B | Cl | H | OCH₃ | H | 31.9 | 1 | | |
| A7 | B | H | OCH₃ | H | H | 32.5 | 2 | 138 | 2 |
| A8 | B | H | Br | CH₃ | H | 32.6 | 2 | | |
| A9 | B | F | OCH₃ | H | H | 37.4 | 2 | | |
| A10 | B | H | C(O)NH2 | H | H | 38.2 | 1 | | |
| A11 | B | H | C(O)OCH₃ | H | H | 40.0 | 1 | | |
| A12 | B | H | OCF₃ | H | H | 41.9 | 2 | | |
| A13 | B | H | Cl | CH₃ | H | 45.0 | 3 | | |
| A14 | B | H | Cl | Cl | H | 52.0 | 1 | | |
| A15 | B | H | CH₃ | H | H | 53.6 | 3 | | |
| A16 | B | H | OCH₃ | H | H | 55.1 | 1 | | |
| A17 | B | H | H | Cl | H | 70.5 | 1 | | |
| A18 | B | CH₃ | Cl | CH₃ | H | 72.0 | 1 | | |
| A19 | B | OCH₃ | H | Cl | H | 81.5 | 1 | | |
| A20 | B | H | CF₃ | H | H | 96.6 | 2 | | |
| A21 | B | H | Cl | F | H | 96.6 | 2 | | |
| A22 | B | H | F | Cl | H | 104 | 1 | | |
| A23 | B | H | i-propyl | H | H | 109 | 1 | | |
| A24 | B | H | H | OCH₃ | H | 122 | 1 | | |
| A25 | B | OCH₃ | H | H | H | 112 | 1 | | |
| A26 | B | H | Cl | H | H | 113 | 1 | | |
| A27 | B | H | C(O)N(CH₃)₂ | H | H | 125 | 1 | | |
| A28 | B | CH₃ | H | OCH₃ | H | 138 | 1 | | |
| A29 | B | CH₃ | H | CH₃ | H | 146 | 1 | | |
| A30 | B | Cl | H | Cl | H | 209 | 1 | | |
| A31 | B | H | -CN | H | H | 234 | 1 | | |
| A32 | B | H | H | -CN | H | 277 | 1 | | |
| A33 | C | H | C(O)OH | H | H | 303 | 1 | | |
| A34 | B | H | H | phenyl | H | 395 | 1 | | |
| A35 | B | H | -S(O)₂CH₃ | H | H | 1110 | 1 | | |

| | | | | | | | | | |
|---|---|---|---|---|---|---|---|---|---|
| * - n is the number of times the assay was performed. | | | | | | | | | |

Ex. A1: Method B1 was used to form 6,7-dimethyl-1'-[(7-methyl-1H-indazol-5-yl)carbonyl]spiro[chromene-2,4'-piperidin]-4(3H)-one as follows. A solution of 6,7-dimethylspiro[chromene-2,4 -piperidin]-4(3H)-one (300 mg, 0.83 mmol) in CH₂Cl₂ (5 mL) was treated with triethylamine (0.70 mL, 5.0 mmol) and O-(7-azabenzotriazol-1-yl)-N,N,N',N'-tetramethyluronium hexafluorophosphate ("HATU") (317 mg, 0.84 mmol). The mixture was stirred at room temperature for 6 hours before removing the solvents under reduced pressured and purified by chromatography to afford the titled compound (50 mg, 48%). ¹H NMR (CDCl₃) δ 8.24 (br s, 1H), 7.71 (s, 1H), 7.59 (s, 1H), 7.33 (s, 1H), 6.80 (s, 1H), 2.66 (m, 3H), 2.26 (s, 3H), 2.20 (s, 3H).

Ex. A2: Method A was used to form 6,7-dimethyl-1'-[(7-methyl-1H-indazol-5-yl)carbonyl]spiro-[chromene-2,4'-piperidin]-4(3H)-one trifluoroacetic acid salt as follows. To 10x75 mm culture tubes was added 400 µL (0.08 mmol) of a 0.2 M solution of 6,7-dimethylspiro[chromene-2,4'piperidin]-4(3H)-one in anhydrous dimethylformamide (DMF) followed by a stir bar. To this was added 400 µL (1 eq) of a 0.2 M solution of the appropriate carboxylic acid in anhydrous DMF. To this was added 160 µL (1 eq) of a 0.5 M solution of triethylamine in anhydrous DMF. To this was added 160 µL (1 eq) of a 0.5 M HATU solution in anhydrous DMF. The tubes were covered with cellophane and the reaction mixtures were stirred for 16 hours. The volatiles from the tubes were removed using a rotary evaporator system with medium heating. Dimethylsulfoxide (1540 µL containing 0.01 % 2,6-di-t-butyl-4-methylphenol (BHT)) was added to each tube (final theoretical concentration 0.05 M). The tubes were covered with cellophane and agitated for 5 minutes or until the product in each tube was dissolved. MS(ACPI) *m*/*z* 404 (M+H)⁺, HPLC RT 1.56 minutes, ¹H NMR (CDCl₃) δ 8.24 (br s, 1H), 7.71 (s, 1H), 7.59 (s, 1H), 7.33 (s, 1H), 6.80 (s, 1H), 2.66 (m, 3H), 2.26 (s, 3H), 2.20 (s, 3H).

Ex. A3: 6-isopropoxy-1'-[(7-methyl-1H-indazol-5-yl)carbonyl]spiro[chromene-2,4'-piperidin]-4(3H)-one, MS(ACPI) m/z (M+H)+ 434, HPLC RT 2.4. ¹H NMR (CDCl₃) δ 8.14 (s, 1H), 7.69 (s, 1H), 7.32 (d, 1H), 7.27-7.28 (m, 2H), 7.10-7.12 (dd, 1H), 6.94-6.96 (d, 1H), 4.49-4.54 (m, 1H), 2.75 (br s, 2H), 2.59 (s, 3H), 1.32-1.33 (d, 6H)

Ex. A4: 6-ethoxy-1'-[(7-methyl-1H-indazol-5-yl)carbonyl]spiro[chromene-2,4'-piperidin]-4(3H)-one, MS(ACPI) m/z (M+H)+ 420 HPLC RT 2.4. ¹H NMR (CDCl₃) δ 8.14 (s, 1H), 7.69 (s, 1H), 7.30-7.31 (d, 1H), 7.12-7.14 (dd, 1H), 6.95-6.97 (d, 1H), 4:01-4:05 (m, 2H), 2.75 (br s, 2H), 2.60 (s, 3H), 1.401.43 (t, 3H)

Ex. A5: N-methyl-1'-[(7-methyl-1H-indazol-5-yl)carbonyl]-4-oxo-3,4-dihydrospiro[chromene-2,4'-piperidine]-6-carboxamide, MS(ACPI) m/z (M+H)+ 433 HPLC RT 1.7

Ex. A6: 5-chloro-7-methoxy-1'-[(7-methyl-1H-indazol-5-yl)carbonyl]spiro[chromene-2,4'-piperidin]-4(3H)-one, MS(ACPI) m/z (M+H)+ 440. ¹H NMR (CDCl₃) δ 8.13 (s, 1H), 7.68 (s, 1H), 7.25 (s, 1H), 6.68 (m, 1H), 6.53-6.54 (d, 1H), 3.91 (s, 3H), 2.73 (s, 2H), 2.58 (s, 3H)

Ex. A7: 6-methoxy-1'-[(7-methyl-1H-indazol-5-yl)carbonyl]spiro[chromene-2,4'-piperidin]-4(3H)-one trifluoroacetic acid salt, MS(ACPI) *m*/*z* 406 (M+H)⁺, 1 H NMR (CDCl3) δ 8.24 (s, 1 H), 7.76 (s, 1 H), 7.35 (s, 1H), 7.32 (d, J=3, 1 H), 7.15 (dd, J=3, 8.8, 1 H), 6.96 (d, J=8.8, 1 H), 2.77 (br s, 2H), 2.63 (s, 3H)

Ex. A8: 6-bromo-7-methyl-1'-[(7-methyl-1H-indazol-5-yl)carbonyl]spiro[chromene-2,4'-piperidin]-4(3H)-one, MS(ACPI) *m*/*z* 486(M+H)⁺, HPLC RT 2.64 minutes

Ex. A9: 5-fluoro-6-methoxy.1'-[(7-methyl-1H-indazol-5-yl)carbonyl]spiro[chromene-2,4'-piperidin]-4(3H)-one, MS(ACPI) *m*/*z* 424 (M+H)⁺, HPLC RT 2.1 minutes

Ex. A10: 1'-[(7-methyl-1H-indazol-5-yl)carbonyl]-4-oxo-3,4-dihydrospiro[chromene-2,4'-piperidine]-6-, carboxamide, MS(ACPI) m/z (M+H)+ 419 HPLC RT 1.6

Ex. A11: methyl 1'-[(7-methyl-1H-indazol-5-yl)carbonyl]-4-oxo-3,4-dihydrospiro[chromene-2,4'-piperidine]-6-carboxylate, MS(ACPI) *m*/*z* 434 (M+H)⁺, HPLC RT 2.15 minutes

Ex. A12: 1'-[(7-methyl-1H-indazol-5-yl)carbonyl]-6-(trifluoromethoxy)spiro[chromene-2,4'-piperidin]-4(3H)-one, MS(ACPI) *m*/*z* 460 (M+H)⁺, HPLC RT 2.5 minutes, ¹H NMR (CDCl₃) δ 8.13 (s, 1H), 7.69-7.74 (m, 2H), 7.38 (dd, J=2.6, 9.3, 1H), 7.07 (d, J=8.8, 1H), 2.80 (s, 2H), 2.59 (s, 3H)

Ex. A13: 6-chloro-7-methyl-1'-[(7-methyl-1H-indazol-5-yl)carbonyl]spiro[chromene-2,4'-piperidin]-4(3H)-one, MS(ACPI) *m*/*z* 424 (M+H)⁺, HPLC RT 2.5 minutes, ¹H NMR (CDCl₃) δ 9.40 (s, 1H), 8.25 (s, 1H), 7.79-7.81 (m, 1H), 7.72 (s, 1H), 7.34 (s, 1 H), 6.91 (s, 1H), 2.72 (s, 2H)

Ex. A14: 6,7-dichloro -1'-[(7-methyl-1H-indazol-5-yl)carbonyl]spiro[chromene-2,4'-piperidin]-4(3H)-one" MS(ACPI) *m*/*z* 444 (M+H)⁺, HPLC RT 2.6 minutes, ¹H NMR (CDCl₃) δ 8.20 (s, 1H), 7.95 (s, 1H), 7.72 (s, 1 H), 7.32 (s, 1 H), 7.22 (s, 1 H), 2.78 (s, 2H), 2.65 (s, 3H)

- Ex. A15: 6-methyl-1'-[(7-methyl-1H-indazol-5-yl)carbonyl]spiro[chromene-2,4'-piperidin]-4(3H)-one, MS(ACPI) *m*/*z* 390 (M+H)⁺, ¹H NMR (CDCl3) δ 8.13 (s, 1H), 7.68-7.69 (m, 2H), 7.34 (dd, J=2.6, 8.3, 1H), 7.27 (s, 1H), 6.93 (d, J=8.8), 2.75 (s, 2H), 2.60 (s, 3H), 2.32 (s, 3H)

Ex. A16: 6-methoxy-1'-[(7-methyl-1H-indazol-5-yl)carbonyl]spiro[chromene-2,4'-piperidin]-4(3H)-one, MS(ACPI) *m*/*z* 406 (M+H)⁺, HPLC RT 2.1 minutes, ¹H NMR (CDCl₃) δ 8.13 (s, 1H), 7.74 (s, 1H), 7.27 (d, J=3.1, 1 H), 7.25 (s, 1H), 7.18 (d, J=3.3, 1 H), 7.16 (d, J=3.1, 1H), 7.04 (s, 1H), 7.02 (s, 1H), 4.91 (s, 2H), 3.78 (s, 3H), 2.60 (s, 3H)

Ex. A17: 7-chloro-1'-[(7-methyl-1H-indazol-5-yl)carbonyl]spiro[chromene-2,4'-piperidin]-4(3H)-one, MS(ACPI) *m*/*z* 410 (M+H)⁺, ¹H NMR (CDCl₃) δ 8.13 (s, 1H), 7.82 (d, J=8.2, 1 H), 7.69 (s, 1H), 7.26 (s, 1H), 7.07 (s, 1H), 7.02-7.03 (m, 1H), 2.77 (s, 2H), 2.58 (s, 3H)

Ex. A18: 6-chloro-5,7-dimethyl-1'-[(7-methyl-1H-indazol-5-yJ)carbonyl]spiro[chromene-2,4'-piperidin]-4(3H)-one, MS(ACPI) *m*/*z* 438(M+H)⁺, HPLC RT 2.7 minutes

Ex. A19: 7-chloro-5-methoxy-1'-[(7-methyl-1H-indazol-5-yl)carbonyl]spiro[chromene-2,4'-piperidin]-4(3H)-one, MS(ACPI) m/z (M+H)+ 440. ¹H NMR (CDCl₃) δ 8.13 (s, 1H), 7.69 (s, 1H), 7.26 (s, 1H), 6.64 (m, 1H), 6.43-6.44 (m, 1 H), 3.86 (s, 3H), 2.76 (s, 2H), 2.59 (s, 3H)

Ex. A20: 1'-[(7-methyl-1H-indazol-5-yl)carbonyl]-6-(trifluoromethyl)spiro[chromene-2,4'-piperidin]-4(3H)-one, MS(ACPI) *m*/*z* 444 (M+H)⁺, HPLC RT 2.5 minutes, ¹H NMR (CDCl₃) δ 8.18 (s, 1H), 8.13 (s, 1H), 7.76 (dd, J=2.0, 8.8, 1H), 7.70 (s, 1H), 7.27)s, 1H), 7.15 (d, J=8.8, 1 H), 2.83 (s, 2H), 2.59 (s, 3H)

Ex. A21: 6-chloro-7-fluoro-1'-[(7-methyl-1H-indazol-5-yl)carbonyl]spiro[chromene-2,4'-piperidin]-4(3H)-one, MS(ACPI) *m*/*z* 426 (M-H)⁻, HPLC RT 2.4 minutes, ¹H NMR (CDCl₃) δ 8.13 (s, 1H), 7.95 (d, J=8.3, 1 H), 7.69 (s, 1 H), 7.27 (s, 1H), 6.85 (d, J=3.1, 1 H), 2.77 (s, 2H), 2.59 (s, 3H)

Ex. A22: 7-chloro-6-fluoro-1'-[(7-methyl-1H-indazol-5-yl)carbonyl]spiro[chromene-2,4'-piperidin]-4(3H)-one, MS(ACPI) *m*/*z* 428 (M+H)⁺, HPLC RT 2.4 minutes, ¹H NMR (CDCl₃) δ 8.17 (s, 1 H), 7.71 (s, 1H), 7.62 (d, J=8.3, 1H), 7.30 (s, 1H), 7.14 (d, J=5.7, 1H), 2.78 (s, 2H), 2.62 (s, 3H)

Ex. A23: 6-isopropyl-1'-[(7-methyl-1H-indazol-5-yi)carbonyl]spiro[chromene-2,4'-piperidin]-4(3H)-one, MS(ACPI) *m*/*z* 418(M+H)⁺, HPLC RT 2.5 minutes

Ex. A24: 7-methoxy-1'-[(7-methyl-1H-indazol-5-yl)carbonyl]spiro[chromene-2,4'-piperidin]-4(3H)-one, MS(ACPI) *m*/*z* 406 (M+H)⁺, HPLC RT 2.0 minutes, ¹H NMR (CDCl₃) δ 8.14 (s, 1H), 7.82 (d, J=8.8, 1H), 7.69 (s, 1H), 7.27 (s, 1H), 6.59 (dd, J=8.8, 2.6, 1H), 6.47 (dd, J=2.0, 1H), 3.87 (s, 3H), 2.73 (s, 2H), 2.60 (s, 3H)

Ex. A25: 5-methoxy-1'-[(7-methyl-1H-indazol-5-yl)carbonyl]spiro[chromene-2,4'-piperidin]-4(3H)-one, MS(ACPI) m/z 406 (M+H)⁺, HPLC RT 1.9 minutes, ¹H NMR (CDCl₃) δ 8.15 (s, 1H), 7.69 (s, 1H), 7.41 (t, J=8.3, 1H), 6.62 (d, J=8.8, 1H), 6.53 (d, J=8.3, 1H), 3.92 (s, 3H), 2.75 (s, 2H), 2.60 (s, 3H)

Ex. A26: 6-chloro-1'-[(7-methyl-1H-indazol-5-yl)carbonyl]spiro[chromene-2,4'-piperidin]-4(3H)-one, MS(ACPI) m/z 410 (M+H)⁺, ¹H NMR (CDCl₃) δ 8.16 (s, 1H), 7.84 (d, J=2.6, 1H), 7.70 (s, 1H), 7.46 (dd. J=8.8, 3.1, 1H), 7.00 (d, J=8.8, 1H), 2.78 (s, 2H), 2.61 (s, 3H)

Ex. A27: N,N-dimethyl-1'-[(7-methyl-1H-indazol-5-yl)carbonyl]-4-oxo-3,4-dihydrospiro[chromene-2,4'-piperidine]-6-carboxamide, MS(ACPI) m/z (M+H)+ 447 HPLC RT 1.7.

Ex. A28: 7-methoxy-5-methyt-1'-[(7-methyl-'1H-indazol-5-yl)carbonyl]spiro[chromene-2,4'-piperidin]-4(3H)-one, MS(ACPI) m/z (M+H)⁺ 420 HPLC RT 2.3. ¹H NMR (CDCl₃) δ 8.13 (s, 1H), 7.69 (s, 1H), 7.27 (s, 1H), 6.36-6.38 (m, 2H), 3.85 (s, 3H), 2.71 (s, 2H), 2.62 (s, 3H), 2.59 (s, 3H)

Ex. A29: 5,7-dimethyl-1'-[(7-methyl-1H-indazol-5-yl)carbonyl]spiro[chromene-2,4'-piperidin]-4(3H).one, MS(ACPI) *m*/*z* 404 (M+H)⁺, HPLC RT 2.5 minutes

Ex. A30: 5,7-dichloro-1'-[(7-methyl-1H-indazol-5-yl)carbonyl]spiro[chromene-2,4'-piperidin]-4(3H)-one, MS(ACPI) *m*/*z* 444 (M+H)⁺, HPLC RT 2.5 minutes

Ex. A31: 1'-[(7-methyl-1H-indazol-5-yl)carbonyl]-4-oxo-3,4-dihydrospiro[chromene-2,4'-piperidine]-6-carbonitrile, MS(ACPI) *m*/*z* 401 (M+H)⁺, HPLC RT 1.9 minutes, ¹H NMR (CDCl₃) δ 8.21-8.23 (m, 2H), 7.77 (dd, J=8.8, 1H), 7.74 (s, 1 H), 7.34 (s, 1H), 7.15 (d, J=8.8, 1 H), 2.85 (s, 2H), 2.66 (s, 3H)

Ex. A32: 1'-[(7-methyl-1H-indazol-5-yl)carbonyl]-4-oxo-3,4-dihydrospiro[chromene-2,4'-piperidine]-7-carbonitrile, MS(ACPI) *m*/*z* 401 (M+H)⁺, HPLC RT 2.3 minutes, ¹H NMR (CDCl₃) δ 8.17 (s, 1H), 7.97 (d, J=7.7, 1H), 7.71 (s, 1H), 7.38 (m, 1H), 7.30-7.32 (m, 1H), 2.84 (s, 2H), 2.62 (s, 3H)

Ex. A33: Method C was used to prepare 1'-[(7-methyl-1H-indazol-5-yl)carbonyl]-4-oxo-3,4-dihydrospiro[chromene-2,4'-piperidine]-6-carboxylic acid trifluoroacetic acid salt. To a solution of methyl 1'-[(7-methyl-1H-indazol-5-yl)carbonyl]-4-oxo-3,4-dihydrospiro[chromene, prepared by Method B, in MeOH/water (1.4 mL, 2:1), was added LiOH (7.5 mg). The solution was heated at 50 °C for 3 hours. The reaction mixture was then cooled down, concentrated, and purified by column chromatography. MS(ACPI) *mlz* 420 (M+H)⁺, HPLC RT 2.0 minutes. MS(ACPI) *mlz* 420 (M+H)⁺, HPLC RT 1.85 minutes.

Ex. A34: 1'-[(7-methyl-1H-indazol-5-yl)carbonyl]-7-phenylspiro[chromene-2,4'-piperidin]-4(3H)-one, MS(ACPI) *m*/*z* 452 (M+H)⁺, HPLC RT 2.6 minutes

Ex. A35: 1'-[(7-methyl-1H-indazol-5-yl)carbonyl]-6-(methylsulfonyl)spiro[chromene-2,4'-piperidin]-4(3H)-one, MS(ACPI) *m*/*z* 454 (M+H)⁺, HPLC RT 1.85 minutes, ¹H NMR (CDCl₃) δ 8.47 (d, J=2.6, 1H), 8.15 (s, 1H), 8.07 (dd, J=1.3, 8.8, 1H), 7.70 (s, 1H), 7.27 (s, 1 H), 7.22 (d, J=8.8, 1 H), 3.08 (s, 3H), 2.86 (s, 2H), 2.60 (s, 3H)

### Examples B1-B14

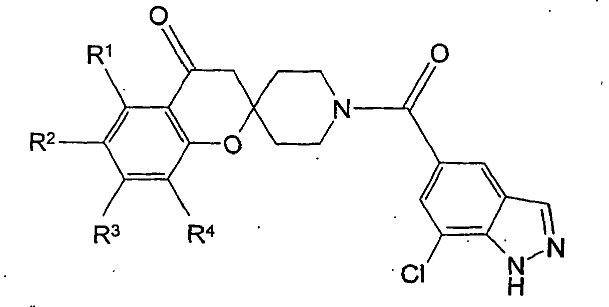

| Ex. | Method | R¹ | R² | R³ | R⁴ | ACC1 IC₅₀ (nM) - | ACC n* | ACC2 IC₅₀ (nM) | ACC2 n* | MS(ACPI) *m*/*z* (M+H)⁺ | HPLC RT (min) |
|---|---|---|---|---|---|---|---|---|---|---|---|
| B1 | B | H | CH₃ | CH₃ | H | 24.1 | 1 | | | 424 | 2.3 |
| B2 | B | H | OCH(CH₃)₂ | n | H | 16.1 | 1 | 31.1 | 1 | 454 | 2.5 |
| B3 | B | H | OCH₂CH₃ | H | H | 21.4 | 1 | 34.3 | 1 | 440 | 2.4 |
| B4 | B | H | Cl | CH₃ | H | 56.8 | 2 | 127 | 1 | 444 | 2.5 |
| B5 | B | H | OCH₃ | H | H | 81.0 | 1 | 231 | 1 | 426 | 2.3 |
| B6 | B | OCH₃ | H | H | H | 109 | 1 | | | 426 | 1.9 |
| B7 | B. | H | H | OCH₃ | H | 166 | 1 | | | 426 | 2.1 |
| 88 | B | H | Cl | H | H | 166 | 1 | | | 428 | 2.5 |
| B9 | B | Cl | H | Cl | H | 180 | 1 | | | 465 | 2.6 |
| B10 | B | H | F | Cl | H | 209 | 1 | | | 448 | 2.4 |
| B11 | B | F | OCH₃ | H | H | 217 | 1 | | | 444 | 2.1 |
| B12 | B | H | CF₃ | H | H | 257 | 3 | | | 464 | 2.5 |
| B13 | B | H | Cl | F | H | 287 | 1 | | | 446 | 2.5 |
| B14 | B | H | CN | H | H | 331 | 1 | | | 421 | 2.2 |

| | | | | | | | | | | | |
|---|---|---|---|---|---|---|---|---|---|---|---|
| * - n is the number of times the assay was performed. | | | | | | | | | | | |

Ex. B1: 1'-[(7-chloro-1H-indazol-5-yl)carbonyl]-6,7-dimethylspiro[chromene-2,4'piperidin]-4(3H)-one ¹H NMR (CDCl₃) 6 8.16 (s, 1H), 7.75 (s, 1H), 7.59 (s, 1H), 7.48 (s, 1H), 6.80 (s, 1H), 5.29 (s, 1H), 2.70 (s, 2H), 2.26 (s, 3H), 2.20 (s, 3H)

Ex. B2: 1'-[(7-chloro-1H-indazol-5-yl)carbonyl]-6-isopropoxyspiro[chromene-2,4'-piperidin]-4(3H)-one ¹H NMR (CDCl₃) δ 8.18 (s, 1 H), 7.78 (s, 1 H), 7.50 (s, 1 H), 7.32-7.33 (d, 1 H), 7.10-7.12 (dd, 1H), 6.94-6.96 (d, 1H), 4.49-4.54 (m, 1H), 2.84 (br s, 2H), 2.75 (br s, 2H), 1.32-1.34 (d, 6H)

Ex. B3: 1'-[(7-chloro-1H-indazol-5-yl)carbonyl]-6-ethoxyspiro[chromene-2,4'-piperidin]-4(3H)-one ¹H NMR (CDCl₃) δ 8.18 (s, 1H), 7.78 (s, 1H), 7.50 (s, 1H), 7.30-7.31 (d, 1H), 7.12-7.15 (dd, 1H), 6.95-6.97 (d, 1 H), 4.01-4.05 (q, 2H), 2.84 (s, 2H), 2.75 (s, 2H), 1.40-1.43 (t, 3H)

Ex. B4: 6-chloro-7-methyl-1'-[(7-chloro-1H-indazol-5-yl)carbonyJ]spirolchromene-2,4'-piperidin]-4(3H)-one ¹H NMR (CDCl₃) δ 8.16 (s, 1H), 7.81 (s, 1 H), 7.74 (s, 1H), 7.47 (s, 1 H), 6.91 (s, 1 H), 2.72 (s, 2H), 2.38 (s, 3H)

Ex. B5: 1'-[(7-chloro-1H-indazol-5-yl)carbonyl]-6-methoxyspiro[chromene-2,4'-piperidin]-4(3H)-one ¹H NMR (CDCl₃) δ 8.16 (s, 1H), 7.75 (m, 1H), 7.48 (m, 1H), 7.28-7.30 (m, 1H), 7.10-7.14 (m, 1H), 6.91-6.95 (m, 1H), 3.79 (s, 3H), 2.73 (s, 2H)

Ex. B6: 1'-[(7-chloro-1H-indazol-5-yl)carbonyl]-5-methoxyspiro[chromene-2,4'-piperidin]-4(3H)-one ¹H NMR (CDCl₃) δ 8.19 (s, 1H), 7.78 (s, 1H), 7.51 (s, 1H), 7.41-7.44 (m, 1H), 6.63 (d, J=8.3, 1H), 6.54 (d, J=8.3, 1 H), 3.93 (s, 3H), 2.75 (s, 2H)

Ex. B7: 1'-[(7-chloro-1H-indazol-5-yl)carbonyl]-7-methoxyspiro[chromene-2,4'-piperidin]-4(3H)-one,'H NMR (CDCl₃) δ 8.18 (s, 1H), 7.83 (d, J=5.2, 1H), 7.78 (s, 1H), 7.50(s, 1H), 6.59-6.62 (m, 1H), 6.47-6.48 (m, 1H), 3.87 (s, 3H), 2.73 (s, 2H)

Ex. B8: 6-chloro-1'-[(7-chloro-1H-indazol-5-yl)carbonyl]spiro[chromene-2,4'-piperidin]4(3H)-one, ¹H NMR (CDCl₃) δ 8.19 (s, 1H), 7.85 (d, J=3.1, 1H), 7.78 (s, 1H), 7.51 (s, 1H), 7.47 (dd, J=8.8, 2.5, 1 H), 7.00 (d, J=8.8, 1H), 2.78 (s, 2H)

Ex. B9: 5,7-dichloro-1'-[(7-chloro-1H-indazol-5-yl)carbonyl]spiro[chromene-2,4'-piperidin]-4(3H)-one, ¹H NMR (CDCl₃) δ 8.18 (s, 1H), 7.77 (s, 1H), 7.50 (s, 1H), 7.08 (d, J=2.1, 1H), 7.01 (d, J=1.5, 1H), 2.81 (s, 1H)

Ex. B10: 7-chloro-1'-[(7-chloro-1H-indazol-5-yl)carbonyl]-6-fluorospiro[chromene-2,4'-piperidin]-4(3H)-one, ¹H NMR (CDCl₃) δ 8.18 (s, 1H), 7.78 (s, 1 H), 7.63 (d, J = 8.3, 1 H), 7.50 (s, 1 H), 7.15 (d, J=5.7, 1H), 2.78 (s, 2H)

Ex. B11: 1'-[(7-chloro-1H-indazol-5-yl)carbonyl]-5-fluoro-6-methoxyspiro[chromene-2,4'-piperidin]-4(3H)-one

Ex. B12: 1'-[(7-chloro-1H-indazol-5-yl)carbonyl]-6-(trifluoromethyl)spiro[chromene-2,4'-piperidin]-4(3H)-one, ¹H NMR (CDCl₃) δ 8.19 (s, 2H), 7.79 (s, 1H), 7.76 (dd, J=8.3, 2.1, 1H), 7.51 (s. 1H), 7.16 (d, J=8.9, 1 H), 2.84 (s, 2H)

Ex. B13: 6-chloro-1'-[(7-chloro-1H-indazol-5-yl)carbonyl]-7-fluorospiro[chromene-2,4'-piperidin]-4(3H)-one, ¹H NMR (CDCl₃) δ 8.15 (s, 1H), 7.90-7.93 (m, 1H), 7.75 (d, J=1.2, 1H), 7.47 (d, J=1.2, 1H), 6.83 (d, J=9.6, 1H), 2.75 (s, 2H)

Ex. B14: 1'-[(7-chloro-1Handazol-5-yl)carbonyl]-4-oxo-3,4-dihydrospiro[chromene-2,4'-piperidine]-6-carbonitrile

### Examples C1-C5

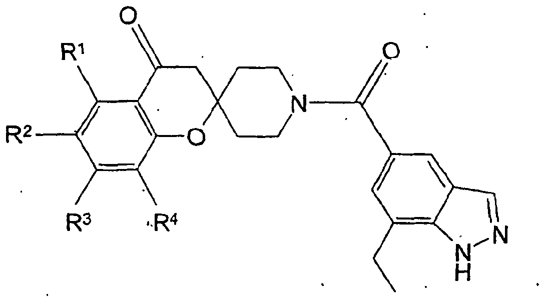

| Ex. | Method | R1 | R2 | R3 | R4 | ACC1 IC₅₀ (nM) | ACC1 n* | MS(ACPI) *mlz* (M+H)⁺ | HPLC RT (min) |
|---|---|---|---|---|---|---|---|---|---|
| C1 | B | H | CH₃ | CH₃ | H | 17.5 | 2 | 418 | 2.5 |
| C2 | B | H | OCH₃ | H | H | 22.6 | 2 | 420 | 2.2 |
| C3 | B | OCH₃ | H | CH₃ | H | 29.5 | 2 | 434 | 2.2 |
| C4 | B | H | Cl | CH₃ | H | 30.6 | 2 | 438 | 2.7 |
| C5 | B | H | CO(O)CH₃ | H | H | 49.8 | 2 | 448 | 2.3 |

| | | | | | | | | | |
|---|---|---|---|---|---|---|---|---|---|
| * - n is the number of times the assay was performed. | | | | | | | | | |

Ex. C1: 1'-[(7-ethyl-1H-indazol-5-yl)carbonyl]-6,7-dimethylspiro[chromene-2,4'-piperidin]-4(3H)-one

Ex. C2: 1'-[(7-ethyl-1H-indazol-5-yl)carbonyl]-6-methoxyspiro[chromene-2,4'-piperidin]-4(3H)-one

Ex. C3: 1'-[(7-ethyl-1H-indazol-5-yl)carbonyl]-5-methoxy-7-methylspiro[chromene-2,4'-piperidin]-4(3H)-one

Ex. C4: 6-chloro-1'-[(7-ethyl-1H-indazol-5-yl)carbonyl]-7-methytspiro[chromene-2,4'-piperidin]-4(3H)-one

Ex. C5: methyl 1'-[(7-ethyl-1H-indazol-5-yl)carbonyl]-4-oxo-3,4-dihydrospiro[chromene-2,4'-piperidine]-6-carboxylate

Examples D1-D3

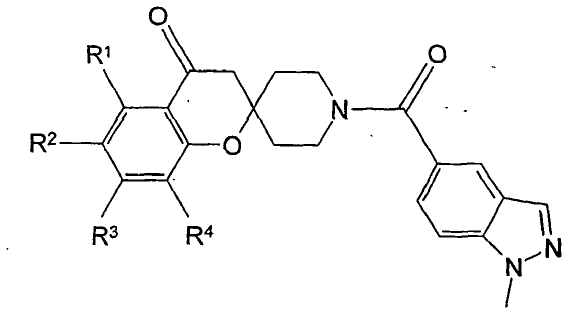

| Ex. | Method | R¹ | R² | R³ | R⁴ | ACC1 IC₅₀ (nM) | ACC1 n* | MS(ACPI) *m*/*z* (M+H)⁺ | HPLC RT (min) |
|---|---|---|---|---|---|---|---|---|---|
| D1 | B | H | CH₃ | CH₃ | H | 57.9 | 1 | 404 | 2.5 |
| D2 | B | H | OCH₃ | H | H | 216 | 1 | 406 | 2.2 |
| D3 | B | H | Cl | CH₃ | H | 250 | 1 | 424 | 2.7 |

| | | | | | | | | | |
|---|---|---|---|---|---|---|---|---|---|
| * - n is the number of times the assay was performed. | | | | | | | | | |

Ex. D1: 6,7-dimethyl-1'-[(1-methyl-1H-indazol-5-yl)carbonyl]spiro[chromene-2,4'-piperidin]-4(3H)-one

Ex. D2: 6-methoxy-1'-[(1-methyl-1H-indazol-5-yl)carbonyl]spiro[chromene-2,4'-piperidin]-4(3H)-one

Ex. D3: 6-chloro-7-methyl-1'-[(1-methyl-1H-indazol-5-yl)carbonyl]spiro[chromene-2,4'-piperidin]-4(3H)-one

### Examples E1-E4

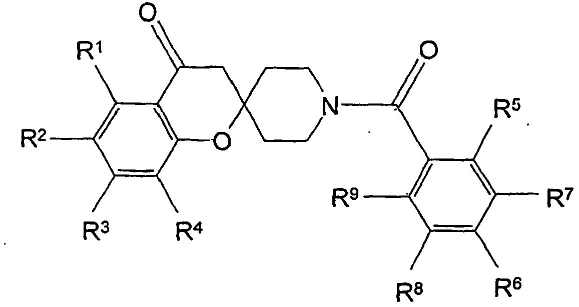

| | | | | | | | | |
|---|---|---|---|---|---|---|---|---|
| Ex. | 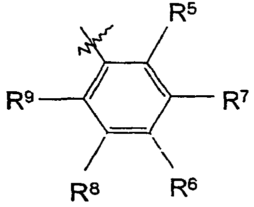 | Method | R¹ | R² | R³ | R⁴ | ACC1 IC₅₀ (nM) | ACC1 n* |
| E1 | 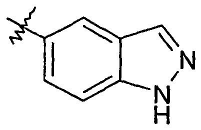 | B | H | CH₃ | CH₃ | H | 96.5 | 2 |
| E2 | 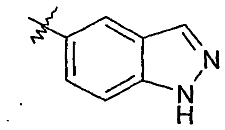 | B | H | Cl | CH₃ | H | 108 | 2 |
| E3 | 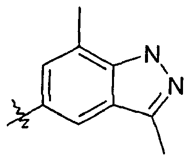 | B | H | CH₃ | CH₃ | H | 9.8 | 1 |
| E4 | 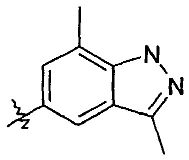 | B | H | C(O)OCH₃ | H | H | 54.3 | 1 |

| | | | | | | | | |
|---|---|---|---|---|---|---|---|---|
| * - n is the number of times the assay was performed. | | | | | | | | |

Ex. E1: 1'-(1H-indazol-5-ylcarbonyl)-6,7-dimethylspiro[chromene-2,4'-piperidin]-4(3H)-one, MS(ACPI) *m*/*z* 390 (M+H)⁺, HPLC RT 2.2 minutes

Ex. E2: 6-chloro-1'-(1H-indazol-5-ylcarbonyl)-7-methylspiro[chromene-2,4'-piperidin]-4(3H)-one, MS(ACPI) *m*/*z* 410 (M+H)⁺, HPLC RT 2.3 minutes

Ex. E3: 1'-[(3,7-dimethyl-1H-indazol-5-yl)carbonyl]-6,7-dimethylspiro[chromene-2,4'-piperidin]-4(3H)-one, MS(ACPI) m/z (M+H)+ 418, HPLC RT 2.4

Ex. E4: methyl 1'-[(3,7-dimethyl-1H-indazol-5-yl)carbonyl]-4-oxo-3,4-dihydrospiro[chromene-2,4'-piperidine]-6-carboxylate, MS(ACPI) m/z (M+H)+ 448 HPLC RT 2.3.

### Examples F1-F5

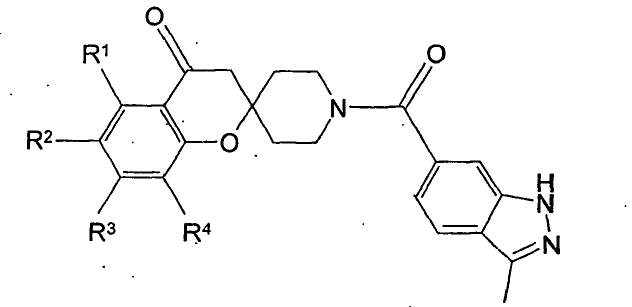

| Ex. | Method | R¹ | R² | R³ | R⁴ | ACC1 IC₅₀ (nM) | ACC1 n* | MS(ACPI) *m*/*z* (M+H)⁺ | HPLC RT (min) |
|---|---|---|---|---|---|---|---|---|---|
| F1 | B | H | CH₃ | CH₃ | H | 35.7 | 1 | 404 | 2.5 |
| F2 | B | H | OCH₃ | H | H | 49.2 | 1 | 406 | 2.1 |
| F3 | B | H | Cl | CH₃ | H | 107 | 1 | 424 | 2.5 |
| F4 | B | H | C(O)OCH₃ | H | H | 126 | 1 | 434 | 2.3 |
| F5 | C | H | C(O)OH | H | H | 586 | 1 | 420 | 2.0 |

| | | | | | | | | | |
|---|---|---|---|---|---|---|---|---|---|
| * - n is the number of times the assay was performed. | | | | | | | | | |

Ex. F1: 6,7-dimethyl-1'-[(3-methyl-1H-indazot-6-yl)carbonyl]spiro[chromene-2,4'-piperidin]-4(3H)-one

Ex. F2: 6-methoxy-1'-[(3-methyl-1H-indazol-6-yl)carbonyl]spiro[chromene-2,4'-piperidin]-4(3H)-one

Ex. F3: 6-chloro-7-methyl-1'-[(3-methyl-1H-indazol-6-yl)carbonyl]spiro[chromene-2,4'-piperidin]-4(3H)-one

Ex. F4: methyl 1'-[(3-methyl-1H-indazof-6-yl)carbonyl]-4-oxo-3,4-dihydrospiro[chromene-2,4'-piperidine]-6-carboxylate

Ex. F5: Method C was used to form 1'-[(3-methyl-1H-indazol-6-yl)carbonyl]-4-oxo-3,4-dihydrospiro[chromene-2,4'-piperidine]-6-carboxylic acid trifluoroacetic acid salt. Method B was used to prepare methyl 1-[(3-methyl-1H-indazol-6-yl)carbonyl]-4-oxo-3,4-dihydrospiro[chromene-2,4 -piperidine]-6-carboxylate. To a solution of methyl 1-[(3-methyl-1H-indazol-6-yl)carbonyl]-4-oxo-3,4-dihydrospiro[chromene-2,4-piperidine]-6-carboxylate in MeOH/water (1.4 mL, 2:1), was added LiOH (7.5 mg). The solution was heated at 50 °C for 3hrs. The reaction mixture was then cooled down, concentrated, and purified by column chromatography (16.6 mg, 33%).

### Examples G1-G5

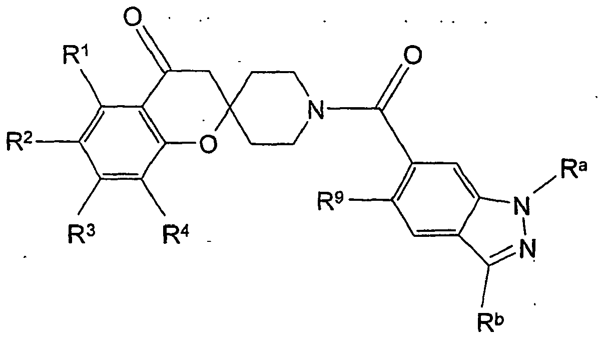

| Ex. | Method | R¹ | R² | R³ | R⁴ | R⁹ | R^{a} | R^{b} | ACC1 IC₅₀ (nM) | ACC1 n* | MS(ACPI) *m*/*z* (M+H)⁺ | HPLC RT (min) |
|---|---|---|---|---|---|---|---|---|---|---|---|---|
| G1 | A1 | H | CH₃ | CH₃ | H | H | H | H | 271 | 1 | 390 | 1.56 |
| G2 | A1 | H | CH₃ | CH₃ | H | H | CH₃ | H | 167 | 2 | 404 | 1.67 |
| G3 | A1 | H | CH₃ | CH₃ | H | H | CH₃ | CH₃ | 75.4 | 2 | 418 | 1.71 |
| G4 | A1 | H | CH₃ | CH₃ | H | H | CH₃ | CH₂CH₃ | 80.1 | 2 | 432 | 1.84 |
| G5 | A | H | Cl | H | H | Br | H | H | >1,000 | 1 | 475 | 1.77 |

| | | | | | | | | | | | | |
|---|---|---|---|---|---|---|---|---|---|---|---|---|
| ^{*} - n is the number of times the assay was performed. | | | | | | | | | | | | |

Ex. G1: 1'-(1H-indazol-6-ylcarbonyl)-6,7-dimethylspiro[chromene-2,4'-piperidin]-4(3H)-one trifluoroacetic acid salt

Ex. G2: 6,7-dimethyl-1'-[(1-methyl-1H-indazol-6-yl)carbonyl]spiro[chromene-2,4'-piperidin]-4(3H)-one

Ex. G3: 1'-[(1,3-dimethyl-1H-indazol-6-yl)carbonyl]-6,7-dimethylspiro[chromene-2,4'-piperidin]-4(3H)-one

Ex. G4: 1'-[(3-ethyl-1-methyl-1H-indazol-6-yl)carbonyl]-6,7-dimethylspiro[chromene-2,4'-piperidin]-4(3H)-one

Ex. G5: 1'-[(7-bromo-1H-indazol-6-yl)carbonyl]-6-chlorospiro[chromene-2,4'-piperidin]-4(3H)-one

### Examples H1-H24

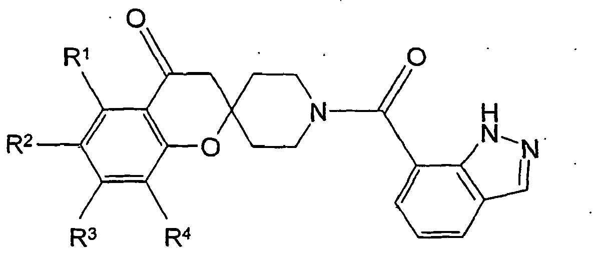

| Ex. | Method | R¹ | R² | R³ | R⁴ | ACC1 IC₅₀ (nM) | ACC1 n* | MS(ACPI) *m*/*z* (M+H)⁺ | HPLC RT (min) |
|---|---|---|---|---|---|---|---|---|---|
| H1 | D | H | CH₃ | CH₃ | H | 163 | 2 | 390 | 2.5 |
| H2 | B | H | CH₃ | Cl | H | 226 | 1 | 410 | 2.55 |
| H3 | B | H | Br | CH₃ | H | 264 | 1 | 454 | 2.65 |
| H4 | B | OCH₃ | H | CH₃ | H | 279 | 1 | 406 | 2.1 |
| H5 | B | H | Cl | CH₃ | H | 321 | 1 | 410 | 2.5 |
| H6 | B | F | OCH₃ | H | H | 381 | 1 | 410 | 2.15 |
| H7 | B | H | OCH₃ | H | H | 562 | 1 | 392 | 2.25 |
| H8 | B | CH₃ | Cl | CH₃ | H | 590 | 1 | 424 | 2.7 |
| H9 | B | H | CH₃ | H | H | 634 | 1 | 376 | 3.2 |
| H10 | B | H | i-propyl | H | H | 693 | 1 | 404 | 2.6 |
| H11 | B | OCH₃ | OCH₃ | OCH₃ | H | 756 | 1 | 452 | 2 |
| H12 | B | H | H | CH₃ | H | 850 | 2 | 376 | 2.3 |
| H13 | E | H | CH₃ | H | CH₃ | 1320 | 1 | 390 | 3.4 |
| H14 | E | H | Cl | H | H | 1390 | 1 | 396 | 3.4 |
| H15 | B | Cl | H | Cl | H | 1480 | 1 | 400 | 2.6 |
| H16 | E | OCH₃ | H | H | H | 1620 | 1 | 392 | 2.7 |
| H17 | B | OCH₃ | Cl | H | H | 1830 | 1 | 426 | 2.3 |
| H18 | B | H | H | phenyl | H | 2210 | 1 | 438 | 2.65 |
| H 19 | D | H | H | F | H | 2350 | 2 | 380 | 2.2 |
| H20 | D | OCH₃ | H | H | H | 2430 | 2 | 392 | 2.0 |
| H21 | B | H | H | OCH₃ | H | 2530 | 1 | 392 | 2.1 |
| H22 | B | H | Cl | H | Cl | 2660 | 1 | 430 | 2.6 |
| H23 | B | H | H | H | H | >3000 | 1 | 362 | 2.2 |
| H24 | B | H | H | H | H | 3540 | 1 | 362 | 2.2 |

| | | | | | | | | | |
|---|---|---|---|---|---|---|---|---|---|
| * - n is the number of times the assay was performed. | | | | | | | | | |

Ex. H1: Method D was used to form 1'-(1H-indazol-7-ylcarbonyl)-6,7-dimethylspiro[chromene-2,4'-piperidin]-4(3H)-one. Specifically, into a 1 dram vial was added 260 µL of 0.25 M solution of 6,7-dimethylspiro[chromene-2,4 -piperidin]-4(3H)-one dissolved in a 1 M triethylamine solution in CH₂Cl₂. Into this was added 260 µL of a 0.25 M solution of 1H-indazole-7-carboxylic acid in CH₂Cl₂. The mixture was vortexed and into this mixture was added 260 µL of HATU in CH₂Cl₂. The vial was vortexed and then shaken at room temperature for 16 hours. The crude reaction mixture was purified by liquid chromatography to provide the title product.

Ex. H2: 7-chloro-1'-(1H-indazol-7-ylcarbonyl)-6-methylspiro[chromene-2,4'-piperidin]-4(3H)-one

Ex. H3: 6-bromo-1'-(1H-indazol-7-ylcarbonyl)-7-methylspiro[chromene-2,4'-piperidin]-4(3H)-one

Ex. H4: 1'-(1H-indazol-7-ylcarbonyl)-5-methoxy-7-methylspiro[chromene-2,4'-piperidin]-4(3H)-one

Ex. H5: 6-chloro-1'-(1 H-indazol-7 -ylcarbonyl)-7 -methylspiro[chromene-2,4'-piperidin]-4(3H)-one

Ex. H6: 5-fluoro-1'-(1H-indazol-7-ylcarbonyl)-6-methoxyspiro[chromene-2,4'-piperidin]-4(3H)-one

Ex. H7: 1'-(1H-indazol-7-ylcarbonyl)-6-methoxyspiro[chromene-2,4'-piperidin]-4(3H)-one

Ex. H8: 6-chloro-1'-(1H-indazol-7-ylcarbonyl)-5,7-dimethylspiro[chromene-2,4'-piperidin]-4(3H)-one

Ex. H9: 1'-(1H-indazol-7 -ylcarbonyl)-6-methylspiro[chromene-2,4'-piperidin]-4(3H)-one

Ex. H10: 1'-(1H-indazol-7-ylcarbonyl)-6-isopropylspiro[chromene-2,4'-piperidin]-4(3H)-one

Ex. H11: 1'-(1H-indazol-7-ylcarbonyl)-5,6,7-trimethoxyspiro[chromene-2,4'-piperidin]-4(3H)-one

Ex. H12: 1'-(1H-indazol-7-ylcarbonyl)-7-methylspiro[chromene-2,4'-piperidin]-4(3H)-one

Ex. H13: Method E was used to form 1'-(1H-indazol-7-ylcarbonyl)-6,8-dimethylspiro[chromene-2,4'-piperidin]-4(3H)-one. Into a 2.2 mL well in a 96 deep-well plate was added a solution of 1 H-indazole-7-carboxylic acid (0.5 mL of 0.5 M DMF solution), a solution of 6,8-dimethylspiro[chromene-2,4 -piperidin]-4(3H)-one (0.5 mL of 0.5 M DMF solution), and a solution of HATU (0.5 mL of 0.5 M DMF solution). To this was added triethylamine (3 equivalents). The plate was sealed and agitated for 16 hours. The solvents were removed by centrifugal evaporation at reduced pressure. The residues were dissolved in CH₂Cl₂ (1 mL), and washed sequentially with K₂CO₃ (2 x 0.7 mL of 0.5 M solution) and water (0.7 mL) before being transferred to a collection plate. The final aqueous waste was re-extracted with CH₂Cl₂ (0.5 mL), combined with the first CH₂Cl₂ extract and evaporated to dryness. MS(ACPI) *m*/*z* 390.(M+H)⁺, HPLC RT 3.4 minutes.

Ex. H14: 6-chloro-1'-(1H-indazol-7-ylcarbonyl)spiro[chromene-2,4'-piperidin]-4(3H)-one

Ex. H15: 5,7-dichloro-1'-(1H-indazol-7-ylcarbonyl)spiro[chromene-2,4'-piperidin]-4(3H)-one

Ex. H16: 1'-(1H-indazol-7-ylcarbonyl)-5-methoxyspiro[chromene-2,4'-piperidin]-4(3H)-one

Ex. H17: 6-chloro-1'-(1H-indazol-7-ylcarbonyl)-5-methoxyspiro[chromene-2,4'-piperidin]-4(3H)-one

Ex. H18: 1'-(1H-indazol-7-ylcarbonyl)-7-phenylspiro[chromene-2,4'-piperidin]-4(3H)-one

Ex. H19: 7-fluoro-1'-(1H-indazol-7-ylcarbonyl)spiro[chromene-2,4'-piperidin]-4(3H)-one

Ex. H20: 1'-(1H-indazol-7-ylcarbonyl)-5-methoxyspiro[chromene-2,4'-piperidin]-4(3H)-one; ¹H NMR (CDCl₃) δ 8.11 (s, 1H), 7.85 (d, J=7.9, 1 H), 7.37-7.42 (m, 2H), 7.14-7.18 (m, 1 H), 6.61 (d, J=8.3, 1 H), 6.52 (d, J=8.3, 1H), 3.90 (s, 3H), 2.72 (s, 2H)

Ex. H21:1'-(1 H-indazol-7-ylcarbonyl)-7-methoxyspiro[chromene-2,4'-piperidin]-4(3H)-one

Ex. H22: 6,8-dichloro-1'-(1H-indazol-7-ylcarbonyl)spiro[chromene-2,4'-piperidin]-4(3H)-one

Ex. H23: 1'-[(1H-indazol-7-yl)carbonyl]-6-chloro-7-methylspiro[chromene-2,4'-piperidin]-4(3H)-one

Ex. H24: 1'-[(1H-indazol-7-yl)carbonyl]-6-chloro-7-methylspiro[chromene-2,4'-piperidin]-4(3H)-one

### Examples J1-J3

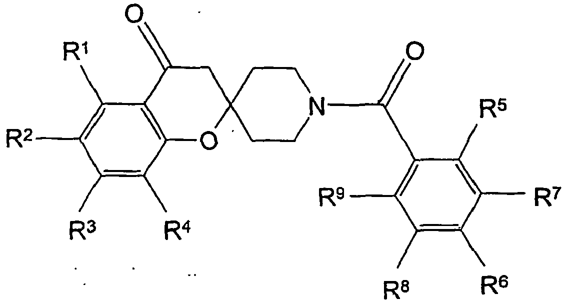

| | | | | | | | | |
|---|---|---|---|---|---|---|---|---|
| Ex. | 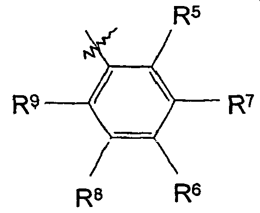 | Method | R¹ | R² | R³ | R⁴ | ACC1 IC₅₀ (nM) | ACC1 n* |
| J1 | 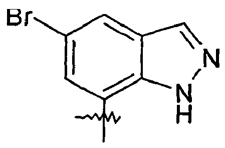 | B | H | Cl | CH₃ | H | 345 | 1 |
| J2 | 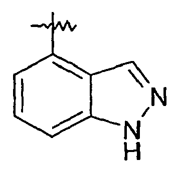 | B | H | Cl | CH₃ | H | 375 | 1 |
| J3 | 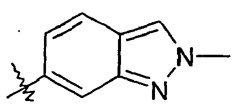 | B | H | CH₃ | CH₃ | H | 353 | 1 |

| | | | | | | | | |
|---|---|---|---|---|---|---|---|---|
| ^{*} - n is the number of times the assay was performed. | | | | | | | | |

Ex. J1: 1'-[(5-bromo-1H-indazol-7-yl)carbonyl]-6-chloro-7-methylspiro[chromene-2,4'-piperidin]-4(3H)-one, MS(ACPI) *m*/*z* 488 (M+H)⁺, HPLC RT 2.8 minutes

Ex. J2: 6-chloro-1'-(1H-indazol-4-ylcarbonyl)-7-methylspiro[chromene-2,4'-piperidin]-4(3H)-one, MS(ACPI) *m*/*z* 410 (M+H)⁺, ¹H NMR (CDCl₃) δ 8.14 (s, 1H), 7.83 (s, 1H), 7.59 (d, J=8.3, 1H), 7.44-7.47 (m, 1H), 7.23 (d, J=6.8, 1H), 6.95 (s, 1H), 2.75 (s, 2H), 2.40 (s, 3H)

Ex. J3: 6,7-dimethyl-1'-[(2-methyl-2H-indazol-6-yl)carbonyl]spiro[chromene-2,4'-piperidin]-4(3H)-one, MS(ACPI) *m*/*z* 404 (M+H)⁺, HPLC RT 2.4 minutes

### Examples K1-K3

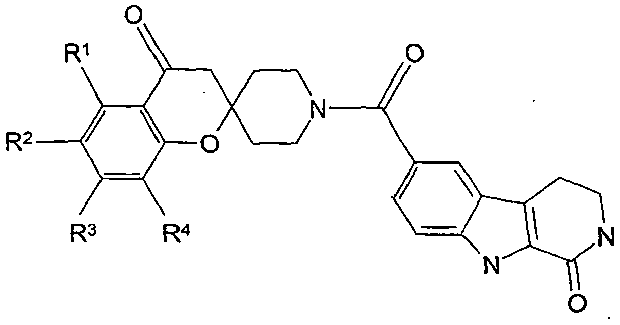

| Ex. | Method | R1 | R2 | R3 | R4 | ACC1 IC₅₀ (nM) | ACC1 n* | MS(ACPI) *m*/*z* (M+H)⁺ | HPLC RT (min) |
|---|---|---|---|---|---|---|---|---|---|
| K1 | A | H | CH₃ | CH₃ | H | 9.98 | 1 | 458 | 1.62 |
| K2 | A | OCH₃ | H | H | H | 28.8 | 1 | 460 | 1.28 |
| K3 | A | H | Cl | H | H | 106 | 1 | 464 | 1.66 |

| | | | | | | | | | |
|---|---|---|---|---|---|---|---|---|---|
| * - n is the number of times the assay was performed. | | | | | | | | | |

Ex. K1: 6,7-dimethyl-1'-[(1-oxo-2,3,4,9-tetrahydro-1H-beta-carbolin-6-yl)carbonyl]spiro[chromene-2,4'-piperidin]-4(3H)-one

Ex. K2: 5-methoxy-1'-[(1-oxo-2,3,4,9-tetrahydro-1H-beta-carbolin-6-yl)carbonyl]spiro[chromene-2,4'-piperidin]-4(3H)-one

Ex. K3: 6-chloro-1'-[(1-oxo-2,3,4,9-tetrahydro-1H-beta-carbolin-6-yl)carbonyl]spiro[chromene-2,4'-piperidin]-4(3H)-one

### Examples L1-L48

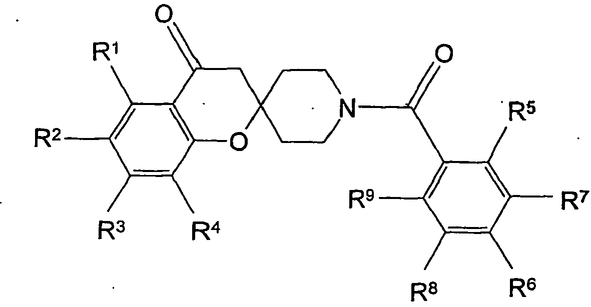

| | | | | | | | | |
|---|---|---|---|---|---|---|---|---|
| Ex. | 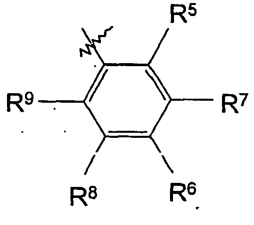 | Method | R¹ | R² | R³ | R⁴ | ACC1 IC₅₀ (nM) | ACC1 n* |
| L1 | 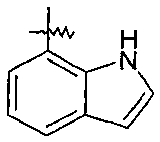 | A1 | H | CH₃ | CH₃ | H | 190 | 1 |
| L2 | 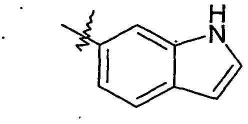 | A1 | H | CH₃ | CH₃ | H | 155 | 2 |
| L3 | 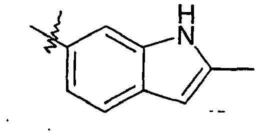 | A1 | H | CH₃ | CH₃ | H | 368 | 2 |
| L4 | 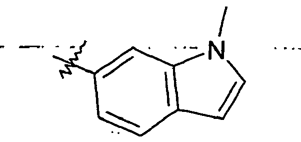 | A | H | CH₃ | CH₃ | H | 83.3 | 1 |
| L5 | 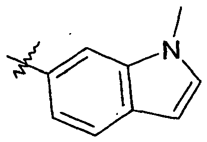 | A | H | Cl | H | H | <1,000 | 1 |
| L6 | 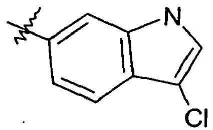 | A | OCH₃ | H | H | H | 47.3 | 1 |
| L7 | 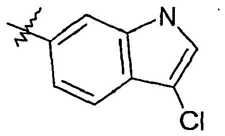 | A | H | Cl | H | H | 183 | 1 |
| L8 | 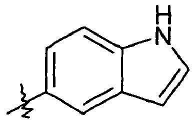 | B | H | Cl | CH₃ | H | 456 | 1 |
| L9 | 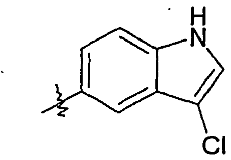 | A | H | CH₃ | CH₃ | H | 43.8 | 1 |
| L10 | 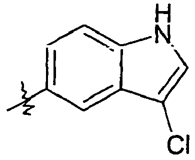 | A | OCH₃ | H | H | H | 101 | 1 |
| L11 | 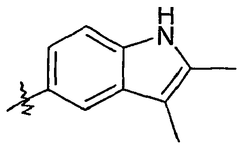 | A | OCH₃ | H | H | H | 113 | 1 |
| L12 | 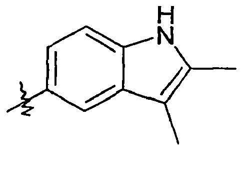 | A | H | Cl | H | H | <1,000 | 1 |
| L13 | 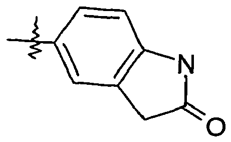 | A | H | CH₃ | CH₃ | H | <3,000 | 1 |
| L14 | 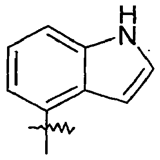 | A1 | H | CH₃ | CH₃ | H | 479 | 2 |
| L15 | 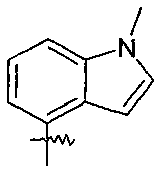 | A1 | H | CH₃ | CH₃ | H | 415 | 2 |
| L16 | 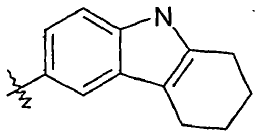 | A | OCH₃ | H | H | H | 65.0 | 1 |
| L17 | 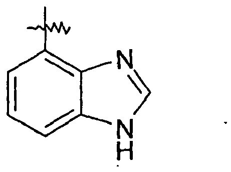 | A1 | H | CH₃ | CH₃ | H | 885 | 1 |
| L18 | 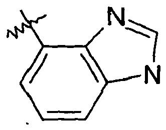 | B | H | Cl | CH₃ | H | 2010 | 1 |
| L19 | 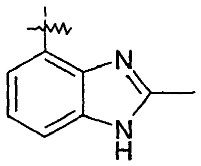 | A1 | H | CH₃ | CH₃ | H | <3000 | 1 |
| L20 | 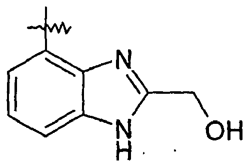 | A1 | H | CH₃ | CH₃ | H | 919 | 2 |
| L21 | 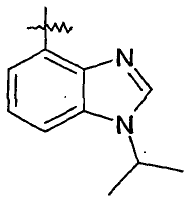 | A1 | H | CH₃ | CH₃ | H | 670 | 2 |
| L22 | 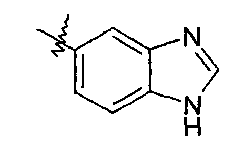 | A1 | H | CH₃ | CH₃ | H | 164 | 2 |
| L23 | 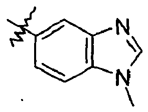 | A1 | H | CH₃ | CH₃ | H | 1170 | 2 |
| L24 | 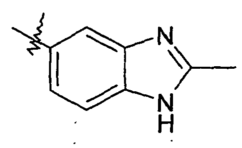 | A1 | H | CH₃ | CH₃ | H | 825 | 2 |
| L25 | 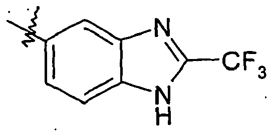 | A1 | H | CH₃ | CH₃ | H | 613 | 2 |
| L26 | 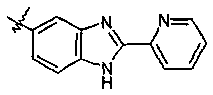 | A1 | H | CH₃ | CH₃ | H | 63.5 | 1 |
| L27 | 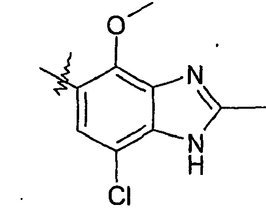 | A | H | Cl | H | H | >1,000 | 1 |
| L28 | 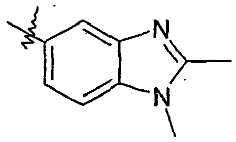 | A | H | CH₃ | CH₃ | H | 3150 | 1 |
| L29 | 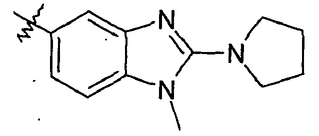 | A | H | CH₃ | CH₃ | H | 130 | 1 |
| L30 | 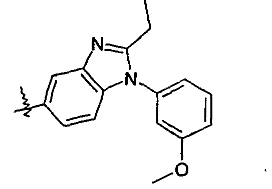 | B | H | H | H | H | 390 | 2 |
| L31 | 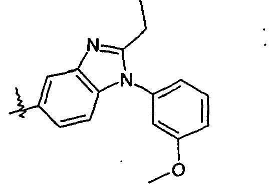 | B | OCH₃ | H | H | H | 1140 | 1 |
| L32 | 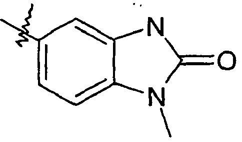 | A1 | H | CH₃ | CH₃ | H | 190 | 1 |
| L33 | 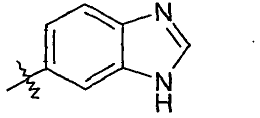 | A | H | CH₃ | CH₃ | H | 99.9 | 1 |
| L34 | 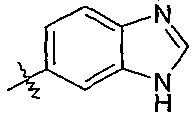 | A | H | Cl | H | H | >1,000 | 1 |
| L35 | 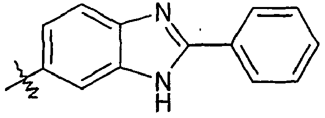 | A1 | OCH₃ | H | H | H | 15.5 | 1 |
| L36 | 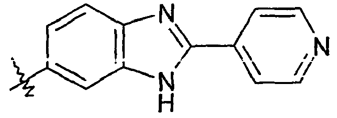 | A | H | CH₃ | CH₃ | H | 50.7 | 1 |
| L37 | 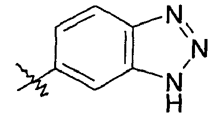 | A | H | CH₃ | CH₃ | H | 151 | 1 |
| L38 | 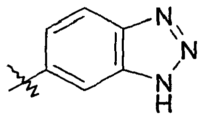 | A | H | Cl | H | H | <1,000 | 1 |
| L39 | 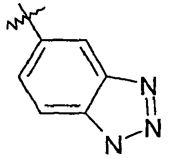 | A1 | H | CH₃ | CH₃ | H | 362 | 1 |
| L40 | 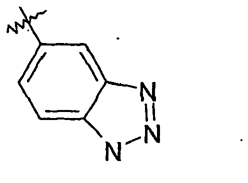 | B | H | Cl | CH₃ | H | 4860 | 1 |
| L41 | 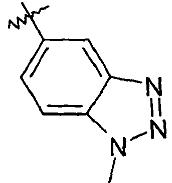 | A1 | H | CH₃ | CH₃ | H | <3000 | 1 |
| L42 | 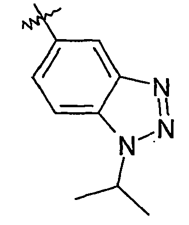 | A | H | Cl | H | H | >1,000 | 1 |
| L43 | 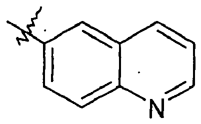 | A1 | H | CH₃ | CH₃ | H | 121 | 1 |
| L44 | 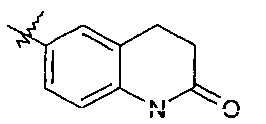 | A | H | CH₃ | CH₃ | H | <3000 | 1 |
| L45 | 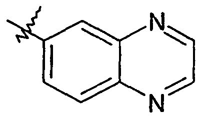 | A | H | CH₃ | CH₃ | H | <3000 | 1 |
| L46 | 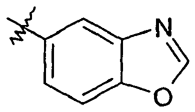 | B | H | Cl | CH₃ | H | 369 | 1 |

| | | | | | | | | |
|---|---|---|---|---|---|---|---|---|
| * - n is the number of times the assay was performed | | | | | | | | |

Ex. L1:1'-(1H-indol-7-ylcarbonyl)-6,7-dimethylspiro[chromene-2,4'-piperidin]-4(3H)-one-trifluoroacetic acid salt, MS(ACPI) *m*/*z* 389 (M+H)⁺, HPLC RT 1.9 minutes

Ex. L2: 1'-(1H-indol-6-ylcarbonyl)-6,7-dimethylspiro[chromene-2,4'-piperidin]-4(3H)-one trifluoroacetic acid salt, MS(ACPI) *m*/*z* 389 (M+H)⁺, HPLC RT 1.80 minutes

Ex. L3: 6,7-dimethyl-1'-[(2-methyl-1H-indol-6-yl)carbonyl]spiro[chromene-2,4'-piperidin]-4(3H)-one trifluoroacetic acid salt, MS(ACPI) *m*/*z* 403 (M+H)⁺, HPLC RT 1.89 minutes

Ex. L4: 6,7-dimethyl-1'-[(1-methyl-1H-indol-6-yl)carbonyl]spiro[chromene-2,4'-piperidin]-4(3H)-one, MS(ACPI) *m*/*z* 403 (M+H)⁺, HPLC RT 1.88

Ex. L5: 6-chloro-1'-[(1-methyl-1 H-indol-6-yl)carbonyl]spiro[chromene-2,4'-piperidin]-4(3H)-one, MS(ACPI) *m*/*z* 409 (M+H)⁺, HPLC RT 2.08.

Ex. L6: 1'-[(3-chloro-1H-indol-6-yl)carbonyl]-5-methoxyspiro[chromene-2,4'-piperidin]-4(3H)-one, MS(ACPI) *m*/*z* 425 (M+H)⁺, HPLC RT 1.63

Ex. L7: 6-chloro-1'-[(3-chloro-1H-indol-6-yl)carbonyl]spiro[chromene-2,4'-piperidin]-4(3H)-one, MS(ACPI) *m*/*z* 429 (M+H)⁺, HPLC RT 1.9

Ex. L8: 6-chloro-1'-(1H-indol-5-ylcarbonyl)-7-methylspiro[chromene-2,4'-piperidin]-4(3H)-one, MS(ACPI) *m*/*z* 456 (M+H)⁺, HPLC Retention Time 2.8 minutes, ¹H NMR (DMSO-d₆) δ 11.29 (s, 1H), 7.63-7.64 (m, 2H), 7.41-7.43 (m, 2H), 7.17 (s, 1H), 7.14-7.15 (m, 2H), 6.48 (s, 1H), 2.87 (s, 2H), 2.35 (s, 3H).

Ex. L9: 1'-[(3-chloro-1H-indol-5-yl)carbonyl]-6,7-dimethylspiro[chromene-2,4'-piperidin]-4(3H)-one, MS(ACPI) *m*/*z* 423 (M+H)⁺, HPLC RT 1.85

Ex. L10: 1'-[(3-chloro-1H-indol-5-yl)carbonyl]-5-methoxyspiro[chromene-2,4'-piperidin]-4(3H)-one, MS(ACPI) *m*/*z* 425 (M+H)⁺, HPLC RT 1.54

Ex. L11:1' -[(2,3-dimethyl-1H-indol-5-yl)carbonyl]-5-methoxyspiro[chromene-2,4'-piperidin]-4(3H)-one, MS(ACPI) *m*/*z* 419 (M+H)⁺, HPLC RT 1.68

Ex. L12: 6-chloro-1'-[(2,3-dimethyl-1H-indol-5-yl)carbonyl]spiro[chromene-2,4'-piperidin]-4(3H)-one, MS(ACPI) *m*/*z* 423 (M+H)⁺, HPLC RT 1.88

Ex. L13: 6,7-dimethyl-1'-[(2-oxo-2,3-dihydro-1H-indol-5-yl)carbonyl]spiro[chromene-2,4'-piperidin]-4(3H)-one, MS(ACPI) *m*/*z* 405 (M+H)⁺, HPLC RT 1.45

Ex. L14: 1'-(1 H-indol-4-ylcarbonyl)-6,7-dimethylspiro[chromene-2,4'-piperidin]-4(3H)-one trifluoroacetic acid salt, MS(ACPI) *m*/*z* 389 (M+H)⁺, HPLC RT 1.7771 minutes

Ex. L15: 6,7-dimethyl-1'-[(1-methyl-1H-indol-4-yl)carbonyl]spiro[chromene-2,4'-piperidin]-4(3H)-one, MS(ACPI) *m*/*z* 403 (M+H)⁺, HPLC RT 1.88 minutes

Ex. L16: 5-methoxy-1'-(2,3,4,9-tetrahydro-1 H-carbazol-6-ylcarbonyl)spiro[chromene-2,4'-piperidin]-4(3H)-one, MS(ACPI) *m*/*z* 445 (M+H)⁺, HPLC RT 1.7

Ex. L17: 1'-(1H-benzimidazol-4-ylcarbonyl)-6,7-dimethylspiro[chromene-2,4'-piperidin]-4(3H)-one trifluoroacetic acid salt, MS(ACPI) *m*/*z* 390 (M+H)⁺, HPLC RT 1.24 minutes

Ex. L18: 1'-(1H-benzimidazol-4-ylcarbonyl)-6-chloro-7-methylspiro[chromene-2,4'-piperidin]-4(3H)-one, MS(ACPI) *m*/*z* 410 (M+H)⁺, HPLC RT 2.2 minutes, ¹H NMR (DMSO-d₆) δ 8.61 (s, 1H), 7.72 (d, J=8.3, 1H), 7.64 (s, 1H), 7.34 (t, J=7.8, 1H), 7.29 (d, J=7.3, 1 H), 7.16 (s, 1 H), 2.86 (s, 2H), 2.35 (s, 3H).

Ex. L19:6,7-dimethyl-1'-[(2-methyl-1H-benzimidazol-4-yl)carbonyl]spiro[chromene-2,4'-piperidin]-4(3H)-one trifluoroacetic acid salt , MS(ACPI) *m*/*z* 404 (M+H)⁺, HPLC RT 1.28 minutes

Ex. L20: 1'-{[2-(hydroxymethyl)-1H-benzimidazol-6-yl]carbonyl}-6,7-dimethylspiro[chromene-2,4'-piperidin]-4(3H)-one trifluoroacetic acid salt, MS(ACPI) *m*/*z* 420 (M+H)⁺, HPLC RT 1.17 minutes

Ex. L21: 1'-[(1-isopropyl-1H-benzimidazol-4-yl)carbonyl]-6,7-dimethylspiro[chromene-2,4'-piperidin]-4(3H)-one trifluoroacetic acid salt, MS(ACPI) *m*/*z* 432 (M+H)⁺, HPLC RT 1.38minutes

Ex. L22: 1'-(1 H-benzimidazol-5-ylcarbonyl)-6,7-dimethylspiro[chromene-2,4'-piperidin]-4(3H)-one trifluoroacetic acid salt, MS(ACPI) *m*/*z* 390 (M+H)⁺, HPLC RT 1.17 minutes

Ex. L23: 6,7-dimethyl-1'-[(1-methyl-1H-benzimidazol-5-yl)carbonyl]spiro[chromene-2,4'-piperidin]-4(3H)-one trifluoroacetic acid salt , MS(ACPI) *m*/*z* 404 (M+H)⁺, HPLC RT 1.21 minutes

Ex. L24: 6,7-dimethyl-1'-[(2-methyl-1H-benzimidazol-5-yl)carbonyl]spiro[chromene-2,4'-piperidin]-4(3H)-one trifluoroacetic acid salt, MS(ACPI) *m*/*z* 404 (M+H)⁺, H PLC RT 1.18 minutes

Ex. L25: 6,7-dimethyl-1'-{[2-(trifluoromethyl)-1 H-benzimidazol-5-yl]carbonyl}spiro[chromene-2,4'-piperidin]-4(3H)-one trifluoroacetic acid salt, MS(ACPI) *m*/*z* 458 (M+H)⁺, HPLC RT 1.68.minutes

Ex. L26: 6,7-dimethyl-1'-[(2-pyridin-2-yl-1H-benzimidazol-5-yl)carbonyl]spiro[chromene-2,4'-piperidin]-4(3H)-one trifluoroacetic acid salt, MS(ACPI) *m*/*z* 467 (M+H)⁺, HPLC RT 1.47 minutes

Ex. L27:1'-[(4-chloro-7-methoxy-2-methyl-1H-benzimidazol-6-yl)carbonyl]-6,7-dimethylspiro[chromene-2,4'-piperidin]-4(3H)-one, MS(ACPI) *m*/*z* 474 (M+H)⁺, HPLC RT 1.45 minutes.

Ex. L28:1'-[(1,2-dimethyl-1H-benzimidazol-5-yl)carbonyl]-6,7-dimethylspiro[chromene-2,4'-piperidin]-4(3H)-one, MS(ACPI) *m*/*z* 344, HPLC RT 1.21 minutes

Ex. L29: 6,7-dimethyl-1'-[(1-methyl-2-pyrrolidin-1-yl-1H-benzimidazol-5-yl)carbonyl]spiro[chromene-2,4'-piperidin]-4(3H)-one, MS(ACPI) *m*/*z* 473 (M+H)⁺, HPLC RT 1.33 minutes

Ex. L30: 1'-{[2-ethyl-1-(3-methoxyphenyl)-1H-benzimidazol-5-yl]carbonyl}spiro[chromene-2,4'-piperidin]-4(3H)-one, MS(ACPI) *m*/*z* 496 (M+H)⁺, HPLC RT 2.4 minutes

Ex. L31: 1'-{[2-ethyl-1-(3-methoxyphenyl)-1H-benzimidazol-5-yl]carbonyl}-5-methoxyspiro[chromene-2,4'-piperidin]-4(3H)-one, MS(ACPI) *m*/*z* 526 (M+H)⁺, HPLC RT 2.1 minutes

Ex. L32:6,7-dimethyl-1'-[(1-methyl-2-oxo-2,3-dihydro-1H-benzimidazol-5-yl)carbonyl]spiro[chromene-2,4'-piperidin]-4(3H)-one, MS(ACPI) *m*/*z* 420 (M+H)⁺, HPLC RT 1.46 minutes

Ex. L33: 1'-(1H-benzimidazol-6-ylcarbonyl)-6,7-dimethylspiro[chromene-2,4'-piperidin]-4(3H)-one, MS(ACPI) *m*/*z* 390 (M+H)⁺, HPLC RT 1.16

Ex. L34:1'-(1H-benzimidazol-5-ylcarbonyl)-6-chlorospiro[chromene-2,4'-piperidin]-4(3H)-one, MS(ACPI) *m*/*z* 396 (M+H)⁺, HPLC RT 1.13

Ex. L35: 5-methoxy-1'-[(2-phenyl-1 H-benzimidazol-6-yl)-carbonyl]spiro[chromene-2,4'-piperidin]-4(3H)-one, MS(ACPI) *m*/*z* 468 (M+H)⁺, HPLC RT 1.12

Ex. L36: 6,7-dimethyl-1'-[(2-pyridin-4-yl-1H-benzimidazol-6-yl)carbonyl]spiro[chromene-2,4'-piperidin]-4(3H)-one trifluoroacetic acid salt, MS(ACPI) *m*/*z* 467 (M+H)⁺, HPLC RT 1.30 minutes

Ex. L37: 1'-(1H-1,2,3-benzotriazol-5-ylcarbonyl)-6,7-dimethylspiro[chromene-2,4'-piperidin]-4(3H)-one, MS(ACPI) *m*/*z* 391 (M+H)⁺, HPLC RT 1.57 minutes

Ex. L38:1'-(1H-1,2,3-benzotriazol-5-ylcarbonyl)-6-chlorospiro[chromene-2,4'-piperidin]-4(3H)-one, MS(ACPI) *m*/*z* 397 (M+H)⁺, HPLC RT 1.47 minutes

Ex. L39: 1'-(1H-1,2,3-benzotriazol-5-ylcarbonyl)-6,7-dimethylspiro[chromene-2,4'-piperidin]-4(3H)-one trifluoroacetic acid salt, MS(ACPI) *m*/*z* 391 (M+H)⁺, HPLC RT 1.46 minutes

Ex. L40: 1'-(1H-1,2,3-benzotriazol-5-ylcarbonyl)-6-chloro-7 -methylspiro[chromene-2,4'-piperidin]-4(3H)-one, 1H NMR (DMSO-d₆) δ 8.11 (s, 1 H), 7.83 (s, 1H), 7.64 (s, 1H), 7.16 (s, 1H), 2.87 (s, 2H), 2.35 (s, 3H); MS(ACPI) *m*/*z* 411 (M+H)⁺, HPLC RT 2.4 minutes

Ex. L41: 6,7-dimethyl-1'-[(1-methyl-1H-1,2,3-benzotriazol-5-yl)carbonyl]spiro[chromene-2,4'-piperidin]-4(3H)-one, MS(ACPI) *m*/*z* 405 (M+H)⁺, HPLC RT 1.53 minutes

Ex. L42: 6-chloro-1'-[(1-isopropyl-1H-1,2,3-benzotriazol-5-yl)carbonyl]spiro[chromene-2,4'-piperidin]-4(3H)-one, MS(ACPI) *m*/*z* 439 (M+H)⁺, HPLC RT 1.72 minutes

Ex. L43: 6,7-dimethyl-1'-(quinolin-6-ylcarbonyl)spiro[chromene-2,4'-piperidin]-4(3H)-one trifluoroacetic acid salt, MS(ACPI) *m*/*z* 401 (M+H)⁺, HPLC RT 1.38 minutes

Ex. L44:6,7-dimethyl-1'-[(2-oxo-1,2,3,4-tetrahydroquinolin-6-yl)carbonyl]spiro[chromene-2,4'-piperidin]-4(3H)-one, MS(ACPI) *m*/*z* 419 (M+H)⁺, HPLC 1.49 minutes

Ex. L45:6,7-dimethyl-1'-(quinoxalin-6-ylcarbonyl)spiro[chromene-2,4'-piperidin]-4(3H)-one, MS(ACPI) *m*/*z* 402 (M+H)⁺, HPLC 1.53 minutes

Ex. L46: 1'-(1,3-benzoxazol-5-ylcarbonyl)-6-chloro-7-methylspiro[chromene-2,4'-piperidin]-4(3H)-one; MS(ACPI) m/z (M+H)+ 411. ¹H NMR (CDCl₃) δ 8.74 (s, 1H), 8.26 (s, 1H), 7.84 (s, 1H), 7.70 (s. 1H), 6.99-7.01 (m, 1H), 6.94 (s, 1H), 6.79-6.81 (d, 1H), 2.74 (s, 2H), 2.41 (s, 3H)

### Examples M1-M36

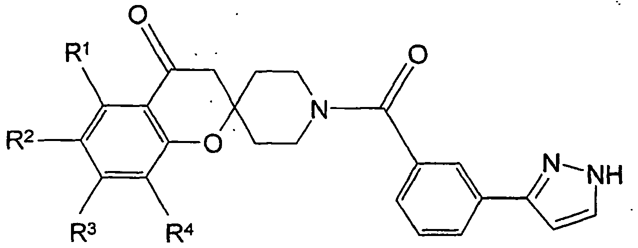

| Ex. | Method | R¹ | R² | R³ | R⁴ | ACC1 IC₅₀ (nM) | ACC1 n* | ACC2 IC₅₀ (nM) | ACC2 n* |
|---|---|---|---|---|---|---|---|---|---|
| M1 | B | OCH₃ | H | CH₃ | H | 37.5 | 2 | | |
| M2 | B | H | CH₃ | CH₃ | H | 73.6 | 1 | | |
| M3 | B | H | Cl | CH₃ | H | 105 | 1 | 434 | 1 |
| M4 | B | H | -C(O)NHCH₃ | H | H | 110 | 1 | | |
| M5 | B | H | -C(O)NH₂ | H | H | 118 | 1 | | |
| M6 | B | H | OCH₃ | H | H | 129 | 1 | | |
| M7 | B | H | CH₃ | Cl | H | 140 | 1 | | |
| M8 | B | H | Cl | Cl | H | 178 | 1 | | |
| M9 | B | H | pyrrolidin-1yl-C(O)- | H | H | 179 | 1 | | |
| M10 | B | H | Br | CH₃ | H | 201 | 1 | | |
| M11 | B | H | C(O)OCH₃ | H | H | 202 | 1 | | |
| M12 | B | H | H | CH₃ | H | 205 | 1 | | |
| M13 | B | H | OCF₃ | H | H | 220 | 1 | | |
| M14 | B | F | OCH₃ | H | H | 223 | 1 | | |
| M15 | B | H | H | Cl | H | 233 | 1 | | |
| M16 | B | Cl | H | Cl | H | 319 | 1 | | |
| M17 | B | H | C(O)NHCH(CH₃)₂ | H | H | 350 | 1 | | |
| M18 | B | OCH₃ | Cl | H | H | 374 | 1 | | |
| M19 | B | H | CF₃ | H | H | 418 | 1 | | |
| M20 | B | H | -S(O)₂CH₃ | H | H | 434 | 1 | | |
| M21 | B | OCH₃ | OCH₃ | OCH₃ | H | 439 | 1 | | |
| M22 | B | CH₃ | Cl | CH₃ | H | 452 | 1 | | |
| M23 | B | H | CH₃ | H | H | 475 | 2 | | |
| M24 | B | H | Cl | F | H | 478 | 1 | | |
| M25 | B | H | H | OCH₃ | H | 481 | 1 | | |
| M26 | B | H | i-propyl | H | H | 516 | 1 | | |
| M27 | B | OCH₃ | H | H | H | 545 | 4 | | |
| M28 | B | H | H | -CN | H | 556 | 1 | | |
| M29 | B | H | -C(O)N(CH₃)₂ | H | H | 574 | 1 | | |
| M30 | B | H | Cl | H | H | 613 | 1 | | |
| M31 | B | H | OCH₃ | OCH₃ | H | 697 | 1 | | |
| M32 | B | H | morpholin-4yl-C(O)- | H | H | 737 | 1 | | |
| M33 | B | H | -CN | H | H | 816 | 2 | 1860 | 1 |
| M34 | B | H | H | F | H | 817 | 4 | | |
| M35 | B | H | Cl | H | Cl | 1400 | 1 | | |
| M36 | B | H | F | Cl | H | 1990 | 1 | | |

| | | | | | | | | | |
|---|---|---|---|---|---|---|---|---|---|
| ^{*} - n is the number of times the assay was performed. | | | | | | | | | |

Ex. M1: 5-methoxy-7-methyl-1'-[3-(1H-pyrazol-3-yl)benzoyl]spiro[chromene-2,4'-piperidin]-4(3H)-one, MS(ACPI) *m*/*z* 432 (M+H)⁺, HPLC RT 2.1 minutes

Ex. M2: 6,7-dimethyl-1'-[3-(1H-pyrazol-3-yl)benzoyl]spiro[chromene-2,4'-piperidin]-4(3H)-one, MS(ACPI) *m*/*z* 416 (M+H)⁺, HPLC RT 2.5 minutes

Ex. M3: 6-chloro-7-methyl-1'-[3-(1H-pyrazol-3-yl)benzoyl]spiro[chromene-2,4'-piperidin]-4(3H)-one, MS(ACPI) *m*/*z* 436 (M+H)⁺, HPLC RT 2.58 minutes

Ex. M4: N-methyl-4-oxo-1'-[3-(1H-pyrazol-3-yl)benzoyl]-3,4-dihydrospiro[chromene-2.4'-piperidine]-6-carboxamide, MS(ACPI) *m*/*z* 445 (M+H)⁺, HPLC RT 1.7 minutes

Ex. M5: 4-oxo-1'-[3-(1H-pyrazol-3-yl)benzoyl]-3,4-dihydrospiro[chromene-2,4'-piperidine]-6-carboxamide, MS(ACPI) *m*/*z* 431 (M+H)⁺, HPLC RT 1.6 minutes

Ex. M6: 6-methoxy-1'-[3-(1H-pyrazol-3-yl)benzoyl]spiro[chromene-2,4'-piperidin]-4(3H)-one, MS(ACPI) *m*/*z* 418 (M+H)⁺, HPLC RT 2.2 minutes

Ex. M7: 7-chloro-6-methyl-1'-[3-(1H-pyrazol-3-yl)benzoyl]spiro[chromene-2,4'-piperidin]-4(3H)-one, MS(ACPI) *m*/*z* 436 (M+H)⁺, HPLC RT 2.59 minutes

Ex. M8: 6,7-dichloro-1'-[3-(1H-pyrazol-3-yl)benzoyl]spiro[chromene-2,4'-piperidin]-4(3H)-one, MS(ACPI) *m*/*z* 456 (M+H)⁺, HPLC RT 2.6 minutes, ¹H NMR (CDCl₃) δ 7.95 (s, 1H), 7.87 (s, 1H), 7.85 (s, 1 H), 7.69 (d, J=2.6, 1H), 7.49 (t, J=7.8, 1 H), 7.38 (d, J=7.8, 1H), 7.21 (s, 1 H), 6.68 (d, J=2.6, 1H), 2.77 (s, 2H)

Ex. M9: 1'-[3-(1H-pyrazol-3-yl)benzoyl]-6-(pyrrolidin-1-ylcarbonyl)spiro[chromene-2,4'-piperidin]-4(3H)-one, MS(ACPI) *m*/*z* 485 (M+H)⁺, HPLC RT 1.9 minutes

Ex. M10: 6-bromo-7-methyl-1'-[3-(1H-pyrazol-3-yl)benzoyl]spiro[chromene-2,4'-piperidin]-4(3H)-one, MS(ACPI) *m*/*z* 480 (M+H)⁺, HPLC RT 2.6 minutes

Ex. M11: methyl 4-oxo-1'-[3-(1H-pyrazol-3-yl)benzoyl]-3,4-dihydrospiro[chromene-2,4'-piperidine]-6-carboxylate, MS(ACPI) *m*/*z* 446 (M+H)⁺, HPLC RT 2.3 minutes

Ex. M12: 7-methyl-1'-[3-(1H-pyrazol-3-yl)benzoyl]spiro[chromene-2,4'-piperidin]-4(3H)-one, MS(ACPI) *m*/*z* 402 (M+H)⁺, HPLC RT 2.4 minutes

Ex. M13: 1'-[3-(1H-pyrazol-3-yl)benzoyl]-6-(trifluoromethoxy)spiro[chromene-2,4'-piperidin]-4(3H)-one, MS(ACPI) *m*/*z* 472 (M+H)⁺, HPLC RT 2.6 minutes, ¹H NMR (CDCl₃) δ 8.03 (s, 1H), 7.92 (s, 1H), 7.89 (d, J=7.8, 1H), 7.80 (m, 1H), 7.73-7.74 (m, 1 H), 7.49-7.55 (m, 1H), 7.43-7.44 (m, 1H), 7.38-7.39 (m, 1H), 7.07 (d, J=9.4, 1 H), 6.72 (br s, 1 H), 2.81 (s, 2H)

Ex. M14: 5-fluoro-6-methoxy-1'-[3-(1H-pyrazol-3-yl)benzoyl]spiro[chromene-2,4'-piperidin]-4(3H)-one, MS(ACPI) *m*/*z* 436.(M+H)⁺, HPLC RT 2.2 minutes

Ex. M15: 7-chloro-1'-[3-(1H-pyrazol-3-yl)benzoyl]spiro[chromene-2,4'-piperidin]-4(3H)-one, MS(ACPI) *m*/*z* 422 (M+H)⁺, HPLC RT 2.5 minutes, ¹H NMR (CDCl₃) δ 7.84-7.85 (m, 3H), 7.82 (d, J=8.8, 1H), 7.50 (t, J=7.8, 1H), 7.41 (d, J=7.8, 1H), 7.01-7.08 (m, 2H), 6.70 (s, 1H), 2.80 (s, 2H)

Ex. M16: 5,7-dichloro-1'-[3-(1H-pyrazol-3-yl)benzoyl]spiro[chromene-2,4'-piperidin]-4(3H)-one, MS(ACPI) *m*/*z* 456 (M+H)⁺, HPLC RT [2.6] minutes

Ex. M17: N-isopropyl-4-oxo-1'-[3-(1H-pyrazol-3-yl)benzoyl]-3,4-dihydrospiro[chromene-2,4'-piperidine]-6-carboxamide, MS(ACPI) *m*/*z* 473 (M+H)⁺, HPLC RT 2.0 minutes

Ex. M18: 6-chloro-5-methoxy-1'-[3-(1H-pyrazol-3-yl)benzoyl]spiro[chromene-2,4'-piperidin]-4(3H)-one, MS(ACPI) *m*/*z* 452.(M+H)⁺, HPLC RT 2.3 minutes

Ex. M19: 1'-[3-(1H-pyrazol-3-yl)benzoyl]-6-(trifluoromethyl)spiro[chromene-2,4'-piperidin]-4(3H)-one, MS(ACPI) *m*/*z* 456 (M+H)⁺, HPLC RT 2.6 minutes, ¹H NMR (CDCl₃) δ 8.18 (d, J=2.0, 1H), 7.85-7.87 (m, 2H), 7.75-7.77 (m, 1 H), 7.65-7.66 (m, 1H), 7.47-7.50 (m, 1 H), 7.37-7.39 (m, 1H), 7.15 (d, J=8.3, 1H), 6.66 (d, J=2.6, 1 H), 2.82 (s, 2H)

Ex: M20: 6-(methylsulfonyl)-1'-[3-(1H-pyrazol-3-yl)benzoyl]spiro[chromene-2,4'-piperidin]-4(3H)-one, MS(ACPI) *m*/*z* 456 (M+H)⁺, HPLC RT 1.7 minutes, ¹H NMR (CD₃OD) δ 8.37 (d, J=2.1, 1 H), 8.10 (dd, J=2.6, 8.8, 1H), 7.88 (br s, 2H), 7.73 (br s, 1H), 7.53 (br s, 1H), 7.34 (d, J=8.8, 1H), 6.75 (s, 1H), 5.51 (s, 1H), 3.32 (s, 3H), 3.13 (s, 2H)

Ex. M21: 5,6,7-trimethoxy-1'-[3-(1H-pyrazol-3-yl)benzoyl]spiro[chromene-2,4'-piperidin]-4(3H)-one, MS(ACPI) *m*/*z* 478 (M+H)⁺, HPLC RT 2.1 minutes

Ex M22: 6-chloro-5,7-dimethyl-1'-[3-(1H-pyrazol-3-yl)benzoyl]spiro[chromene-2,4'-piperidin]-4(3H)-one, MS(ACPI) *m*/*z* 450 (M+H)⁺, HPLC RT 2.85 minutes

Ex. M23: 6-methyl-1'-[3-(1H-pyrazol-3-yl)benzoyl]spiro[chromene-2,4'-piperidin]-4(3H)-one, MS(ACPI) *m*/*z* 402 (M+H)⁺, HPLC RT [2.4] minutes

Ex. M24: 6-chloro-7-fluoro-1'-[3-(1H-pyrazol-3-yl)benzoyl]spiro[chromene-2,4'-piperidin]-4(3H)-one, MS(ACPI) *m*/*z* 440 (M+H)⁺, HPLC RT 2.6 minutes, ¹H NMR (CDCl₃) δ 7.94-7.97 (m, 1H), 7.84-7.85 (m, 2H), 7.69 (d, J=2.1, 1 H), 7.50 (t, J=7.8, 1 H), 7.40 (d, J=7.2, 1 H), 6.84 (d, J=9.3, 1 H), 6.69 (d, J=2.1, 1 H), 2.77 (s, 2H)

Ex. M25: 7-methoxy-1'-[3-(1 H-pyrazol-3-yl)benzoyl]spiro[chromene-2,4'-piperidin]-4(3H)-one, MS(ACPI) *m*/*z* 418.(M+H)⁺, HPLC RT 2.25 minutes

Ex. M26: 5-methoxy-1'-[3-(1H-pyrazol-3-yl)benzoyl]spiro[chromene-2,4'-piperidin]-4(3H)-one, MS(ACPI) *m*/*z* 430 (M+H)⁺, HPLC RT 2.6 minutes

Ex. M27: 5-methoxy-1'-[3-(1H-pyrazol-3-yl)benzoyl]spiro[chromene-2,4'-piperidin]-4(3H)-one, MS(ACPI) *m*/*z* 418 (M+H)⁺, HPLC RT 2.0 minutes

Ex. M28: 1'-(1H-Indazol-7-ylcarbonyl)-6-methoxyspiro[chromene-2,4'-piperidin]-4(3H)-one, MS(ACPI) *m*/*z* 413 (M+H)⁺, HPLC RT 2.3 minutes, ¹H NMR (CDCl₃) δ 7.97 (d, J=7.8, 1H), 7.94 (m, 1 H), 7.89 (d, J=7.7, 1 H), 7.81 (d, J=2.6, 1 H), 7.53 (t, J=7.8; 1 H), 7.44 (d, J=7.8, 1 H), 7.38 (s, 1H), 7.30 (d, J=6.7, 1H), 6.72 (d, J=2.0, 1 H), 2.86 (s, 2H)

Ex. M29: N,N-dimethyl-4-oxo-1'-[3-(1H-pyrazol-3-yl)benzoyl]-3,4-dihydrospiro[chromene-2,4'-piperidine]-6-carboxamide, MS(ACPI) *m*/*z* 459 (M+H)⁺, HPLC RT 1.9 minutes

Ex. M30: 6-Chloro-1'-[3-(1H-pyrazol-3-yl)benzoyl]spiro[chromene-2,4'-piperidin]-4(3H)-one, MS(ACPI) *m*/*z* 422.(M+H)⁺, HPLC RT 2.4 minutes

Ex. M31: 6,7-Dimethoxy-1'-[3-(1H-pyrazol-3-yl)benzoyl]spiro[chromene-2,4'-piperidin]-4(3H)-one, MS(ACPI) *m*/*z* 448 (M+H)⁺, HPLC RT 2.3 minutes

Ex. M32: 6-(morpholin-4-ylcarbonyl)-1'-[3-(1H-pyrazol-3-yl)benzoyl]spiro[chromene-2,4'-piperidin]-4(3H)-one, MS(ACPI) *m*/*z* 501 (M+H)⁺, HPLC RT 1.9 minutes

Ex. M33: 4-oxo-1'-[3-(1H-pyrazol-3-yl)benzoyl]-3,4-dihydrospiro[chromene-2,4'-piperidine]-6-carbonitrile, MS(ACPI) *m*/*z* 413 (M+H)⁺, HPLC RT 2.2 minutes, ¹H NMR (CDCl₃) δ 8.21 (d, J=2.1, 1H), 7.90 (s, 1H), 7.87 (d. J=7.8, 1 H), 7.76 (dd, J=8.3, 2.0, 1H), 7.72 (d, J=2.6, 1H), 7.50 (t, J=7.7, 1H), 7.40 (d, J=7.3, 1 H), 7.15 (d, J=8.3, 1 H), 6.69 (d, J=2.0, 1 H), 2.84 (s, 2H)

Ex. M34: 7-Fluoro-1'-[3-(1H-pyrazol-3-yl)benzoyl]spiro[chromene-2,4'-piperidin]-4(3H)-one, MS(ACPI) *m*/*z* 406 (M+H)⁺, HPLC RT 2.25 minutes

Ex. M35: 6,8-dichloro-1'-[3-(1H-pyrazol-3-yl)benzoyl]spiro[chromene-2,4'-piperidin]-4(3H)-one, MS(ACPI) *m*/*z* 456.(M+H)⁺, HPLC RT 2.6 minutes

Ex. M36: 7-chloro-6-fluoro-1'-[3-(1H-pyrazol-3-yl)benzoyl]spiro[chromene-2,4'-piperidin]-4(3H)-one, MS(ACPI) *m*/*z* 440 (M+H)⁺, HPLC RT 2.5 minutes, 1 H NMR (CDCl3) δ 7.86-7.88 (m, 2H), 7.70 (d, J=2.1, 1 H), 7.62 (d, J=8.3, 1 H), 7.49 (t, J=7.8, 1 H), 7.39 (d, J=7.8, 1H), 7.14 (d, J=5.7, 1 H), 2.77 (s, 2H)

### Examples N1-N3

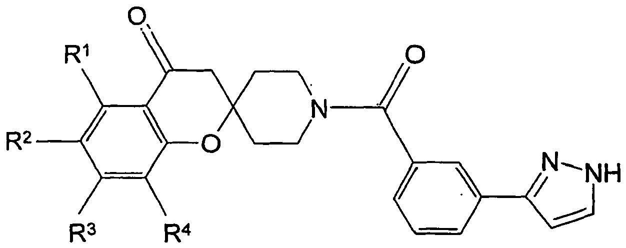

| Ex. | Method | R¹ | R² | R³ | R⁴ | ACC1 IC₅₀ (nM) | ACC1 n* | ACC2 IC₅₀ (nM) | ACC2 n* | MS(ACPI) *m*/*z* (M+H)⁺ | HPLC RT (min) |
|---|---|---|---|---|---|---|---|---|---|---|---|
| N1 | C | H | C(O)OH | H | H | 891 | 1 | | | 432 | 1.9 |
| N2 | B | H | OCH₂CH₃ | H | H | 43.2 | 1 | 91.7 | 1 | 432 | 2.5 |
| N3 | B | H | OCH(CH₃)₂ | H | H | 48.3 | 1 | 54.8 | 1 | 446 | 2.6 |

| | | | | | | | | | | | |
|---|---|---|---|---|---|---|---|---|---|---|---|
| * - n is the number of times the assay was performed. | | | | | | | | | | | |

Ex. N1: 4-oxo-1'-[3-(1H-pyrazol-3-yl)benzoyl]-3,4-dihydrospiro[chromene-2,4'-piperidine]-6-carboxylic acid trifluoroacetic acid salt

Ex. N2: 6-ethoxy-1'-[3-(1H-pyrazol-3-yl)benzoyl]spiro[chromene-2,4'-piperidin]-4(3H)-one, ¹H NMR (CDCl₃) δ 7.87 (s, 1H), 7.85 (s, 1H), 7.67-7.68 (d, 1 H), 7.46-7.49 (m, 1H), 7.37-7.39 (d, 1 H), 7.30 (m, 1 H), 7.12-7.14 (dd, 1 H), 6.94-6.96 (d, 1H), 6.66-6.67 (d, 1 H), 4.01-4.05 (q, 2H), 2.74-2.75 (m, 2H), 1.40-1.43 (t, 3H)

Ex. N3:: 6-isopropoxy-1'-[3-(1H-pyrazol-3-yl)benzoyl]spiro[chromene-2,4'-piperidin]-4(3H)-one, ¹H NMR (CDCl₃) δ 7.87 (s, 1H), 7.85 (s, 1H), 7.67 (m, 1 H), 7.46-7.49 (m, 1 H), 7.37-7.39 (m, 1H), 7.31-7.32 (d, 1 H), 7.09-7.11 (dd, 1 H), 6.93-6.95 (d, 1H), 6.66-6.67 (m, 1 H), 4.49-4.55 (q, 1 H), 2.74-2.75 (m, 2H), 1.32-1.33 (d, 6H)

### Examples O1-O4

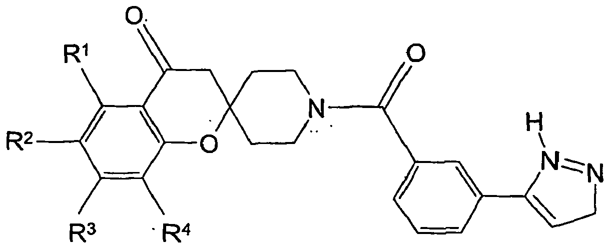

| Ex. | Method | R¹ | R² | R³ | R⁴ | ACC1 IC₅₀ (nM) | ACC1 n* | MS(ACPI) *m*/*z* (M+H)⁺ | HPLC RT (min) |
|---|---|---|---|---|---|---|---|---|---|
| O1 | B | H | H | H | H | 916 | 1 | 388 | 2.1 |
| O2 | B | CI | H | OCH₃ | H | 55.0 | 1 | 452 | |
| O3 | B | OCH₃ | H | Cl | H | 455 | 1 | 452 | |
| O4 | B | CH₃ | H | OCH₃ | H | 468 | 1 | 432 | 2.5 |

| | | | | | | | | | |
|---|---|---|---|---|---|---|---|---|---|
| * - n is the number of times the assay was performed. | | | | | | | | | |

Ex. O1: 1'-[3-(1 H-pyrazol-5-yl)benzoyl]spiro[chromene-2,4'-piperidin]-4(3H)-one

Ex. O2: 5-chloro-7-methoxy-1'-[3-(1H-pyrazol-5-yl)benzoyl]spiro[chromene-2,4'-piperidin]-4(3H)-one, ¹H NMR (CDCl₃) δ 7.87 (s, 1H), 7.84-7.85 (d, 1 H), 7.63-7.64 (d, 1H), 7.44-7.47 (m, 1 H), 7.34-7.36 (m, 1 H), 6.66-6.67 (d, 1 H), 6.63-6.64 (d, 1 H), 6.53 (m, 1H), 3.91 (s, 3H), 2.71-2.72 (m, 2H)

Ex. O3: 7-chloro-5-methoxy-1'-[3-(1H-pyrazol-5-yl)benzoyl]spiro[chromene-2,4'-piperidin]-4(3H)-one; ¹H NMR (CDCl₃) δ 7.88 (s, 1 H), 7.84-7.85 (d, 1 H), 7.63-7.64 (m, 1 H), 7.44-7.48 (m, 1H), 7.35-7.36 (m, 1H), 6.63 (m, 2H), 6.42-6.43 (m, 1 H), 3.85 (s, 3H), 2.72-2.73 (m, 1H)

Ex. O4: 7-methoxy-5-methyl-1'-[3-(1H-pyrazol-5-yl)benzoyl]spiro[chromene-2,4'-piperidin]-4(3H)-one, ¹H NMR (CDCl₃) δ 7.84-7.87 (m, 2H), 7.64-7.65 (d, 1H), 7.45-7.48 (m, 1H), 7.36-7.38 (d, 1H), 6.65 (d, 1H), 6.35-6.37 (d, 1H), 3.84 (s, 2H), 2.69-2.70 (m, 1H), 2.61 (s, 3H)

### Examples P1-P15

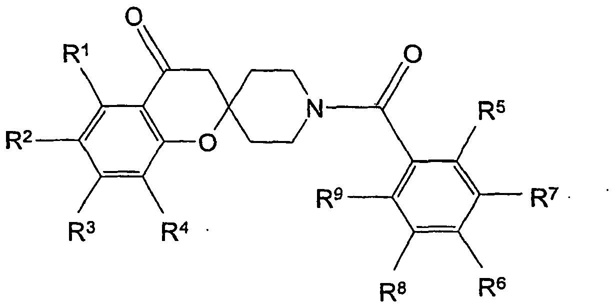

| Ex. | 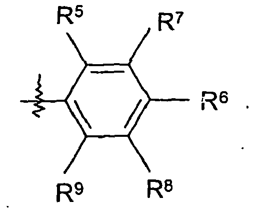 | Method | R¹ | R² | R³ | R⁴ | ACC1 IC₅₀ (nM) | ACC1 n* |
|---|---|---|---|---|---|---|---|---|
| P1 | 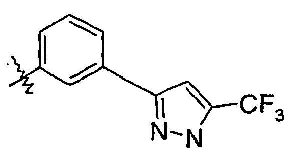 | F | H | CH₃ | CH₃ | H | 215 | 1 |
| P2 | 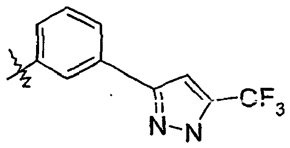 | B | H | Cl | CH₃ | H | 426 | 1 |
| P3 | 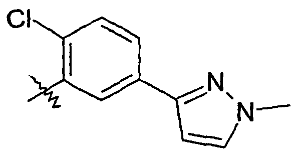 | A1 | H | CH₃ | CH₃ | H | <3000 | 1 |
| P4 | 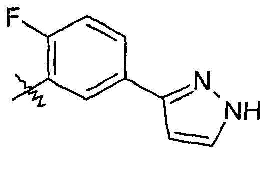 | A1 | H | CH₃ | CH₃ | H | <3000 | 1 |
| P5 | 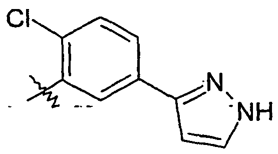 | A1 | H | CH₃ | CH₃ | H | <3000 | 1 |
| P6 | 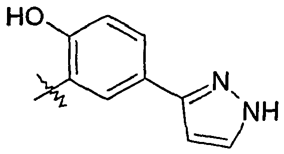 | A1 | H | CH₃ | CH₃ | H | <3000 | 1 |
| P7 | 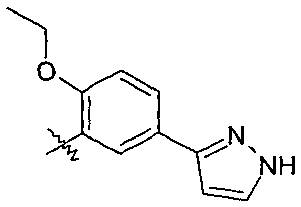 | B | H | CH₃ | CH₃ | H | <3000 | 1 |
| P8 | 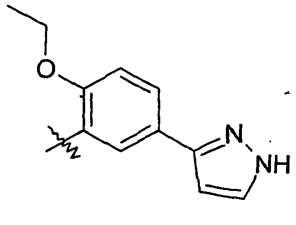 | B1 | H | CH₃ | CH₃ | H | 621 | 1 |
| P9 | 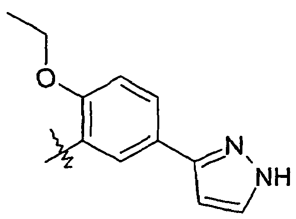 | B | H | Cl | CH₃ | H | 1060 | 1 |
| P10 | 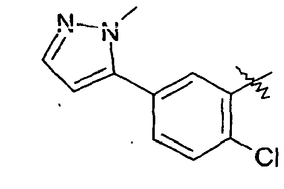 | A1 | H | CH₃ | CH₃ | H | <3000 | 1 |
| P11 | 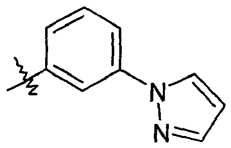 | A1 | H | CH₃ | CH₃ | H | 399 | 1 |
| P12 | 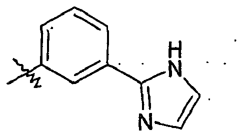 | A1 | H | CH₃ | CH₃ | H | 73.9 | 1 |
| P13 | 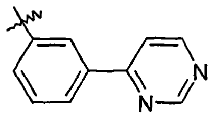 | A1 | H | CH₃ | CH₃ | H | <3000 | 1 |
| P14 | 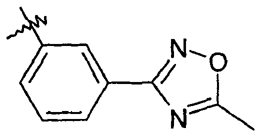 | A1 | H | CH₃ | CH₃ | H | <3000 | 1 |
| P15 | 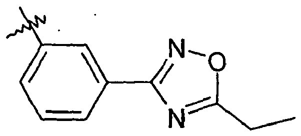 | A1 | H | CH₃ | CH₃ | H | 7033 | 1 |

| | | | | | | | | |
|---|---|---|---|---|---|---|---|---|
| * - n is the number of times the assay was performed. | | | | | | | | |

Ex. P1: Using Method F, 6,7-dimethyl-1'-{3-[5-(trifluoromethyl)-1H-pyrazol-3-yl]benzoyl}spiro-[chromene-2,4'-piperidin]-4(3H)-one was prepared as follows. To a flask was added 3-[5-(trifluoromethyl)-1 H-pyrazol-3-yl]benzoic acid (84.5 mg, 0.33 mmol) and thionyl chloride (357 mg, 3.0 mmol) and the mixture was heated to 60° C for 1 hour. The reaction mixture was concentrated and azeotroped with toluene (3x) and the residue dried under reduced pressure. 6,7-Dimethylspiro[chromene-2,4 -piperidin]-4(3H)-one (73.6 mg, 0.3 mmol) was dissolved in CH₂Cl₂ (1 mL) and DIEA (85 mg, 0.12 mL, 0.66 mmol). This solution was added to a solution of the acid chloride in CH₂Cl₂ (to a final concentration of 1 M). The resultant mixture was stirred overnight at room temperature. The material was purified by liquid chromatography (Biotage Flash 40, 98:2 CH₂Cl₂/MeOH) to provide the title material (96 mg, 70%). MS(ACPI) *m*/*z* 484 (M+H)⁺, HPLC RT 2.9 minutes, ¹H NMR (CDCl₃) δ 7.69 (s, 1H), 7.59 (s, 1H), 7.54 (d, J=7.4, 1 H), 7.35-7.39 (m., 1 H), 7.29-7.31 (m, 1H), 6.78 (s, 1 H), 6.70 (s, 1 H), 2.68 (s, 2H), 2.26 (s, 3H), 2.20 (s, 3H).

Ex. P2: 6-chloro-7-methyl-1'-{3-[5-(trifluoromethyl)-1H-pyrazol-3-yl]benzoyl}spiro[chromene-2,4'-piperidin]-4(3H)-one trifluoroacetic acid salt, MS(ACPI) *m*/*z* 504 (M+H)⁺, HPLC RT 3.0 minutes

Ex. P3: 1'-[2-chloro-5-(1-methyl-1H-pyrazol-3-yl)benzoyl]-6,7-dimethylspiro[chromene-2,4'-piperidin]-4(3H) one, MS(ACPI) *m*/*z* 464 (M+H)⁺, HPLC RT 1.88 minutes

Ex. P4:1'-[2-fluoro-5-(1H-pyrazol-3-yl)benzoyl]-6,7-dimethylspiro[chromene-2,4'-piperidin]-4(3H)-one trifluoroacetic acid salt, MS(ACPI) *m*/*z* 434 (M+H)⁺, HPLC RT 1.71 minutes

Ex. P5: 1'-[2-chloro-5-(1H-pyrazol-3-yl)benzoyl]-6,7-dimethylspiro[chromene-2,4'-piperidin]-4(3H)-one trifluoroacetic acid salt , MS(ACPI) *m*/*z* 450 (M+H)⁺, HPLC RT 1.76 minutes

Ex. P6: 1'-[2-hydroxy-5-(1 H-pyrazol-3-yl)benzoyl]-6,7-dimethylspiro[chromene-2,4'-piperidin]-4(3H)-one trifluoroacetic acid salt , MS(ACPI) *m*/*z* 432 (M+H)⁺, HPLC RT 1.48 minutes

Ex. P7: 1'-[2-ethoxy-5-(1H-pyrazol-3-yl)benzoyl]-6,7-dimethylspiro[chromene-2,4'-piperidin]-4(3H)-one trifluoroacetic acid salt, MS(ACPI) *m*/*z* 460 (M+H)⁺, HPLC RT 1.73 minutes

Ex. P8: 1'-[2-ethoxy-5-(1H-pyrazol-3-yl)benzoyl]-6,7-dimethylspiro[chromene-2,4'-piperidin]-4(3H)-one MS(ACPI) *m*/*z* 460 (M+H)⁺, HPLC RT 2.6 minutes

Ex. P9: 6-chloro-1'-[2-ethoxy-5-(1H-pyrazol-3-yl)benzoyl]-7-methylspiro[chromene-2,4'-piperidin]-4(3H)-one, MS(ACPI) *m*/*z* 480 (M+H)⁺, HPLC RT 2.8 minutes

Ex. P10: 1'-[2-chloro-5-(1-methyl-1H-pyrazol-5-yl)benzoyl]-6,7-dimethylspiro[chromene-2,4'-piperidin]-4(3H)-one, MS(ACPI) *m*/*z* 464 (M+H)⁺, HPLC RT 1.83minutes

Ex. P11: 6,7-Dimethyl-1'-[3-(1H-pyrazol-1-yl)benzoyl]spiro[chromene-2,4'-piperidin]-4(3H)-one, MS(ACPI) *m*/*z* 416 (M+H)⁺, HPLC RT 1.78 minutes

Ex. P12: 1'-[3-(1H-imidazol-2-yl)benzoyl]-6,7-dimethylspiro[chromene-2,4'-piperidin]-4(3H)-one trifluoroacetic acid salt, MS(ACPI) *m*/*z* 416 (M+H)⁺ 416, HPLC RT 1.26

Ex. P13: 6,7-Dimethyl-1'-[3-(pyrimidin-4-yl)benzoyl]spiro[chromene-2,4'-piperidin]-4(3H)-one, MS(ACPI) *m*/*z* 428 (M+H)⁺, HPLC RT 1.64 minutes

Ex. P14: 6,7-Dimethyl-1'-[3-(5-methyl-1,2,4-oxadiazol-3-yl)benzoyl]spiro[chromene-2,4'-piperidin]-4(3H)-one, MS(ACPI) *m*/*z* 432 (M+H)⁺, HPLC RT 1.82 minutes

Ex. P15: 6,7-Dimethyl-1'-[3-(5-ethyl-1,2,4-oxadiazol-3-yl)benzoyl]spiro[chromene-2,4'-piperidin]-4(3H)-one, MS(ACPI) *m*/*z* 446 (M+H)⁺, HPLC RT 1.95 minutes

### Biological Protocols

The utility of the compounds of Formula (1), and the pharmaceutically acceptable salts of the compounds, in the treatment of diseases (such as are detailed herein) in animals, particularly mammals (e.g., humans) may be demonstrated by the activity thereof in conventional assays known to one of ordinary skill in the relevant art, including the *in vitro* and *in vivo* assays described below. Such assays also provide a means whereby the activities of the compounds of Formula (1) can be compared with the activities of other known compounds.

The following protocols can of course be varied by those skilled in the art.

### Direct Inhibition of the Activities of ACC1 and ACC2

The ACC inhibitory activity of the Formula (1) compounds of this invention, and the salts of such compounds, were demonstrated by methods based on standard procedures. For example direct inhibition of ACC1 and ACC2 activity, for compounds of Formula (1) were determined using preparations of ACC1 from rat liver and ACC2 from rat skeletal muscle.

[1] Preparation of ACC1 and ACC2. ACC1 was obtained from rat liver and ACC2 was obtained from rat skeletal muscle based upon standard procedures such as those described by Thampy and Wakil (J. Biol. Chem. 260: 6318-6323; 1985) using the following method.

Male CD rats weighing 150-200 g are fasted for 2 days and then fed a high sucrose diet (AIN-76A rodent diet; Cat # D10001, Research Diets Inc., New Brunswick, N.J.), for 3 days at which time they are sacrificed by CO₂ asphyxiation. The livers (for ACC1 preparation) or skeletal muscle tissue (for ACC2 preparation) are removed, rinsed in ice-cold phosphate-buffered saline (PBS), and homogenized in 5 volumes of homogenization buffer (50 mM potassium phosphate, pH 7.5, 10 mM EDTA, 10 mM 2-mercaptoethanol, 2 mM benzamidine, 0.2 mM phenylmethylsulfonylfluoride (PMSF), 5 mg/L each leupeptin, aprotinin, and antitrypsin) in a Waring® blender for 1 minute at 4 °C. All subsequent operations are carried out at 4 °C. The homogenate is made 3% with respect to polyethylene glycol (PEG) by the addition of 50% PEG solution and centrifuged at 20,000 x g for 15 minutes. The resulting supernatant is adjusted to 5% PEG with the addition of 50% PEG solution and stirred for 5 minutes. The pellet (contains ACC activity) is collected by centrifugation at 20,000 x g for 20 minutes, rinsed with ice-cold doubly distilled water to remove excess PEG and re-suspended in one-fourth the original homogenate volume with homogenization buffer. Ammonium sulfate (200 g/liter) is slowly added with stirring. After 45 minutes the enzyme is collected by centrifugation for 30 minutes at 20,000 x g, re-suspended in 10 ml of 50 mM HEPES, pH7.5, 0.1 mM DTT, 1.0 mM EDTA, and 10% glycerol and desalted on a Sephadex^{™} G-25 column (2.5 cm x 50 cm) (Pharmacia, Piscataway New Jersey now GE Healthcare) [THERE ARE 13 DIFFERENT G25 COLUMNS - WHICH ONE WAS USED?] equilibrated with the same buffer. The desalted enzyme preparation is stored in aliquots at -70 °C. Immediately prior to use, frozen ACC1 or ACC2 aliquots are thawed, diluted to 500 µg/ml in buffer containing 50 mM HEPES, pH7.5, 10 mM MgCl2, 10 mM tripotassium citrate, 2.0 mM dithiothreitol (DTT), and 0.75 mg/ml fatty acid-free bovine serum albumin (BSA) and pre-incubated at 37 °C for 30 minutes.

[2] Measurement of ACC inhibition. The procedures for measuring ACC1 inhibition and ACC2 inhibition are identical except for the source of the isozyme. For measurement of ACC activity and assessment of ACC inhibition, test compounds are dissolved in dimethylsulfoxide (DMSO) and 1 µL aliquots are placed in 0.5 ml polypropylene tubes. Control tubes contain 1 µL of DMSO alone: Tubes are incubated at 37 °C in a constant temperature water bath. All assay tubes receive 139 µL of substrate buffer containing 50 mM HEPES, pH7.5, 2.0 mM MgCl₂ 2.0 mM tripotassium citrate, 2 mM DTT, 0.755 mg/ml BSA, 25 µM acetyl-CoA, 4.0 mM ATP, and 12.5 mM KH[¹⁴C]O₃ (2 x10⁶ cpm). The reaction is then initiated by the addition of 10 µL of pre-incubated ACC fraction prepared as described above. After 7 minutes the reaction is terminated by the addition of 50 µL of 6N HCl and a 150 µL aliquot of the reaction mixture is transferred to glass scintillation vials and evaporated to dryness at 90 °C for at least 1 hour. The dried vials are then cooled, 0.5 ml of water and 5.5 ml of Ready Safe liquid scintillation fluid (Beckman Coulter Inc., Fullerton, CA) are added, and the radioactivity is determined using a liquid scintillation counter. Tubes that received HCl before ACC served as blanks.

[3] Specificity for ACC1 vs ACC2 inhibition. The specificity of a compound for inhibiting ACC1 vs ACC2 can be determined by comparing the concentration of test compound required to inhibit 50% of the activity contained in an aliquot of ACC1 as compared with the concentration of the same compound required to inhibit 50% of the activity of an aliquot of ACC2.

### Measurement of ACC Inhibition in Cultured Cells

The ACC inhibitory activity of compounds of this invention, and the salts of such compounds, was confirmed in cultured human cells using methods based on standard procedures. For example, since ACC catalyzes the first committed step in the biosynthesis of fatty acids, the *in vivo* activity of the certain compounds of Formula (1) was confirmed by measuring the ability of compounds, and the salts of such compounds, to prevent the formation of radio labeled fatty acids from radio labeled acetate in cultured mammalian hepatocytes or in cultured human hepatoma cells of the Hep-G2 cell line (ATCC HB 8065). Direct assessment of malonyl-CoA production in cells isolated from tissues that do (e.g. liver and adipose tissue) or do not synthesize fatty acids (e.g. skeletal muscle) can also be used to determine ACC inhibition in cells isolated from those tissues.

[1] Measurement of fatty acid synthesis inhibition in cultured cells. Fatty acid synthesis is assessed in cultured mammalian hepatocytes or in human hepatoma cells of the Hep-G2 cell line by measuring incorporation of [2-¹⁴C]acetate into saponifiable lipids essentially as previously described for assessment of sterol synthesis (Harwood et al. Biochem. Pharmacol. 53: 839-864,1997; Petras et al. J. Lipid Res. 40: 24-38,1999) with the following modifications to allow assessment fatty acid synthesis. For example, Hep-G2 cells grown in T-75 flasks and released by trypsin treatment as previously described (Harwood et al. Biochem. Pharmacol. 53: 839-864,1997; Petras et al. J. Lipid Res. 40: 24-38, 1999), are seeded in 24 well plates at a density of 1.2x10⁵ cells/well and maintained in 1.0 mL of Supplemented Dulbecco's minimal essential media (DMEM) medium (DMEM medium containing 10% heat-inactivated fetal bovine serum, 2 mM L-glutamine, 40 .µg/mL gentamicin) for 7 days in a 37 °C., 5% CO₂ incubator with medium changes on days 3 and 5. At this time, cultures reach 80-90% confluency and maintained a >90% cell viability (Trypan blue dye exclusion). On day 8, the medium is removed and replaced with fresh medium containing 1% DMSO ± the test compound. Immediately after compound addition, 25 µL of media containing 4 µCi of [2-¹⁴C]acetate (56 mCi/mmol) is added to each incubation well. Plates are then sealed with parafilm to avoid evaporation, and cells are incubated at 37 °C for 6 hours with gentle shaking. After incubation, the samples are saponified by addition to each well of 1 ml of 5 N KOH in MeOH, followed first by incubation for 2 hours at 70 °C and then by overnight incubation at room temperature: Mixtures are transferred to glass conical tubes and extracted three times with 4.5 ml hexane to remove the nonsaponifyable lipids (e.g. cholesterol, post-squalene cholesterol precursors and other non-saponifiable lipids). The remaining aqueous phase (containing fatty acid sodium salts) is acidified to pH<2 by addition of 0.5 ml of 12 M HCl. The resulting mixtures are transferred to glass conical tubes and extracted three times with 4.5 ml hexane. The pooled organic fractions (containing protonated fatty acids) are dried under nitrogen, re-suspended in 50 µL of chloroform:methanol::1:1(v/v) and applied to 1x20 cm channels of Silica Gel 60C TLC plates. Channels containing non-radioactive fatty acids were included on selected TLC plates as separation markers. TLC plates were developed in hexane:diethyl ether:acetic acid (70:30:2), air dried, and visualized for radioactive fatty acids by analysis using a Berthold Linear Radioactivity Analyzer (Berthold, Gaithersburg, Md., USA) that reports radioactive peak location and integrated peak area. Inhibition of fatty acid synthesis by the test compound can is expressed as the concentration required to reduce by 50% the dpm [2-¹⁴C]acetate incorporated into saponifiable lipids during the 6 hour incubation at 37 °C.

[2] Measurement of malonyl-CoA production inhibition in cultured cells. Direct assessment of malonyl-CoA production in cells isolated from tissues that either do (e.g. liver and adipose tissue) or do not synthesize fatty acids (e.g. skeletal muscle), through its stoichiometric conversion to radio labeled palmitate in the presence of purified fatty acid synthetase and radio labeled acetate, can also be used to determine ACC inhibition in cells isolated from those tissues as previously described (McGarry et al. J. Biol. Chem. 253: 8291-8293,1978). The procedure as it relates to whole tissues is outlined below and can be readily adapted to cultured cells by those skilled in the art.

### Acute in vivo Assessment of ACC Inhibition in Experimental Animals

The ACC inhibitory activity of compounds of this invention, and the salts of such compounds, can be confirmed *in vivo* by evaluation of their ability to inhibit hepatic fatty acid production and to stimulate whole body fatty acid oxidation using methods based on standard procedures. For example, since ACC catalyzes the first committed step in the biosynthesis of fatty acids, the *in vivo* activity of these compounds can be confirmed by measuring the ability of the compounds of this invention, and the salts of such compounds, to prevent the formation of radio labeled fatty acids from radio labeled acetate in the livers of treated mammals.

Direct assessment of radio labeled malonyl-CoA production from radio labeled acetate in tissues that either do (e.g. liver and adipose tissue) or do not synthesize fatty acids (e.g. skeletal muscle) can also be used to determine ACC inhibition in those tissues. Since reduced malonyl-CoA levels as a consequence of ACC inhibition, relieve the malonyl-CoA mediated feedback inhibition of carnitine-palmitoyl transferase 1 (CPT1), the enzyme that catalyzes the rate limiting reaction in mitochondrial fatty acid oxidation, the *in vivo* activity of the compounds of this invention, and the salts of such compounds, can be confirmed by measuring their ability to increase the utilization of fatty acids as a source of energy, as assessed by a reduction in respiratory quotient in treated mammals.

[1] Measurement of fatty acid synthesis inhibition in experimental animals. Incorporation of [2-¹⁴C]acetate into saponifyable lipids in the livers of mammals (e.g. CD1 mice, C57BI/6J-ob/ob mice, Sprague Dawley rats(available from Charles River Boston, Mass. or Jackson Labs Bar Harbor, Me.)) can be measured essentially as previously described for assessment of hepatic sterol synthesis (Harwood et al, Biochem. Pharmacol, 40: 1281-1293, 1990; Harwood et al. Biochem. Pharmacol. 53: 839-864,1997) with the following modifications to allow for assessment fatty acid synthesis. For example, Sprague Dawley rats are administered a 0.1 ml per 40 g body weight of an oral bolus of vehicle (e.g. water or 0.5% methylcellulose in water) ± test compound. One to four hours after compound administration, animals receive an intraperitoneal injection of 0.5 ml of [2-¹⁴C]acetate (64 µCi/ml; 57 mCi/mmol). One hour after radiolabel administration, animals are sacrificed by CO₂ asphyxiation and two, 0.75 g liver pieces are removed and saponified at 70 µC. for 120 minutes in 1.5 ml of 2.5 M NaOH. After saponification, 2.5 ml of absolute EtOH are added to each sample and the solutions are mixed and allowed to stand overnight. Petroleum ether, 4.8 ml, is then added to each sample and the mixtures are first shaken vigorously for 2 minutes then centrifuged at 1000 x g in a bench-top Sorvall® for 5 minutes. The resultant petroleum ether layers, which contain the nonsaponifyable lipids (e.g. cholesterol, post-squalene cholesterol precursors and other non-saponifiable lipids), are removed and discarded. The remaining aqueous layer (containing fatty acid sodium salts) is acidified to pH<2 by addition of 0.6 ml of 12 M HCl and extracted two times with 4.8 ml of petroleum ether. The pooled organic fractions (containing protonated fatty acids) are transferred to liquid scintillation vials, dried under nitrogen, dissolved in 7 ml of Aqua sol liquid scintillation fluid, and assessed for radioactivity using a liquid scintillation counter. Inhibition of fatty acid synthesis by the test compound is expressed as the concentration required to reduce by 50% the dpm [2-¹⁴C]acetate incorporated into saponifiable lipids during the 1 hour interval between radio labeled acetate injection and CO₂ asphyxiation.

Some compounds of the present were evaluated for inhibition of fatty acid synthesis as described above. As shown below, these compounds were all observed to inhibit the synthesis of fatty acids *in vivo*.

| Compound | Percent Inhibition at 10mg/kg | Percent Inhibition at 30mglkg |
|---|---|---|
| A16 | 68 | 83 |
| A13 | | 64 |
| A21 | | 59.5 |
| A12 | | 38.7 |
| A1 | | 35.4 |
| A9 | | 33.5 |
| E1 | | 30.9 |
| E2 | | 30.7 |

[2] Measurement of malonyl-CoA production inhibition in experimental animals. Direct assessment of malonyl-CoA production in tissues that either do (e.g. liver and adipose tissue) or do not synthesize fatty acids (e.g. skeletal muscle), through its stoichiometric conversion to radio labeled palmitate in the presence of purified fatty acid synthetase and radio labeled acetyl-CoA, can also be used to determine ACC inhibition in those tissues as previously described (McGarry et al. J. Biol. Chem. 253: 8291-8293, 1978). The animals are treated with vehicle ± test compound as described in [1] Measurement of fatty acid synthesis inhibition in experimental animals above. Briefly, assays are carried out in duplicate in stoppered glass test tubes. Reaction mixtures contain, in 1.025 ml of 200 mM potassium phosphate buffer (pH=7.0), 2.5 mM dithiothreitol, 2.0 mM EDTA, 0.2 mM NADPH, 1 mg/ml fatty acid free bovine serum albumin, 4.4 µM [3H]acetyl-CoA (.about.150,000 dpm/nmol), and appropriate quantities of malonyl-CoA standard or test tissue extract. Tissue extracts are prepared from tissues (e.g. liver and skeletal muscle) that are freeze-clamped within 10 seconds after CO₂ asphyxiation by first pulverizing the tissue under liquid nitrogen then extracting 1 g of powdered tissue with 5 ml of 6% (w/v) HClO₄ and neutralizing the extract to pH 6.0 with KOH and centrifugation to remove particulate residue. Reactions are initiated by addition of 25 milliunits (mU) of purified fatty acid synthetase. After a 45 minute incubation at 37 °C, reactions are terminated by addition of 25 µL of 70% (w/v) HClO₄ and nascent palmitate is then extracted by addition to each tube of 1 ml EtOH then 5 ml petroleum ether. After vigorous mixing for 30 seconds and centrifugation to facilitate phase separation, the petroleum ether phase is transferred to a second glass tube containing 2 ml water, shaken, re-centrifuged, and 2.0 ml of the petroleum ether phase is transferred to liquid scintillation vials, dried, and assessed for radioactivity in a liquid scintillation counter after addition of 10 ml Aquasol liquid scintillation fluid (PerkinElmer, Shelton, CT). Blanks containing no added malonyl-CoA nor liver extract are included with each series of determinations and subtracted from all values. Inhibition of malonyl-CoA production by the test compound is expressed as the concentration required to reduce by 50% the dpm [2-¹⁴C]acetyl-CoA incorporated into palmitate during the 45 minute incubation at 37°C.

[3] Measurement of Fatty Acrid. Oxidation Stimulation in Rats.

The ACC inhibitory activity of compounds of this invention, and the salts of such compounds, can be further confirmed *in vivo* by assessing the ability of ACC inhibition to increase fatty acid utilization by employing methods based on standard procedures. For example, during a shift from the oxidation of carbohydrate to the oxidation of fatty acids or a shift from fatty acid synthesis to oxidation, there is a decrease in respiratory quotient (RQ)=ratio of CO₂ production/O₂ consumption. Because fatty acids are in a more reduced state than carbohydrates (such as glucose), there is greater amount of oxygen consumed for each CO₂ produced and therefore a lower RQ. If an animal is utilizing only carbohydrate, RQ=1.0, whereas if an animal is utilizing only fatty acids, RQ=0.7. Thus, the RQ in animals, including humans and companion animals, is an indirect measure of type of fuel being utilized. Indirect calorimetry is commonly used in animals, including humans, by those skilled in the relevant art to measure RQ.

Those skilled in the art understand that decreased RQ and the concomitant shifting fuel utilization from the oxidation of carbohydrate to the oxidation of fat may decrease body fat stores and be efficacious with respect to the treatment of, e.g., obesity, metabolic syndrome and diabetes.

The ability of the compounds of this invention, and the salts of such compounds, to generate a decrease in RQ response may be demonstrated according to the following protocol. This *in vivo* screen is designed to evaluate the efficacy of compounds that are ACC inhibitors, using as an efficacy endpoint measurement of whole body oxygen consumption, CO₂ production and RQ. The protocol involves administering a single dose of compound to Sprague Dawley rats. Male Sprague Dawley rats having a body weight range of from about 350-400 g are housed under standard laboratory conditions prior to the initiation of the study.

On the day of testing the compound, oxygen consumption and RQ is measured using an open circuit, indirect calorimeter (Oxymax, Columbus Instruments, Columbus, Ohio 43204). The Oxymax gas sensors are calibrated with N₂ gas and a gas mixture (about 0.5% of CO₂, about 20.5% of O₂, about 79% of N₂) before each experiment. The subject rats are removed from their home cages and their body weights recorded. The rats are placed into the sealed chambers (43x43x10 cm) of the Oxymax (one rat per chamber), the chambers are placed in the activity monitors, and the air flow rate through the chambers is set at about 1.6 Umin. The Oxymax software calculates the oxygen consumption (mL/kg/h) by the rats based on the flow rate of air through the chambers and the difference in oxygen content at the inlet and output ports. The activity monitors have 15 infrared light beams spaced about one inch apart on each axis, and ambulatory activity is recorded when two consecutive beams are broken, and the results are recorded as counts.

Baseline oxygen consumption, RQ and ambulatory activity are measured about every 10 minutes for about 1 to 3.5 hours. After obtaining baseline data, the chambers are opened and a test compound and a vehicle are administered by oral gavage as a single dose. A test compound is dissolved in vehicle containing about 0.5% of methyl cellulose in water or other vehicle. The dosing volume is about 1 ml. After dosing the rats are returned to the Oxymax chambers, the lids of the chambers are closed and measurements are made every 10 minutes for about 3 to 6 hours after dosing. Change in RQ in response to test compound or vehicle is calculated on individual rats by dividing the average of the post-dosing values (excluding values obtained during time periods where ambulatory activity exceeds 100 counts) by the average of the pre-dosing baseline values (excluding the first 5 values and values obtained during time periods where ambulatory activity exceeds 100 counts) and expressing the data as % change in RQ.

### Sub-Chronic and Chronic Efficacy in Experimental Animals

The compounds of the present invention are readily adapted to clinical use as hyperinsulinemia reversing agents, insulin sensitizing agents, anti-obesity agents and anti-atherosclerotic agents. Such activity can be determined by the amount of test compound that reduces insulin levels, blunts the rise and/or accelerates the reduction in insulin and glucose levels in response to an oral glucose challenge, reduces body weight and/or reduces body composition (e.g. reduces the percentage of body fat), and reduces the accumulation of lipid deposition in the blood vessel walls relative to a control vehicle without test compound in mammals, for example Sprague Dawley rats fed either chow, a high sucrose diet or a high fat diet for from 3-8 weeks prior to and during test compound administration or male ob/ob mice or cholesterol-fed rabbits.

Also, since the concentration of insulin in blood is related to the promotion of vascular cell growth and increased renal sodium retention, (in addition to the other actions, e.g., promotion of glucose utilization) and these functions are known causes of hypertension, the compounds of this invention, by virtue of their hypoinsulinemic action, prevent, arrest and/or regress hypertension.

[1] Subchronic assessment of anti-diabetic efficacy in rats and mice. The antidiabetic potential of a Formula (1) compound of this invention, their prodrugs and the salts of such compounds and prodrugs can be demonstrated by evaluating their anti-hyperinsulinemia potential and insulin sensitizing potential using methods based on standard procedures. For example, the anti-hyperinsulinemia potential and insulin sensitizing potential of these compounds can be demonstrated in Sprague Dawley rats fed either a standard rodent diet, a high sucrose diet (AIN-76A rodent diet; Cat # D10001, Research Diets Inc., New Brunswick, N.J.) or a high fat diet (Cat # D12451, Research Diets Inc., New Brunswick, N.J.) *ad libitum* for from 3-4 weeks prior to and during test compound administration or in 4-8 week old male C57BL/6J-ob/ob mice (obtained from Jackson Laboratory, Bar Harbor, Me.) fed standard rodent diet ad libitum. Animals are treated for 1 to 8 weeks with test compound administered either by oral gavage in water or in 0.25% methylcellulose in water using a S.D., B.I.D. or T.I.D. dosing regimen or via in feed administration using a powdered version of the above-mentioned diets.

For studies in which the anti-hyperinsulinemia potential of Formula (1) compounds are evaluated, at various times during the study or at sacrifice (by CO₂ asphyxiation), blood is collected either from a tail vein of unanesthesized rats or from the retro-orbital sinus of unanesthesized mice, or from the vena cava of rats or mice at sacrifice into 0.5 ml serum separator tubes. The freshly collected samples are centrifuged for two minutes at 10,000xg at room temperature, and the serum supernatant is stored at -80 °C until analysis. Serum insulin concentration is determined using Equate®. RIA INSULIN kits (double antibody method; as specified by the manufacturer) available from Binax, South Portland, Me. The interassay coefficient of variation is 10%. The serum insulin lowering activity of the test compounds are determined by statistical analysis (unpaired t-test) of the mean serum insulin concentration between the test compound group and the vehicle-treated control group.

For studies in which the insulin-sensitizing potential of test compounds is evaluated, at various times during the study fasted animals are administered an oral or intraperitoneal 1.0 g/kg body weight bolus of glucose, and blood is collected either from a tail vein of unanesthesized rats or from the retro-orbital sinus of unanesthesized mice, at various times up to 2 hours after glucose administration into 0.5 ml serum separator tubes. The freshly collected samples are centrifuged for two minutes at 10,000xg at room temperature, and the serum supernatant is stored at -80 °C. until analysis. Serum insulin concentration is determined using Equate® RiA INSULIN kits as described above. Serum glucose concentration is determined using the Abbott VP^{™} (Abbott Laboratories, Diagnostics Division, Irving, Tex.) and VP Super System® Autoanalyzer (Abbott Laboratories, Irving, Tex.), or by the Abbott Spectrum CCX^{™} (Abbott - Laboratories, Irving, Tex.) using the A-Gent^{™} Glucose-UV Test reagent system (Abbott Laboratories, Irving, Tex.) (a modification of the method of Richterich and Dauwalder, Schweizerische Medizinische Wochenschrift, 101: 860 (1971)). The insulin-sensitizing activity of the test compounds are determined by statistical analysis (unpaired t-test) of the mean difference in peak insulin and glucose concentrations and the rate of insulin and glucose disappearance from the plasma after their respective peak levels between the test compound group and the vehicle-treated control group.

For studies in which the lipid-lowering potential of test compounds is evaluated, at various times during the study or at sacrifice (by CO₂ asphyxiation), blood is collected either from a tail vein of unanesthesized rats or from the retro-orbital sinus of unanesthesized mice, or from the vena cava of rats or mice at sacrifice into 0.5 ml serum separator tubes. The freshly collected samples are centrifuged for two minutes at 10,000xg at room temperature, and the serum supernatant is stored at -80 °C until analysis. Serum triglycerides are determined using the Abbott VP^{™} and VP Super System® Autoanalyzer (Abbott Laboratories, Irving, Tex.), or the Abbott Spectrum CCX^{™} (Abbott Laboratories, Irving, Tex.) using the AGent^{™} Triglycerides Test reagent system (Abbott Laboratories, Diagnostics Division, Irving, Tex.) (lipase-coupled enzyme method; a modification of the method of Sampson, et al., Clinical Chemistry 21: 1983 (1975)). Serum total cholesterol levels are determined using the Abbott VP^{™} and VP Super System® Autoanalyzer (Abbott Laboratories, Irving, Tex.), and A-Gent^{™} Cholesterol Test reagent system (cholesterol esterase-coupled enzyme method; a modification of the method of Allain, et al. Clinical Chemistry 20: 470 (1974)) using 100 and 300 mg/dl standards. Serum free fatty acid concentration is determined utilizing a kit from Amano International Enzyme Co., Inc., as adapted for use with the Abbott VP^{™} and VP Super System® Autoanalyzer (Abbott Laboratories, Irving, Tex.), or the Abbott Spectrum CCX^{™} (Abbott Laboratories, Irving, Tex.). The serum triglyceride, cholesterol and free fatty acid lowering activity of the test compounds are determined by statistical analysis (unpaired t-test) of the mean serum triglyceride, cholesterol, and free fatty acid concentrations between the test compound group and the vehicle-treated control group.

[2] Subchronic assessment of anti-obesity efficacy in rats and mice. The anti-obesity potential of a Formula (1) compound of this invention, their prodrugs and the salts of such compounds and prodrugs can be demonstrated by evaluating their potential to produce a reduction in body weight, a reduction in percentage body fat, and a reduction in plasma leptin levels.

For example, the body weight reduction, percentage body fat reduction, and plasma leptin reduction potential of test compounds can be demonstrated in Sprague Dawley rats fed either a standard rodent diet, a high sucrose diet (AIN-76A rodent diet; Cat # D10001, Research Diets Inc., New Brunswick, N.J.) or a high fat diet (Cat # D12451, Research Diets Inc., New Brunswick, N.J.) ad libitum for from 3-4 weeks prior to and during test compound administration or in 4-8 week old male C57BL/6J-bb/ob mice (obtained from Jackson Laboratory, Bar Harbor, ME) fed standard rodent diet *ad libitum*. Animals are treated for 1 to 8 weeks with a test compound administered either by oral gavage in water or 0.25% methylcellulose in water using a S.D., B.I.D. or T.I.D. dosing regimen or via in feed administration using a powdered version of the above-mentioned diets.

Whole body weight loss can be assessed simply be comparison of total body weight before and after treatment with a test compound. For assessment of weight loss and change in body composition (e.g. the change in percentage body fat and in the ratio of lean body mass to fat mass) treated and control animals were lightly anesthetized and scanned using dual-energy x-ray absorptiometry (DEXA, QDR-1000/W, Hologic Inc., Waltham, Mass.) equipped with "Regional High Resolution Scan" software. The scan field size was adjusted to accommodate the size of the species being evaluated. Resolution was 0.0254 x 0.0127 cm and scan speed was 7.25 mm/second. The whole body weight, percentage body fat, and ratio of fat mass to lean body mass lowering activity of the test compounds are determined by statistical analysis (unpaired t-test) of the mean whole body weight, percentage body fat, and ratio of fat mass to lean body mass between the test compound group and the vehicle-treated control group.

Changes in plasma leptin levels closely parallel changes in percentage body fat and are therefore a useful marker for assessing anti-obesity potential. For assessment of changes in plasma leptin levels in response to treatment with test compounds, at various times during the study or at sacrifice (by CO₂ asphyxiation), blood is collected either from a tail vein of unanesthesized rats or from the retro-orbital sinus-of unanesthesized mice, or from the vena cava of rats or mice at sacrifice into 0.5 ml serum separator tubes. The freshly collected samples are centrifuged for two minutes at 10,000 x g at room temperature, and the serum supernatant is stored at -80 °C until analysis. Serum leptin concentration is determined using LINCO rat leptin RIA kit (Cat # RL-83K; double antibody method as specified by the manufacturer) available from LINCO, St Charles, Mo. The serum leptin towering activity of the test compounds is determined by statistical analysis (unpaired t-test) of the mean serum leptin concentration between the test compound group and the vehicle-treated control group.

[3] Chronic assessment of anti-atherosclerotic efficacy in rabbits. To demonstrate the anti-atherosclerotic potential of a Formula (1) compound of this invention, and the salts of such compounds, anti-atherosclerotic effects of the can be determined by the amount of test compound required to reduce the lipid deposition in rabbit aorta. Male New Zealand White rabbits are fed a diet containing 0.2% cholesterol and 10% coconut oil for 4 days (meal-fed once per day). Rabbits are bled from the marginal ear vein and total plasma cholesterol values are determined from these samples. The rabbits are then assigned to treatment groups so that each group has a similar mean ± SD for total plasma cholesterol concentration, HDL cholesterol concentration and/or triglyceride concentration. After group assignment, rabbits are dosed daily with test compound given as a dietary admix or on a small piece of gelatin based confection. Control rabbits receive only the dosing vehicle, be it the food or the gelatin confection. The cholesterol/coconut oil diet is continued along with the test compound administration throughout the study. Plasma cholesterol and/or triglyceride values can be determined at any point during the study by obtaining blood from the marginal ear vein. After 3-5 months, the rabbits are sacrificed and the aorta are removed from the thoracic arch to the branch of the iliac arteries. The aorta are cleaned of adventitia, opened longitudinally and then stained with Sudan IV as described by Holman et. al. (Lab. Invest. 1958, 7, 42-47). The percent of the surface area stained is quantitated by densitometry using an Optimas Image Analyzing System (Image Processing Systems). Reduced lipid deposition is indicated by a reduction in the percent surface area stained in the compound-receiving group in comparison with the control rabbits.

## Claims

1. A compound, having the formula 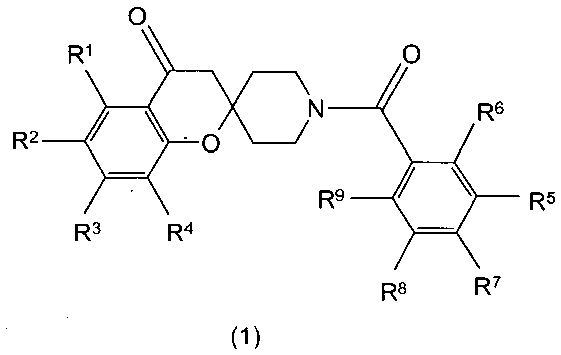 or a pharmaceutically acceptable salt thereof, wherein:
(a) R¹ is H, OH, halo, cyano, C₁₋₃ alkyl, C₁₋₃ alkoxy, C₁₋₃ haloalkyl, C₁₋₃ haloalkoxy, C₁₋₃ alkylsulfonyl-, -CO(O)H, -C(O)OC₁₋₃ alkyl or phenyl, wherein said phenyl is optionally substituted with one to five R¹⁰;
(b) each R¹⁰ is independently OH, halo, cyano, C₁₋₃ alkyl, C₁₋₃ alkoxy, C₁₋₃ haloalkyl or C₁₋₃ haloalkoxy;
(c) R² and R³ are each independently H, OH, halo, cyano, C₁₋₃ alkyl, C₁₋₃ alkoxy, C₁₋₃ haloalkyl, C₁₋₃ haloalkoxy, C₁₋₃ alkylsulfonyl-, -CO(O)H, -C(O)OC₁₋₃ alkyl, -C(O)NR¹¹R¹², or phenyl wherein said phenyl is optionally substituted with one to five R¹⁰;
(d) R¹¹ and R¹² are taken separately and are each independently H or C₁₋₃ alkyl, or R¹¹ and R¹² are taken together, with the nitrogen to which they are attached, to form a 4-7-membered heterocycloalkyl;
(e) R⁴ is H, halo, cyano, C₁₋₃ alkyl or C₁₋₃ haloalkyl;
(f) R⁶ is taken separately and is H, OH, halo, C₁₋₃ alkyl, C₁₋₃ alkoxy, C₁₋₃ haloalkyl or C₁₋₃ haloalkoxy;
(g) R⁷ is taken separately and is H, OH, halo, C₁₋₃ alkyl, C₁₋₃ alkoxy, C₁₋₃ haloalkyl or C₁₋₃ haloalkoxy;
(h) R⁵ is taken separately and is a 4-7-membered heteroaryl optionally substituted with halo, C₁₋₃ alkyl, C₁₋₃ alkoxy, C₁₋₃ alkyl-OH, C₁₋₃ haloalkyl or C₁₋₃; or
(i) R⁵ is taken together with R⁶ or R⁷, and with the phenyl to which R⁵ and R⁶ or R⁷ are attached, to form a polycyclic heterocyclic radical, with a nitrogen-bearing ring wherein at least one nitrogen atom is bound to a carbon atom of said phenyl, wherein the nitrogen-bearing ring is optionally fused to cyclohexene, 5,6-dihydro-pyridine or 5,6-dihydro-1H-pyridin-2-one, and wherein the nitrogen-bearing ring is optionally substituted independently with one to two oxo, halo, C₁₋₃ alkyl, C₁₋₃ alkoxy, C₁₋₃ alkyl-OH, C₁₋₃ haloalkyl, C₁₋₃ haloalkoxy, 4-7-membered heteroaryl, 4-7-membered heterocycloalkyl or phenyl, wherein said phenyl is optionally substituted with one to five R¹⁰, provided that R⁵ is not taken together with R⁶ to form a benzotriazolyl or a benzooxadiazolyl and provided that R⁵ is not taken together with R⁷ to form a benzooxadiazolyl; and
(j) R³ and R⁹ are independently H, OH, halo, C₁₋₃ alkyl, C₁₋₃ alkoxy, C₁₋₃ haloalkyl or C₁₋₃ haloalkoxy,
provided that said compound is not 1'-(1H-1,2,3-benzotriazol-5-ylcarbonyl)-5-methoxyspiro[chromene-2,4'-piperidin]-4(3H)-one; 6-chloro-7-methyl-1'-[3-(1H-pyrazol-4-yl)benzoyl]spiro[chromene-2,4'-piperidin]-4(3H)-one; 6,7-dimethyl-1'-[3-(1H-pyrazol-4-yl)benzoyl]spiro[chromene-2,4'-piperidin]-4(3H)-one; or6,7-dimethyl-1'-[3-(1H-pyrazol-4-yl)benzoyl]spiro[chromene-2,4'-piperidin]-4(3H)-one.

2. A compound of Claim 1, or a pharmaceutically acceptable salt thereof, wherein R⁵ and R⁷ are taken together, wherein said optionally substituted nitrogen-bearing ring optionally contains a second N, O, or S heteroatom, and wherein said nitrogen-bearing ring is optionally fused to cyclohexene, 5,6-dihydro-pyridine or 5,6-dihydro-1H-pyridin-2-one.

3. A compound of Claim 2, or a pharmaceutically acceptable salt thereof, wherein R⁵ and R⁷ are taken together wherein said polycyclic heterocyclic radical is 1H-indazolyl, 1 H-benzoimidazolyl, quinolyl, 1,2,3,4-tetrahydroquinolyl, quinoxalyl, 1H-indolyl, 2,3-dihydro-1H-benzoimidazolyl, 1H-benzo-[d][1,2,3]triazolyl, 6,7,8,9-tetrahydro-5H-carbazolyl, 2,3,4,9-tetrahydro-1H-pyrido-[3,4-b]indolyl or benzooxazolyl, and wherein the nitrogen-bearing ring of said polycyclic heterocyclic radical is optionally substituted.

4. A compound of Claim 3, or a pharmaceutically acceptable salt thereof, wherein the polycyclic heterocyclic radical is optionally substituted 1H-indazolyl, 1H-benzoimidazolyl, 1H-indolyl or 2,3,4,9-tetrahydro-1H-pyrido[3,4-b]indolyl.

5. A compound of Claim 4, or a pharmaceutically acceptable salt thereof, wherein:
(a) R¹ is H, halo, CH₃ or OCH₃;
(b) R³ is H, halo, CH₃ or OCH₃; and
(c) R⁴ is H.

6. A compound of Claim 1, or a pharmaceutically acceptable salt thereof, wherein R⁵ and R⁶ are taken together, wherein said optionally substituted nitrogen-bearing ring optionally contains a second N, O, or S heteroatom, and wherein said nitrogen-bearing ring is optionally fused to cyclohexene, 5,6-dihydro-pyridine or 5,6-dihydro-1H-pyridin-2-one.

7. A compound of Claim 6, or a pharmaceutically acceptable salt thereof, wherein R⁵ and R⁶ are taken together wherein said polycyclic heterocyclic radical is 1H-indazolyl, 1 H-benzoimidazolyl, 1 H-indolyl or 2,3-dihydro-1H-benzoimidazolyl, and wherein the nitrogen-bearing ring of said heterocyclic radical is optionally substituted.

8. A compound of Claim 7, or a pharmaceutically acceptable salt thereof, wherein the polycyclic heterocyclic radical is optionally substituted 1H-indazolyl.

9. A compound of Claim 8, or a pharmaceutically acceptable salt thereof, wherein:
(a) R¹ is H, halo, CH₃ or OCH₃;
(b) R³ is H, halo, CH₃ or OCH₃; and
(c) R⁴ is H.

10. A compound of Claim 1, or a pharmaceutically acceptable salt thereof, wherein R⁵ is taken separately and is optionally substituted pyrazolyl, imidazolyl, oxadiazolyl or pyrimidinyl.

11. A compound of Claim 10, or a pharmaceutically acceptable salt thereof, wherein R⁵ is taken separately and is optionally substituted pyrazolyl or imidazolyl.

12. A compound of Claim 11, or a pharmaceutically acceptable salt thereof, wherein:
(a) R¹ is H, halo, CH₃ or OCH₃;
(b) R³ is H, halo, CH₃ or OCH₃; and
(c) R⁴ is H.

13. A pharmaceutical composition comprising:
(1) A compound according to any of the claims 1 to 12; and
(2) a pharmaceutically acceptable carrier, vehicle, diluent or excipient.

14. A compound according to any of the claims 1 to 12, for use in treating obesity or a condition of being overweight in a mammal.

15. The compound of Claim 14 wherein said mammal is a human.

## Patentansprüche

1. Verbindung mit der Formel 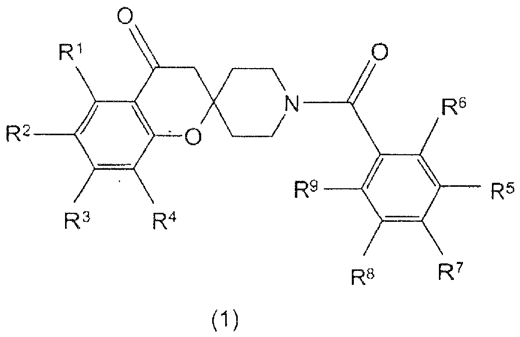 oder ein pharmazeutisch verträgliches Salz davon, wobei:
(a) R¹ H, OH, Halogen, Cyano, C₁₋₃-Alkyl, C₁₋₃-Alkoxy, C₁₋₃-Halogenalkyl, C₁₋₃-Halcgenalkoxy, C₁₋₃-Alkylsulfonyl-, -CO(O)H, -C(O)OC₁₋₃-Alkyl oder Phenyl ist, wobei das Phenyl gegebenenfalls mit einem bis fünf R¹⁰ substituiert ist;
(b) jedes R¹⁰ unabhängig OH, Halogen, Cyano, C₁₋₃-Alkyl, C₁₋₃-Alkoxy, C₁₋₃Halogenalkyl oder C₁₋₃-Halogenalkoxy ist;
(c) R² und R³ jeweils unabhängig H, OH, Halogen, Cyano, C₁₋₃-Alkyl, C₁₋₃-Alkoxy, C₁₋₃-Halogenalkyl, C₁₋₃-Halogenalkoxy, C₁₋₃-Alkylsulfonyl-, -CO(O)H, -C(O)OC₁₋₃-Alkyl, -C(O)NR¹¹R¹² oder Phenyl sind, wobei das Phenyl gegebenenfalls mit einem bis fünf R¹⁰ substituiert ist;
(d) R¹¹ und R¹² getrennt sind und jeweils unabhängig H oder C₁₋₃-Alkyl sind oder
R¹¹ und R¹² mit dem Stickstoff, an den sie gebunden sind, zusammengenommen sind, um ein 4-7-gliedriges Heterocycloalkyl zu bilden;
(e) R⁴ H, Halogen, Cyano, C₁₋₃-Alkyl oder C₁₋₃-Halogenalkyl ist;
(f) R⁶ getrennt genommen wird und H, OH, Halogen, C₁₋₃-Alkyl, C₁₋₃-Alkoxy, C₁₋₃-Halogenalkyl oder C₁₋₃-Halogenalkoxy ist;
(g) R⁷ getrennt genommen wird und H, OH, Halogen, C₁₋₃-Alkyl, C₁₋₃-Alkoxy, C₁₋₃-Halogenalkyl oder C₁₋₃Halogenalkoxy ist;
(h) R⁵ getrennt genommen ist und ein 4-7-gliedriges Heteroaryl ist, das gegebenenfalls mit Halogen, C₁₋₃-Alkyl, C₁₋₃-Alkoxy, C₁₋₃-Alkyl-OH, C₁₋₃-Halogenalkyl oder C₁₋₃ substituiert ist oder
(i) R⁵ mit R⁶ oder R⁷ und mit dem Phenyl, an das R⁵ und R⁶ oder R⁷ gebunden sind, unter Bildung eines polycyclischen heterocyclischen Restes zusammengenommen wird, und zwar mit einem Stickstoff tragenden Ring, wobei wenigstens ein Stickstoffatom an ein Kohlenstoffatom des Phenyls gebunden ist, wobei der Stickstoff tragende Ring gegebenenfalls an Cyclohexen, 5,6-Dihydropyridin oder 5,6-Dihydro-1H-pyridin-2-on anneliert ist und wobei der Stickstoff tragende Ring gegebenenfalls unabhängig mit einem bis zwei Oxo, Halogen, C₁₋₃-Alkyl, C₁₋₃-Alkoxy, C₁₋₃-Alkyl-OH, C₁₋₃-Halogenalkyl, C₁₋₃-Halogenalkoxy, 4-7-gliedrigem Heteroaryl, 4-7-gliedrigem Heterocycloalkyl oder Phenyl substituiert ist, wobei das Phenyl gegebenenfalls mit einem bis fünf R¹⁰ substituiert ist, mit der Maßgabe, dass R⁵ nicht unter Bildung eines Benzotriazolyl oder eines Benzooxadiazolyl mit R⁶ zusammengenommen wird, und mit der Maßgabe, dass R⁵ nicht unter Bildung eines Benzooxadiazolyls mit R⁷ zusammengenommen wird, und
(j) R⁸ und R⁹ unabhängig H, OH, Halogen, C₁₋₃-Alkyl, C₁₋₃-Alkoxy, C₁₋₃-Halogenalkyl oder C₁₋₃-Halogenalkoxy sind,
mit der Maßgabe, dass die Verbindung nicht 1'-(1H-1,2,3-Benzotriazol-5-ylcarbonyl)-5-methoxyspiro[chromen-2,4'-piperidin]-4(3H)-on; 6-Chlor-7-methyl-1'-[3-(1H-pyrazol-4-yl)benzoyl]spiro[chromen-2,4'-piperidin]-4(3H)-on; 6,7-Dimethyl-1'-[3-(1H-pyrazol-4-yl)benzoyl]spiro[chromen-2,4'-piperidin]-4(3H)-on oder 6,7-Dimethyl-1'-[3-(1H-pyrazol-4-yl)benzoyl]spiro[chromen-2,4'-piperidin]-4(3H)-on ist.

2. Verbindung gemäß Anspruch 1 oder ein pharmazeutisch verträgliches Salz davon, wobei R⁵ und R⁷ zusammen genommen werden, wobei der gegebenenfalls substituierte Stickstoff tragende Ring gegebenenfalls ein zweites N-, O- oder S-Heteroatom enthält und wobei der Stickstoff tragende Ring gegebenenfalls an Cyclohexen, 5,6-Dihydropyridin oder 5,6-Dihydro-1H-pyridin-2-on anneliert ist.

3. Verbindung gemäß Anspruch 2 oder ein pharmazeutisch verträgliches Salz davon, wobei R⁵ und R⁷ zusammengenommen sind, wobei der polycyclische heterocyclische Rest 1H-Indazolyl, 1H-Benzoimidazolyl, Chinolyl, 1,2,3,4-Tetrahydrochinolyl, Chinoxalyl, 1H-Indolyl, 2,3-Dihydro-1H-benzoimidazolyl, 1H-Benzo-[d][1,2,3]triazolyl, 6,7,8,9-Tetrahydro-5H-carbazolyl, 2,3,4,9-Tetrahydro-1H-pyrido[3,4-b]indolyl oder Benzooxazolyl ist und wobei der Stickstoff tragende Ring des polycyclischen heterocyclischen Restes gegebenenfalls substituiert ist.

4. Verbindung gemäß Anspruch 3 oder ein pharmazeutisch verträgliches Salz davon, wobei der polycyclische heterocyclische Rest gegebenenfalls substituiertes 1H-Indazolyl, 1H-Benzoimidazolyl, 1H-Indolyl oder 2,3,4,9-Tetrahydro-1H-pyrido[3,4-b]indolyl ist.

5. Verbindung gemäß Anspruch 4 oder ein pharmazeutisch verträgliches Salz davon, wobei:
(a) R¹ H, Halogen, CH₃ oder OCH₃ ist;
(b) R³ H, Halogen, CH₃ oder OCH₃ ist und
(c) R⁴ H ist.

6. Verbindung gemäß Anspruch 1 oder ein pharmazeutisch verträgliches Salz davon, wobei R⁵ und R⁶ zusammengenommen sind, wobei der gegebenenfalls substituierte Stickstoff tragende Ring gegebenenfalls ein zweites N-, O- oder S-Heteroatom enthält und wobei der Stickstoff tragende Ring gegebenenfalls an Cyclohexen, 5,6-Dihydropyridin oder 5,6-Dihydro-1H-pyridin-2-on anneliert ist.

7. Verbindung gemäß Anspruch 6 oder ein pharmazeutisch verträgliches Salz davon, wobei R⁵ und R⁶ zusammengenommen sind, wobei der polycyclische heterocyclische Rest 1H-Indazolyl, 1H-Benzoimidazolyl, 1H-Indolyl oder 2,3-Dihydro-1H-benzoimidazolyl ist und wobei der Stickstoff tragende Ring des heterocyclischen Restes gegebenenfalls substituiert ist.

8. Verbindung gemäß Anspruch 7 oder ein pharmazeutisch verträgliches Salz davon, wobei der polycyclische heterocyclische Rest gegebenenfalls substituiertes 1H-Indazolyl ist.

9. Verbindung gemäß Anspruch 8 oder ein pharmazeutisch verträgliches Salz davon, wobei:
(a) R¹ H, Halogen, CH₃ oder OCH₃ ist;
(b) R³ H, Halogen, CH₃ oder OCH₃ ist und
(c) R⁴ H ist.

10. Verbindung gemäß Anspruch 1 oder ein pharmazeutisch verträgliches Salz davon, wobei R⁵ getrennt genommen ist und gegebenenfalls substituiertes Pyrazolyl, Imidazolyl, Oxadiazolyl oder Pyrimidinyl ist.

11. Verbindung gemäß Anspruch 10 oder ein pharmazeutisch verträgliches Salz davon, wobei R⁵ getrennt genommen ist und gegebenenfalls substituiertes Pyrazolyl oder Imidazolyl ist.

12. Verbindung gemäß Anspruch 11 oder ein pharmazeutisch verträgliches Salz davon, wobei:
(a) R¹ H, Halogen, CH₃ oder OCH₃ ist;
(b) R³ H, Halogen, CH₃ oder OCH₃ ist und
(c) R⁴ H ist.

13. Pharmazeutische Zusammensetzung, umfassend:
(1) eine Verbindung gemäß einem der Ansprüche 1 bis 12 und
(2) einen pharmazeutisch verträglichen Träger, ein pharmazeutisch verträgliches Vehikel, Verdünnungsmittel oder Exzipiens.

14. Verbindung gemäß einem der Ansprüche 1 bis 12 zur Verwendung bei der Behandlung von Adipositas oder eines Zustandes der Übergewichtigkeit bei einem Säuger.

15. Verbindung gemäß Anspruch 14, wobei der Säuger ein Mensch ist.

## Revendications

1. Composé de formule 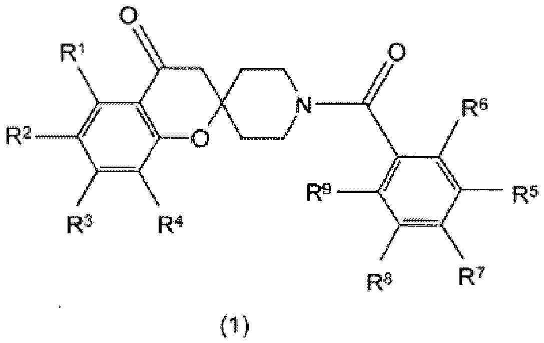 ou l'un de ses sels pharmaceutiquement acceptables, dans laquelle :
(a) R¹ est H, OH, un halogène, un cyano, un alkyle en C₁ à C₃, un alcoxy en C₁ à C₃, un halogénoalkyle en C₁ à C₃, un halogénoalcoxy en C₁ à C₃, un alkylsulfonyle en C₁ à C₃, -CO(O)H, un -C(O)O-alkyle en C₁ à C₃ ou un phényle, ledit phényle étant facultativement substitué par un à cinq R¹⁰ ;
(b) chaque R¹⁰ est indépendamment OH, un halogène, un cyano, un alkyle en C₁ à C₃, un alcoxy en C₁ à C₃, un halogénoalkyle en C₁ à C₃ ou un halogénoalcoxy en C₁ à C₃ ;
(c) R² et R³ sont chacun indépendamment H, OH, un halogène, un cyano, un alkyle en C₁ à C₃, un alcoxy en C₁ à C₃, un halogénoalkyle en C₁ à C₃, un halogénoalcoxy en C₁ à C₃, un alkylsulfonyle en C₁ à C₃, -CO(O)H, un -C(O)O-alkyle en C₁ à C₃, -C(O)NR¹¹R¹² ou un phényle, ledit phényle étant facultativement substitué par un à cinq R¹⁰ ;
(d) R¹¹ et R¹² sont pris séparément et sont chacun indépendamment H ou un alkyle en C₁ à C₃, ou R¹¹ et R¹² sont pris ensemble, avec l'azote auquel ils sont liés, pour former un hétérocycloalkyle de 4 à 7 chaînons ;
(e) R⁴ est H, un halogène, un cyano, un alkyle en C₁ à C₃ ou un halogénoalkyle en C₁ à C₃ ;
(f) R⁶ est pris séparément et est H, OH, un halogène, un alkyle en C₁ à C₃, un alcoxy en C₁ à C₃, un halogénoalkyle en C₁ à C₃ ou un halogénoalcoxy en C₁ à C₃ ;
(g) R⁷ est pris séparément et est H, OH, un halogène, un alkyle en C₁ à C₃, un alcoxy en C₁ à C₃, un halogénoalkyle en C₁ à C₃ ou un halogénoalcoxy en C₁ à C₃ ;
(h) R⁵ est pris séparément et est un hétéroaryle de 4 à 7 chaînons facultativement substitué par un halogène, un alkyle en C₁ à C₃, un alcoxy en C₁ à C₃, un alkyl (en C₁ à C₃)-OH, un halogénoalkyle en C₁ à C₃ ou un C₁ à C₃ ; ou
(i) R⁵ est pris avec R⁶ et R⁷, et avec le phényle auquel R⁵ et R⁶ ou R⁷ sont liés, pour former un radical hétérocyclique polycyclique, ayant un cycle azoté dans lequel au moins un atome d'azote est lié à un atome de carbone dudit phényle, dans lequel le cycle azoté est facultativement fusionné à un cyclohexène, une 5,6-dihydro-pyridine ou une 5,6-dihydro-1H-pyridin-2-one, et dans lequel le cycle azoté est facultativement substitué indépendamment par un à deux groupes oxo, halogène, alkyle en C₁ à C₃, alcoxy en C₁ à C₃, alkyl (en C₁ à C₃)-OH, halogénoalkyle en C₁ à C₃, halogénoalcoxy en C₁ à C₃, hétéroaryle de 4 à 7 chaînons, hétérocyclo-alkyle de 4 à 7 chaînons ou phényle, ledit phényle étant facultativement substitué par un à cinq R¹⁰, à condition que R⁵ ne soit pas pris avec R⁶ pour former un benzotriazolyle ou un benzooxadiazolyle et à condition que R⁵ ne soit pas pris avec R⁷ pour former un benzooxadiazolyle ; et
(j) R⁸ et R⁹ sont indépendamment H, OH, un halogène, un alkyle en C₁ à C₃, un alcoxy en C₁ à C₃, un halogénoalkyle en C₁ à C₃ ou un halogénoalcoxy en C₁ à C₃,
à condition que ledit composé ne soit pas la 1'-(1H-1,2,3-benzotriazol-5-ylcarbonyl)-5-méthoxyspiro-[chromène-2,4'-pipéridin]-4(3H)-one ; la 6-chloro-7-méthyl-1'-[3-(1H-pyrazol-4-yl)benzoyl]spiro[chromène-2,4'-pipéridin]-4(3H)-one ; la 6,7-diméthyl-1'-[3-(1H-pyrazol-4-yl)benzoyl]spiro[chromène-2,4'-pipéridin]-4(3H)-one ; ou la 6,7-diméthyl-1'-[3-(1H-pyrazol-4-yl)benzoyl]spiro[chromène-2,4'-pipéridin]-4(3H)-one.

2. Composé selon la revendication 1, ou l'un de ses sels pharmaceutiquement acceptables, dans lequel R⁵ et R⁷ sont pris ensemble, dans lequel ledit cycle azoté facultativement substitué contient facultativement un second hétéroatome N, O ou S, et dans lequel ledit cycle azoté est facultativement fusionné à un cyclohexène, une 5,6-dihydro-pyridine ou une 5,6-dihydro-1H-pyridin-2-one.

3. Composé selon la revendication 2, ou l'un de ses sels pharmaceutiquement acceptables, dans lequel R⁵ et R⁷ sont pris ensemble, dans lequel ledit radical hétérocyclique polycyclique est un 1H-indazolyle, un 1H-benzoimidazolyle, un quinolyle, un 1,2,3,4-tétrahydroquinolyle, un quinoxalyle, un 1H-indolyle, un 2,3-dihydro-1H-benzoimidazolyle, un 1H-benzo-[d][1,2,3] triazolyle, un 6,7,8,9-tétrahydro-5H-carbazolyle, un 2,3,4,9-tétrahydro-1H-pyrido-[3,4-b]indolyle ou un benzooxazolyle, et dans lequel le cycle azoté dudit radical hétérocyclique polycyclique est facultativement substitué.

4. Composé selon la revendication 3, ou l'un de ses sels pharmaceutiquement acceptables, dans lequel le radical hétérocyclique polycyclique est un 1H-indazolyle facultativement substitué, un 1H-benzoimidazolyle facultativement substitué, un 1H-indolyle facultativement substitué ou un 2,3,4,9-tétrahydro-1H-pyrido[3,4-b]indolyle facultativement substitué.

5. Composé selon la revendication 4, ou l'un de ses sels pharmaceutiquement acceptables, dans lequel :
(a) R¹ est H, un halogène, CH₃ ou OCH₃ ;
(b) R³ est H, un halogène, CH₃ ou OCH₃ ; et
(c) R⁴ est H.

6. Composé selon la revendication 1, ou l'un de ses sels pharmaceutiquement acceptables, dans lequel R⁵ et R⁶ sont pris ensemble, dans lequel ledit cycle azoté facultativement substitué contient facultativement un second hétéroatome N, O ou S et dans lequel ledit cycle azoté est fusionné facultativement à un cyclohexène, une 5,6-dihydro-pyridine ou une 5,6-dihydro-1H-pyridin-2-one.

7. Composé selon la revendication 6, ou l'un de ses sels pharmaceutiquement acceptables, dans lequel R⁵ et R⁶ sont pris ensemble, dans lequel ledit radical hétérocyclique polycyclique est un 1H-indazolyle, un 1H-benzoimidazolyle, un 1H-indolyle ou un 2,3-dihydro-1H-benzoimidazolyle, et dans lequel le cycle azoté dudit radical hétérocyclique polycyclique est facultativement substitué.

8. Composé selon la revendication 7, ou l'un de ses sels pharmaceutiquement acceptables, dans lequel le radical hétérocyclique polycyclique est un 1H-indazolyle facultativement substitué.

9. Composé selon la revendication 8, ou l'un de ses sels pharmaceutiquement acceptables, dans lequel :
(a) R¹ est H, un halogène, CH₃ ou OCH₃ ;
(b) R³ est H, un halogène, CH₃ ou OCH₃ ; et
(c) R⁴ est H.

10. Composé selon la revendication 1, ou l'un de ses sels pharmaceutiquement acceptables, dans lequel R⁵ est pris séparément et est un pyrazolyle facultativement substitué, un imidazolyle facultativement substitué, un oxadiazolyle facultativement substitué ou un pyrimidinyle facultativement substitué.

11. Composé selon la revendication 10, ou l'un de ses sels pharmaceutiquement acceptables, dans lequel R⁵ est pris séparément et est un pyrazolyle facultativement substitué ou un imidazolyle facultativement substitué.

12. Composé selon la revendication 11, ou l'un de ses sels pharmaceutiquement acceptables, dans lequel :
(a) R¹ est H, un halogène, CH₃ ou OCH₃ ;
(b) R³ est H, un halogène, CH₃ ou OCH₃ ; et
(c) R⁴ est H

13. Composition pharmaceutique, comprenant :
(1) un composé selon l'une quelconque revendication 1 à 12 ; et
(2) un support, véhicule, diluant ou excipient pharmaceutiquement acceptable.

14. Composé selon l'une quelconque revendication 1 à 12, pour utilisation dans le traitement de l'obésité ou d'un état de surpoids chez un mammifère.

15. Composé selon la revendication 14, dans lequel ledit mammifère est un humain.
